# EUROPEAN PATENT APPLICATION

(11) **EP 4 516 892 A1**
(43) Date of publication of application: **05.03.2025**
(21) Application number: 23795657.8
(22) Date of filing: 28.04.2023
(51) Int. Cl.: C12N 1/20, A61K 35/74, A61P 35/00, C12R 1/01

(54) **PHARMACEUTICAL COMPOSITION, MEGASPHAERA, AND USE THEREOF**

(30) Priority: 28.04.2022 CN 202210471327; 28.04.2022 CN 202210471322; 28.04.2022 CN 202210470811; 09.01.2023 CN 202310029845; 09.01.2023 CN 202310037770; 09.01.2023 CN 202310029843
(71) Applicant: Moon (Guangzhou) Biotech Co., Ltd., Guangzhou, Guangdong 510535 (CN)
(72) Inventor: HUANG, Baojia, Guangzhou, Guangdong 510535 (CN); ZHANG, Dongya, Guangzhou, Guangdong 510535 (CN); ZHAO, Guozhen, Guangzhou, Guangdong 510535 (CN); JIANG, Xianzhi, Guangzhou, Guangdong 510535 (CN)
(74) Representative: Boult Wade Tennant LLP
(86) International application number: PCT/CN2023/091798
(87) International publication number: WO 2023/208225

(57) **Abstract**

Provided herein are *Megasphaera* sp. strains deposited with the Guangdong Microbial Culture Collection Center with deposit numbers of GDMCC No: 62001, GDMCC No: 62000, and GDMCC No: 61999, respectively. 16S rRNAs of the strains are set forth in SEQ ID NOs: 1-3, respectively. The bacterial strains are highly productive of butyric acid and/or acetic acid. Further provided is use of the strains in the preparation of a pharmaceutical composition for preventing and/or treating tumors.

## Description

### CROSS-REFERENCE TO RELATED APPLICATIONS

The present application claims the right of priority to Chinese patent application No. 202210470811.2 filed on April 28, 2022; 202210471327.1 filed on April 28, 2022; 202210471322.9 filed on April 28, 2022; 202310029843.3 filed on January 9, 2023; 202310029845.2 filed on January 9, 2023; and 202310037770.2 filed on January 9, 2023, the contents of which are incorporated herein by reference in their entirety.

### TECHNICAL FIELD

The present disclosure relates to the field of biomedicine, and in particular to a pharmaceutical composition, *Megasphaera,* and use thereof.

### BACKGROUND OF THE INVENTION

The bacteria of the genus *Megasphaera* mainly exist in the intestinal tract of humans or animals, and belong to strictly anaerobic microorganisms with extremely high requirements for nutrition and culture environment and a long growth cycle. In addition, in the context of verification of pharmaceutical effect, the bacteria of this genus are difficult to verify through *in vitro* cell experiments due to their anaerobic nature. During *in vivo* experiments in animals, the bacteria of this genus are difficult to maintain a stable viable bacterial count due to the difficulty in culturing and high degree of anaerobicity, and there is also a problem of insufficient repeatability. These *in vivo* and *in vitro* related technical problems limit discovery and use of new species of the genus *Megasphaera.*

Michael Scharl, *et al.* reported in Cell Host Microbe that *Clostridium* is associated with low tumor load, demonstrated that a mixture of symbiotic *Clostridium* strains could produce powerful anti-tumor effects through CD8+ T cells, and demonstrated the feasibility of using specific intestinal bacteria as monotherapy in cancer treatment by using multiple solid tumor models. CC4, a mixture of *Clostridium* strains, can exert anti-cancer effects by activating CD8+ T cells and downregulating immunosuppressive factors. In addition, the ability of CC4 to increase infiltration of CD8+ T cells results in tumors in a highly immunogenic state, which is beneficial to improving the response rate of CRC patients to aPD-1 therapy. These findings open a new chapter of intestinal flora supplementation as an independent therapy.

The short-chain fatty acids (SCFAs) valeric acid and butyric acid can enhance the anti-tumor activities of cytotoxic T lymphocytes (CTLs) and chimeric antigen receptor (CAR) T cells through metabolic and epigenetic reprogramming. Such SCFAs are rare bacterial metabolites produced by low-abundance symbionts such as *Megasphaera massiliensis,* which can be classified as a valerate-producing bacterial species. Interestingly, dominant symbiotic bacteria cannot produce valeric acid. Studies have shown that *in vitro* treatment of CTLs and CAR T cells with valeric acid and butyric acid can increase the function of mTOR as a central cellular metabolism sensor and inhibit the activity of class I histone deacetylase. Such reprogramming leads to increased production of effector molecules such as CD25, IFN-γ, and TNF-α and significantly enhances the anti-tumor activities of antigen-specific CTLs and ROR1-targeting CAR T cells in syngeneic mouse melanoma and pancreatic cancer models. These experimental data reveal microbial molecules that can be used to enhance anti-tumor cellular immunity and support the identification of valeric acid and butyric acid as two SCFAs with therapeutic efficacy in cellular immunotherapy of cancers.

How to regulate the immune and inflammatory responses caused by intestinal flora so as not to induce a tumor and enhance the efficacy of anti-tumor drugs is still being explored. If these problems can be solved, it will be of great significance for future innovation and revolution in the field of tumor treatment. Furthermore, development of new methods for treating diseases requires exploration of new bacterial species and strains as well as characterization of new functional chemical entities. These will help accelerate the development of new microbiome-directed therapies based on novel bacterial species and functional chemicals.

In the prior art, such as in the field of probiotics or FMT transplantation, a mixture of multiple microorganisms is usually used to act on the intestinal tract. There is also prior art that uses a mixture of multiple bacteria as an anti-tumor drug. However, the mechanism of action of multiple bacteria on indications is more complex, and the influence of bacteria on each other has not been well studied. Use of multiple bacteria as a viable bacterial drug will disrupt the homeostasis of the intestinal flora. In contrast, a single bacterium has less impact on the homeostasis of the intestinal flora. In addition, the use of mixed bacteria requires additional consideration of whether the bacteria will affect each other's activities, and it is also not clear whether the synergistic effect of the mixed bacteria or a particular single bacterium plays a role in the immune process. Short-chain fatty acids are important metabolites that activate immunity. Since the presence of multiple bacteria will also affect the short-chain fatty acid pathway, it is unclear which bacterium plays a role in the short-chain fatty acid pathway and effectively generates a short-chain fatty acid for activating immune cells.

Among *Megasphaera* species, *M. massiliensis* has been extensively studied for the treatment or prevention of neurodegenerative diseases, as well as autoimmune and inflammatory diseases. Currently, only *M. massiliensis* can be effectively used to prevent and treat the indication of tumors.

There are also studies showing that *M*. *elsdenii* can be used for colorectal cancer. However, the experimental model involves direct interaction of the strain with the tumor, and the mechanism of action is actually converting excess lactic acid in the intestinal tract into other products, thereby preventing or treating the cancer caused by excessive lactic acid in the intestinal environment. No mechanism verification was conducted to extend it to other cancers such as liver cancer. More microbial species under the genus *Megasphaera* that are directed against tumors by stimulating the immune system have yet to be developed.

16S rRNA is a taxonomic basis for species. There are obvious taxonomic differences in 16S rRNA between strains of different species. Usually, 16S rRNA is used to distinguish species in the prior art. The same species indicates that different strains of the same species are highly similar at gene level. The high similarity at gene level also renders the proteins they express highly similar, and thus the bacteria have similar functions.

It is impossible to determine which strains of different species under the same genus, or different strains of the same species, can be used for tumors. Existing LBP anti-tumor viable bacterial drugs are all verified for anti-tumor effects directly through experiments on a single species strain that has been screened. This process requires a lot of time and manpower to establish experimental models and observe and analyze experimental results, which results in slow progress in development of anti-tumor viable bacterial drugs. For the research and development of microbial medicines, there is an urgent need to obtain the common properties of species under the genus *Macrosphaeria* based on the common characteristics of bacterial genera (species), and screen out strains that can be effectively used for indications such as tumors.

In view of this, the present invention is proposed.

### SUMMARY OF THE INVENTION

In one aspect, the present disclosure relates to a pharmaceutical composition comprising a bacterium of the genus *Megasphaera*;
the bacterium of the genus *Megasphaera* does not comprises *Megasphaera massiliensis*; the 16S rRNA sequence of the bacterium of the genus *Megasphaera* is ≥ 95% identical to SEQ;
the SEQ comprises at least one of SEQ ID No: 1, SEQ ID No: 2, and SEQ ID No: 3.

The composition can be used for treating a disease and inhibiting tumor growth, especially for preventing and treating a tumor.

Preferably, the 16S rRNA sequence of the bacterium of the genus *Megasphaera* is ≥ 95% (e.g., 95%, 95.5%, 96%, 96.5%, 97%, 97.5%, 98%, 98.5%, 98.65%, 99%, 99.5%, or 99.9%) identical to SEQ ID No: 1.

Preferably, the bacterium of the genus *Megasphaera* comprises at least one *of Megasphaera elsdenii, Megasphaera stantonii, Megasphaera indica, Megasphaera paucivorans, Megasphaera sueciensis, Megasphaera micronuciformis, Megasphaera hexanoica,* and *Megasphaera cerevisiae.*

Preferably, the bacterium of the genus *Megasphaera* has a genetic element for expressing EC2.8.3.9 enzyme.

Preferably, the bacterium of the genus *Megasphaera* has at least one of a butyrate-producing pathway, a pyruvate-producing pathway, and a 4-aminobutyrate-producing pathway.

Preferably, the integrity of the butyrate-producing pathway of the bacterium of the genus *Megasphaera* is ≥ 70%.

Preferably, the bacterium of the genus *Megasphaera* can inhibit the activity of histone acetylase.

Preferably, the histone acetylase comprises class I histone acetylase.

Preferably, the metabolite of the bacterium of the genus *Megasphaera* comprises a short-chain fatty acid; the short-chain fatty acid can inhibit the activity of histone acetylase; and the short-chain fatty acid can upregulate the amount of intracellular cytokines.

Preferably, the short-chain fatty acid comprises at least one of acetic acid, propionic acid, butyric acid, butyric-valeric acid, valeric acid, and hexanoic acid.

The short-chain fatty acid can enhance the anti-tumor activity of cytotoxic T cells, enhance immune response, inhibit tumor cell growth, inhibit tumor cell spread, inhibit immune escape of the tumor, enhance the efficacy of a therapeutic agent, regulate the level of symbiotic microorganisms in the intestinal tract, reduce intestinal barrier permeability, upregulate a pro-inflammatory cytokine, or increase infiltration of T cells in the tumor.

Preferably, the short-chain fatty acid exists in the form of a short-chain fatty acid salt *in vivo.*

Preferably, the short-chain fatty acid salt comprises at least one of acetate, propionate, and butyrate.

Preferably, the cell comprises at least one of macrophage, monocyte, peripheral blood mononuclear cell, and monocyte-derived dendritic cell.

Preferably, the cytokine comprises at least one of a pro-inflammatory cytokine, a protective immunogenic cytokine, and a chemokine.

Preferably, the cytokine comprises at least one of interferon γ, interleukin-1β, interleukin-6, interleukin-10, interleukin-12, interleukin-23, MCP-1, MIG, RANTES, IP-10, and tumor necrosis factor.

Preferably, the bacterium of the genus *Megasphaera* comprises at least one of viable bacteria, attenuated bacteria, dead bacteria, lyophilized bacteria, and irradiated bacteria.

Preferably, the administration route of the pharmaceutical composition comprises at least one of oral administration, rectal administration, sublingual administration, and administration by injection.

Preferably, the administration by injection comprises at least one of intradermal injection, intraperitoneal injection, subcutaneous injection, intravenous injection, intratumoral injection, and subtumoral injection.

In another aspect, the present disclosure relates to a bacterial strain, which has a 16S rRNA sequence with ≥ 95% identity to SEQ ID NO: 1.

Preferably, the bacterial strain was deposited with the Guangdong Microbial Culture Collection Center with a deposit number of GDMCC No: 62001, GDMCC No: 62000, or GDMCC No: 61999.

The bacterial strain can be used for preventing and treating various diseases, especially tumors.

In another aspect, the present disclosure relates to a bacterium of the genus *Megasphaera,* which has a 16S rRNA sequence with ≥ 95% identity to SEQ ID NO: 1.

The bacterium of the genus *Megasphaera* can be used for preventing and treating various diseases, especially tumors.

In another aspect, the present disclosure relates to a microbial agent, comprising the bacterial strain or a metabolite thereof, or the bacterium of the genus *Megasphaera* or a metabolite thereof.

The microbial agent can inhibit the growth of tumor cells.

In another aspect, the present disclosure relates to a drug for preventing and/or treating a tumor, comprising the pharmaceutical composition and a co-drug;
wherein the co-drug comprises at least one of a radiotherapeutic agent, a targeting drug, a chemotherapeutic agent, a photosensitizing agent, a photothermal agent, or an immunotherapeutic agent; preferably, the chemotherapeutic agent comprises at least one of paclitaxel, camptothecin, 5-fluorouracil, cisplatin, doxorubicin, mitomycin, or epirubicin; preferably, the photosensitizing agent comprises at least one of boron dipyrrole, chlorin, or Rose Bengal; preferably, the photothermal agent comprises at least one of indocyanine green, new indocyanine green, or gold nanoparticle rods; preferably, the immunotherapeutic agent comprises at least one of a PD-1 antibody, a CTLA-4 antibody, a PD-L1 antibody, or a PD-L1 inhibitor; preferably, the PD-L1 inhibitor comprises at least one of durvalumab, atezolizumab, or avelumab; preferably, the PD-1 antibody or PD-L1 antibody is selected from pembrolizumab or nivolumab; preferably, the CTLA-4 antibody is selected from ipilimumab.

Preferably, the tumor comprises at least one of liver cancer, colon cancer, rectal cancer, colorectal cancer, lung cancer, breast cancer, cervical cancer, ovarian cancer, pancreatic cancer, bile duct cancer, kidney cancer, fibrosarcoma, metastatic melanoma, neuroblastoma, lymphoma, leukemia, gastric cancer, hematological malignancy, or prostate cancer.

The administration mode of the composition comprises: sequentially administering the first drug and the second drug, simultaneously administering the first drug and the second drug, administering the first drug before administering the second drug, or administering the first drug and the second drug according to their respective frequencies of use.

Preferably, the anti-tumor drug comprises a PD-1 inhibitor or a target drug, and the effective dose of the PD-1 inhibitor or target drug is 1-100 mg/kg.

More preferably, the effective dose of the PD-1 inhibitor or target drug comprises: 1 mg/kg, 2 mg/kg, 3 mg/kg, 4 mg/kg, 5 mg/kg, 6 mg/kg, 7 mg/kg, 8 mg/kg, 10 mg/kg, 20 mg/kg, 30 mg/kg, 40 mg/kg, 50 mg/kg, 60 mg/kg, 70 mg/kg, 80 mg/kg, 90 mg/kg, or 100 mg/kg; and optionally, the target drug is lenvatinib.

The drug, in combination with the co-drug for treating a tumor, has a better therapeutic effect on the tumor.

In another aspect, the present disclosure relates to use of the pharmaceutical composition, the bacterial strain, or the bacterium of the genus *Megasphaera* in the manufacture of a medicament for treating and/or preventing a disease;
preferably, the disease comprises at least one of a tumor, an infectious disease, a metabolic disease, an autoimmune disease, an inflammatory disease, or a neurological disease;
preferably, the diseases comprises at least one of liver cancer, colon cancer, rectal cancer, colorectal cancer, lung cancer, breast cancer, ovarian cancer, pancreatic cancer, bile duct cancer, kidney cancer, fibrosarcoma, metastatic melanoma, cervical cancer, neuroblastoma, lymphoma, leukemia, gastric cancer, hematological malignancy, prostate cancer, type II diabetes, impaired glucose tolerance, insulin resistance, obesity, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic disease, dyslipidemia, non-alcoholic fatty liver disease, non-alcoholic steatohepatitis, atherosclerosis, nephropathy, diabetic neuropathy, diabetic retinopathy, dermatosis, dyspepsia, edema, asthma, arthritis, graft-versus-host disease, Crohn's disease, ulcerative colitis, allogeneic transplant rejection, chronic inflammatory bowel disease, systemic lupus erythematosus, psoriasis, rheumatoid arthritis, multiple sclerosis, Hashimoto's disease, food allergy, hay fever, *Clostridium difficile* infection, meningitis, myelitis, TNF-mediated inflammatory disease, gastrointestinal inflammatory disease, cardiovascular inflammatory disease, chronic obstructive pulmonary disease, depression, anxiety, post-traumatic stress disorder, obsessive-compulsive disorder, Parkinson's disease, cerebral atrophy, angiosclerotic Parkinson's disease, atherosclerotic Parkinson's disease, mild cognitive impairment, cognitive impairment, or Alzheimer's disease.

As compared to the prior art, the present invention has the following beneficial effects:
In the present disclosure, several anaerobic strains belonging to the *genus Megasphaera* has been isolated and screened out from the human intestinal tract, which are natural strains (non-cloned and non-processed strains). Through identification, it has been determined that they belong to a new species under the genus *Megasphaera.*

All of the strains can express EC2.8.3.9 enzyme, have a butyrate production pathway with at least 70% integrity, can effectively produce butyric acid, can inhibit the growth of tumor cells, and have certain application value in the prevention and treatment of tumors.

In the present disclosure, utilizing the similar commonality *of Megasphaera* in butyrate production, strains were screened for candidate drugs by further gene and butyrate pathway analysis, thereby improving screening efficiency. It has been verified that all of the screened strains can be effectively used for tumors. The screened bacterial strains can inhibit the activity of histone acetylase by regulating short-chain fatty acids/short-chain fatty acid salts. The bacterial strains achieve anti-tumor effects by producing a short-chain fatty acid/short-chain fatty acid salt (butyrate) alone, or achieve anti-tumor effects or treat other diseases by producing butyrate in combination with upregulating a pro-inflammatory cytokine or a chemokine and/or downregulating an anti-inflammatory factor, preferably, promoting production of one or more pro-inflammatory cytokines by human macrophages, monocytes, peripheral blood mononuclear cells, or monocyte-derived dendritic cells, increasing infiltration of T cells in tumors, or the like.

The anti-tumor drug of the present disclosure is a fully viable bacterial drug, which has a little influence on the steady-state balance of intestinal flora.

The anti-tumor viable bacteria drug of the present disclosure has been proven to be effective against tumors by directly activating immunity.

The method of the present disclosure can be used to efficiently screen out more anti-tumor strains of the genus *Megapshaera,* thereby saving research and development costs.

The microbial preparation provided herein comprises the strain *of Megasphaera* or a metabolite thereof, and has the effect of inhibiting tumor growth. The drug for preventing and/or treating tumors as provided herein comprises the strain *of Megasphaera* isolated and screened from the human intestinal tract or a metabolites thereof, which can be used for treating tumors and has a lower side effect.

The pharmaceutical composition provided herein can achieve a better effect of treating tumors by combining a drug containing the strain of *Megasphaera* with other drugs for treating the tumors. The pharmaceutical composition provided herein can regulate at least one short-chain fatty acid or regulate lactate, and is useful for the treatment of metabolic diseases and tumors.

In addition, the present application provides a drug for preventing or treating a tumor, which comprises a microbial strain of the genus *Megapshaera* and a pharmaceutically acceptable excipient, vehicle, or liquid preparation. Experiments showed that the above drug had preventive and therapeutic effects on lung tumors, liver tumors, pancreatic tumors (the cell lines are KPC and Panc02), glioma (the cell line is GL261), and fibrosarcoma (the cell line is WEHI164).

Specifically, after the pancreatic cancer cell line PanO2 was subcutaneously inoculated into mice to develop tumors, an intervention with the microbial strain of the genus *Megapshaera* exhibited an effect of significantly inhibiting the growth of pancreatic tumors.

After the glioma cell line GL261 was subcutaneously inoculated into mice to develop tumors, an intervention with the microbial strain of the genus *Megapshaera* exhibited an effect of significantly inhibiting the growth of glioma.

After the fibrosarcoma cell line WEHI164 was subcutaneously inoculated into mice to develop tumors, an intervention with the microbial strain of the genus *Megapshaera* exhibited an effect of significantly inhibiting the growth of fibrosarcoma.

In addition, it should be noted that the drug for preventing or treating a tumor can also be a composition, which comprises the microbial strain of the genus *Megapshaera* and a co-drug; alternatively, comprises the microbial strain of the genus *Megapshaera* and an immunosuppressor. The compositions obtained by combining the microbial strain of the genus *Megapshaera* as described above with the co-drug/immunosuppressor also have preventive and therapeutic effects on lung tumors, liver tumors, pancreatic tumors (the cell line is PanO2), glioma (the cell line is GL261), and fibrosarcoma (the cell line is WEHI164).

The co-drug comprises at least one of a radiotherapeutic agent, a targeting drug, a chemotherapeutic agent, a photosensitizing agent, a photothermal agent, an immunotherapeutic agent, and an agent for enhancing cell therapy, wherein:
the chemotherapeutic agent comprises at least one of paclitaxel, camptothecin, 5-fluorouracil, cisplatin, doxorubicin, mitomycin, or epirubicin; the photosensitizing agent comprises at least one of boron dipyrrole, chlorin, or Rose Bengal; and the photothermal agent comprises at least one of indocyanine green, new indocyanine green, or gold nanoparticle rods;
the immunosuppressor comprises at least one of a PD-1 antibody, a CTLA-4 antibody, a PD-L1 antibody, or a PD-L1 inhibitor; the PD-L1 inhibitor is selected from durvalumab, atezolizumab, or avelumab; the PD-1 antibody or PD-L1 antibody is selected from pembrolizumab or nivolumab; and the CTLA-4 antibody is selected from ipilimumab.

Specifically, MNH05026, MNH22004, or MNH27256 in combination with a PD-1 antibody demonstrates the effects of significantly reducing tumor volume and weight and significantly improving the response rate to tumor treatment in an ectopic syngeneic tumor model formed by subcutaneously inoculating the pancreatic cancer cell line PanO2 into mice.

MNH05026, MNH22004, or MNH27256 in combination with a PD-1 antibody demonstrates the effects of significantly reducing tumor volume and weight and significantly improving the response rate to tumor treatment in an ectopic syngeneic tumor model formed by subcutaneously inoculating the glioma cell line GL261 into mice.

MNH05026, MNH22004, or MNH27256 in combination with a PD-1 antibody demonstrates the effects of significantly reducing tumor volume and weight and significantly improving the response rate to tumor treatment in an ectopic syngeneic tumor model formed by subcutaneously inoculating the fibrosarcoma cell line WEHI164 into mice.

As used herein, the term "enhancing" the efficacy of a cell therapy (e.g., CAR-T) refers to a result of administration of the composition of the present disclosure from the cell therapy (e.g., in the case of CAR-T, T-cell-mediated immune response, particularly against a tumor antigen), when compared to the absence of such administration. For example, a subject treated with the composition of the present disclosure and a cell therapy can exhibit greater such therapeutic effects from the cell therapy than a control subject treated with the cell therapy rather than the composition of the present disclosure.

The microbial strain of the genus *Megapshaera,* a metabolite of the microbial strain of the genus *Megapshaera,* and/or a supernatant of the microbial strain of the genus *Megapshaera* in the bacterial strain, microbial agent, drug, and pharmaceutical composition provided in the present disclosure can inhibit tumor growth. Preferably, the inhibiting tumor growth comprises at least one of inhibiting tumor volume growth, inhibiting tumor weight increase, activating systemic immunity in mice, enhancing the efficacy of an immunotherapeutic agent or CAR-T therapeutic agent, and improving tumor response rate.

Preferably, the unit dose of the drug provided herein comprises 9×10⁸ - 2×10⁹ CFU/mL or 1×10⁶ - 2×10⁹ CFU/mg of the microbial strain of the genus *Megapshaera;* and preferably, the unit dose is at least one of 9×10⁸ CFU/mL or 1×10⁹ CFU/mg, 9.2×10⁹ CFU/mL or 1.2×10⁹ CFU/mg, 9.3×10⁹ CFU/mL or 1.4×10⁹ CFU/mg, 9.4×10⁹ CFU/mL or 1.1×10⁹ CFU/mg, 1.2×10⁹ CFU/mL or 1.8×10⁹ CFU/mg, 1.4×10⁹ CFU/mL or 1.6×10⁹ CFU/mg, 1.7×10⁹ CFU/mL or 1.2×10¹⁰ CFU/mg, 1.8×10⁹ CFU/mL or 1.4×10⁹ CFU/mg, 1.6×10⁹ CFU/mL or 1.6×10¹⁰ CFU/mg, 1.8×10¹⁰ CFU/mL or 1.8×10¹⁰ CFU/mg, and 2×10¹⁰ CFU/mL or 2×10¹⁰ CFU/mg.

### BRIEF DESCRIPTION OF THE DRAWINGS

In order to more clearly illustrate the technical solutions in the specific embodiments of the present disclosure or the prior art, the accompanying drawings to be used in the description of the specific embodiments or the prior art will be briefly introduced below. Obviously, the accompanying drawings in the following description relate to some embodiments of the present disclosure. For those of ordinary skill in the art, other drawings can be obtained based on these accompanying drawings without exerting any creative effort.
Fig. 1 shows the colonial morphology of the strains MNH05026, MNH22004, and MNH27256 cultured on anaerobic blood plates for 48 h;
Fig. 2 shows the microscopic morphology of the strains MNH05026, MNH22004, and MNH27256;
Fig. 3 shows the microscopic morphology of the strains MNH05026, MNH22004, and MNH27256 upon Gram-staining;
Fig. 4 shows the microscopic morphology of the strains MNH05026, MNH22004, and MNH27256 upon spore staining;
Fig. 5 shows the results of tolerance of the strains MNH05026, MNH22004, and MNH27256 to different concentrations of NaCl;
Fig. 6 shows the results of tolerance of the strains MNH05026, MNH22004, and MNH27256 to to different pH values;
Fig. 7 shows the results of tolerance of the strains MNH05026, MNH22004, and MNH27256 to different concentrations of bile salts;
Fig. 8 shows the results of culturing the strain MNH05026 in the culture medium API 20;
Fig. 9A shows the 16S rRNA gene phylogenetic tree of the strain MNH05026;
Fig. 9B shows the 16S rRNA gene phylogenetic tree of the strain MNH 22004;
Fig. 9C shows the 16S rRNA gene phylogenetic tree of the strain MNH 27256;
Fig. 10 shows the expression of immunoreactive effector factors in macrophages regulated by the strain MNH05026;
Fig. 11 shows the expression of immunoreactive effector factors in macrophages regulated by the strain MNH22004;
Fig. 12 shows the expression of immunoreactive effector factors in macrophages regulated by the strain MNH27256;
Fig. 13 shows the expression of immunoreactive effector factors in Primary PBMCs regulated by the strain MNH05026;
Fig. 14 shows the expression of immunoreactive effector factors in Primary PBMCs regulated by the strain MNH22004;
Fig. 15 shows the expression of immunoreactive effector factors in Primary PBMCs regulated by the strain MNH27256;
Fig. 16 shows the therapeutic effect of MNH05026 alone or in combination with anti-PD-1 on liver cancer;
Fig. 17 shows the therapeutic effect of MNH05026 in combination with Levatinib on liver cancer;
Fig. 18 shows the therapeutic effect of MNH05026 in combination with anti-PD-1 on lung cancer;
Fig. 19 shows the therapeutic effect of MNH27256 alone and in combination with anti-PD-1 on lung cancer;
Fig. 20 shows an immunoassay of tumor-bearing mice activated by the strain MNH27256;
Fig. 21 is a graph showing the inhibitory effects of MNH22004 and MNH05026 on deacetylase activity; and
FIG. 22 is a graph showing the results of an increase in transcriptional activity of the IFNβ reporter promoted by MNH27256.

### DETAILED DESCRIPTION OF THE INVENTION

The technical solutions of the present disclosure will be clearly and completely described below with reference to the accompanying drawings and specific embodiments. However, those skilled in the art will understand that the embodiments described below are part of the embodiments of the present disclosure, rather than all of the embodiments, and are intended to illustrate the present disclosure only, but should not be regarded as limiting the scope of the present disclosure. All other embodiments obtained by those of ordinary skill in the art based on the embodiments of the present disclosure without exerting any creative effort fall within the scope of protection of the present disclosure. If no specific conditions are specified in the examples, the experiments were carried out according to conventional conditions or conditions recommended by the manufacturer. The reagents or instruments used with no manufacturer indicated are all conventional products that are commercially available.

In some embodiments, the method further comprises administering a prebiotic to the subject. In some embodiments, the prebiotic is fructooligosaccharide, galactooligosaccharide, trans-galactooligosaccharide, xylooligosaccharide, chitooligosaccharide, soy oligosaccharide, gentiooligosaccharide, isomaltooligosaccharide, mannooligosaccharide, maltooligosaccharide, mannooligosaccharide, lactulose, lactosucrose, palatinose, glycosyl sucrose, guar gum, arabic gum, tagatose, amylose, amylopectin, pectin, xylan, or cyclodextrin.

It is known in the art that bacterial species can be classified and identified by traditional classification methods and molecular biology methods. The traditional classification methods include, for example, observation of cell morphology, Gram staining, flagella staining, various metabolic experiments, etc. The molecular biology methods include ribosomal RNA sequencing, determination methods based on whole genome sequencing, etc.

16S rRNA is a ribosomal RNA of prokaryotes. The 16S rRNA gene consists of a variable region and a conserved region. The conserved region is common to all bacteria, while the variable region differs to different extent among different bacteria. By comparing the 16S rRNA gene sequences of bacteria, a phylogenetic tree can be drawn based on their sequence differences and evolutionary distances.

The "identity" between the sequences of two nucleic acid molecules can be determined by a known computer algorithm, such as the "FASTA" program, the GCG program package, BLASTN, or FASTA. The commercially or publicly available program can also be, for example, the DNAStar "MegAlign" program.

With the rapid development of the second-generation and third-generation sequencing technologies, species identification based on whole genome sequencing has become possible, and renders the results of species identification more accurate. The average nucleotide identity (ANI) of bacterial genomes refers to the similarity of homologous genes between two bacterial genomes. The ANI value can be calculated by BLAST or other methods. In the field of bacterial taxonomy, it is generally believed that two strains will be considered as belonging to the same species only when the ANI value reaches 95% or above (Jain C, Rodriguez-R L M, Phillippy A M, et al. High throughput ANI analysis of 90K prokaryotic genomes reveals clear speciesboundaries[J]. Nature Communications, 2018, 9(1): 5114.).

At present, there are various well-established tools for calculating ANI values, such as the local calculation softwares Jspecies (/jspecies) and Gegenees (/documentation. html), and the online calculation tools ANI caculator (http:/enveomics.gatech.edu/), EzGenome (/ezgenome/ani), and ANItools.

By using the above methods, those skilled in the art can determine whether an isolated strain belongs to the new species of the genus *Megasphaera* as discovered by the present inventors. For example, when the average nucleotide identity (ANI) value to the strain deposited with GDMCC No: 62001, GDMCC No: 62000, or GDMCC No: 61999 is at least 95%, e.g., 95.5%, 96%, 96.5%, 97%, 97.5%, 98%, 98.5%, 99%, 99.5%, 99.6%, 99.7%, 99.8%, 99.9%, or 100%, it can be determined that the strains belong to the same species.

The *Megasphaera* sp. as described herein can be prepared into a pharmaceutical composition, for example, by using a pharmaceutically acceptable excipient. The pharmaceutical composition comprises a pharmaceutically effective amount of the strain of the *Megasphaera* sp., such as a strain deposited with GDMCC No: 62001, GDMCC No: 62000, or GDMCC No: 61999. Similarly, the *Megasphaera* species to which the strains deposited with GDMCC No: 62001, GDMCC No: 62000, and GDMCC No: 61999 belong can also be prepared into a pharmaceutical composition, for example, by using a pharmaceutically acceptable excipient, which comprises a pharmaceutically effective amount of the strain of the *Megasphaera* sp.

A suitable pharmaceutically acceptable excipient that can be used is, for example, a carrier, an excipient, a diluent, a lubricant, a wetting agent, an emulsifier, a suspension stabilizer, a preservative, a sweetener, or a flavor. For example, the pharmaceutically acceptable excipient is one or more of lactose, glucose, sucrose, sorbitol, mannose, starch, gum arabic, calcium phosphate, alginate, gelatin, calcium silicate, fine crystalline cellulose, polyvinylpyrrolidone, cellulose, water, syrup, methylcellulose, methylparaben, propylparaben, talc, magnesium stearate, and mineral oil.

The solid MM01 culture medium involved in the present disclosure comprises: peptone 5 g/L, trypsinized casein 5 g/L, yeast powder 10 g/L, beef extract 5 g/L, glucose 5 g/L, K₂HPO₄ 2 g/L, sodium acetate 2 g/L, Tween 80 1 mL/L, hemoglobin 5 mg/L, L-cysteine hydrochloride 0.5 g/L, vitamin K1 1 uL/L, an inorganic salt solution 8 ml/L, which comprises 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride per L of the inorganic salt solution, and agar 15 g/L.

The liquid MM01 culture medium involved in the present disclosure comprises: peptone 5 g/L, trypsinized casein 5 g/L, yeast powder 10 g/L, beef extract 5 g/L, glucose 5 g/L, K₂HPO₄ 2 g/L, sodium acetate 2 g/L, Tween 80 1 mL/L, hemoglobin 5 mg/L, L-cysteine hydrochloride 0.5 g/L, vitamin K1 1 uL/L, and an inorganic salt solution 8 ml/L, which comprises 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride per L of the inorganic salt solution.

The AC liquid medium comprises (per liter): peptone, 20 g; glucose, 5 g; yeast extract, 3 g; beef extract powder, 3 g; and vitamin C, 0.2 g; pH 7.0.

The anaerobic blood plate (purchased from Huankai Microbial) has the following formula (per liter): pancreatic digest of casein 10.0 g; cardiac pancreatic digest 3.0 g; corn starch 1.0 g; pepsin digest of meat 5.0 g; yeast extract powder 5.0 g; sodium chloride 5.0 g; agar 15.0 g; sterile defibrinated goat blood 50-100 mL; and distilled water 1000 mL; final pH 7.3±0.2.

The TSB liquid medium (tryptic soytone broth) comprises the following components (per liter): tryptic soytone 17.0 g; papain hydrolysate of soybean 3.0 g; dipotassium hydrogen phosphate 2.5 g; sodium chloride 5.0 g; and glucose 2.5 g; pH 7.3±0.2.

The culture media as described above can be prepared using conventional methods of formulation and sterilization.

The term "supernatant" in the context of the present disclosure refers to a culture supernatant of the bacterial strain according to the prevent disclosure, optionally comprising compounds and/or cell debris of the strain, and/or metabolites and/or molecules secreted by the strain.

The pharmaceutical composition provided herein can comprise a pharmaceutically acceptable excipient, diluent, or carrier. Acceptable carriers or diluents for therapeutic use are well known in the pharmaceutical field. Examples of suitable carriers include lactose, starch, dextrose, methylcellulose, magnesium stearate, mannitol, sorbitol, etc. Examples of suitable diluents include ethanol, glycerol, and water. The pharmaceutical carrier, excipient, or diluent can be selected depending on the intended route of administration and standard pharmaceutical practice. The pharmaceutical composition can comprise any suitable binder, lubricant, suspending agent, coating agent, solubilizer, or the like as or in addition to the carrier, excipient or diluent. Examples of a suitable binder include starch, gelatin, a natural sugar, and a natural or synthetic gum. The natural sugar is, for example, glucose, anhydrous lactose, free-flowing lactose, β-lactose, or corn sweetener. The natural or synthetic gum is, for example, gum arabic, tragacanth, sodium alginate, carboxymethyl cellulose, or polyethylene glycol. Examples of a suitable lubricant include sodium oleate, sodium stearate, magnesium stearate, sodium benzoate, sodium acetate, sodium chloride, or the like. A preservative, a stabilizer, a dye, or even a flavoring agent can be provided in the pharmaceutical composition. Examples of the preservative include sodium benzoate, sorbic acid, or parabens. An antioxidant or a suspending agent can also be used. An antioxidant or a suspending agent can also be used.

The term "administration" or "administering" generally refers to the route by which a composition (e.g., a pharmaceutical composition) is given to a subject. Examples of routes of administration include oral, rectal, topical, inhalation (nasal), or injection administration. The injection administration includes intravenous (IV), intramuscular (IM), intratumoral (IT), and subcutaneous (SC) administration. The pharmaceutical composition as described herein can be administered in any form by any effective route, including, but not limited to, intratumoral, oral, parenteral, enteral, intravenous, intraperitoneal, topical, transdermal (e.g., using any standard patch), intracutaneous, intraocular, intranasal, local, non-oral, e.g. aerosol, inhalation, subcutaneous, intramuscular, buccal, sublingual, (trans)rectal, vaginal, intraarterial and intrathecal administration. In a preferred embodiment, the pharmaceutical composition as described herein is administered orally, rectally, intratumorally, topically, intravesically, by injection into or near draining lymph nodes, intravenously, by inhalation or aerosol, or subcutaneously. In another preferred embodiment, the pharmaceutical composition as described herein is administered orally, intratumorally, or intravenously.

The "identity" between the nucleic acid sequences of two nucleic acid molecules can be determined as percent identity by a known computer algorithm, such as the "FASTA" program, using default parameters, e.g., in Pearson et al. (Other programs include the GCG package (Devereux, J., et al., Nucleic Acids Research 12(I):387 (1984)), BLASTP, BLASTN, and FASTA, for example, the BLAST function of the NCBI database). Other commercially or publicly available programs include the DNAStar "MegAlign" program.

The term "increase", "enhancement" or "improvement" refers to a change such that the difference between post-treatment and pre-treatment states is at least 10%, 20%, 30%, 40%, 50%, 60%, 70%, 80%, 90%, 2-fold, 4-fold, 10-fold, 100-fold, 10³-fold, 10⁴-fold, 10⁵-fold, 10⁶-fold, and/or 10⁷-fold as appropriate. The characteristics that may be increased include the number of immune cells, bacterial cells, stromal cells, myeloid-derived suppressor cells, fibroblasts, or metabolites; the levels of cytokines; or other physical parameters (such as tumor size).

The term "reduction", "decrease" or "decline" refers to a change such that the difference between post-treatment and pre-treatment states is at least 10%, 20%, 30%, 40%, 50%, 60%, 70%, 80%, 90%, 1/100, 1/1000, 1/10,000, 1/100,000, 1/1,000,000, or undetectable as appropriate. The characteristics that may be reduced include the number of immune cells, bacterial cells, stromal cells, myeloid-derived suppressor cells, fibroblasts, or metabolites; the levels of cytokines; or other physical parameters such as ear thickness (e.g., in animal models of DTH) or tumor size.

As used herein, the term "metabolite" refers to a compound, composition, or molecule that is used as a substrate or product in any cellular or microbial metabolic reaction, or an ion, cofactor, catalyst or nutrient from any cellular or microbial metabolic reaction.

The term "strain" refers to a member of a bacterial species that has a genetic characteristic such that it can be distinguished from closely related members of the same bacterial species. The genetic characteristic can be the absence of all or part of at least one gene, the absence of all or part of at least one regulatory region (e.g., promoter, terminator, riboswitch, or ribosome binding site), the absence ("curing" of at least one natural plasmid), the presence of at least one recombinant gene, the presence of at least one mutated gene, the presence of at least one exogenous gene (a gene from another species), at least one mutated regulatory region (e.g., promoter, terminator, riboswitch, or ribosome binding site), the presence of at least one non-natural plasmid, the presence of at least one antibiotic resistance cassette, or a combination thereof. The genetic characteristic between different strains can be identified by PCR amplification, optionally followed by DNA sequencing of the genomic region of interest or the entire genome. In cases where one strain (as compared to another strain of the same species) acquires or loses antibiotic resistance or acquires or loses biosynthesis ability (e.g., an auxotrophic strain), the strain or nutrient/metabolite can be identified by selection or counter-selection using an antibiotic.

The term "supernatant" in the context of the present disclosure refers to a culture supernatant of the bacterial strain according to the prevent disclosure, optionally comprising compounds and/or cell debris of the strain, and/or metabolites and/or molecules secreted by the strain.

The term "subject" or "patient" refers to any mammal. A subject or patient "in need thereof" as described herein refers to an individual in need of treatment (or prevention) of a disease. The mammal includes humans, laboratory animals (e.g., primates, rats, or mice), farm animals (e.g., cows, sheep, goats, or pigs), and domestic pets (e.g., dogs, cats, or rodents). The subject can be a human. The subject can be a non-human mammal, including, but not limited to, dog, cat, cow, horse, pig, donkey, goat, camel, mouse, rat, guinea pig, sheep, camel, monkey, gorilla, or chimpanzee. The subject or patient can be healthy or can suffer from a metabolic disease at any stage of development.

As used herein, the term "treating" a disease in a subject or "treating" a subject suffering or suspected of suffering from a disease refers to administering to the subject a drug therapy, such as one or more agents, thereby reducing or preventing the deterioration of at least one symptom of the disease. Therefore, in one embodiment, "treating" means inter alia delaying progression, accelerating remission, inducing remission, increasing remission, accelerating recovery, increasing the efficacy of an alternative therapy, decreasing the resistance to an alternative therapy, or a combination thereof.

As used herein, the term "prevention" is well-recognized in the art and, when used in connection with a condition such as local recurrence, is well known in the art. It comprises administering a treatment that reduces the development of, or delays the onset of, a symptom of a medical disorder in a subject as compared to a subject who does not receive the composition. Therefore, the prevention of a cancer includes, for example, reducing the number of detectable tumors in a patient population receiving prophylactic treatment relative to an untreated control population, and/or delaying the occurrence of detectable tumors in a treated population relative to an untreated control population, for example, by a statistically and/or clinically significant amount.

In the present disclosure, the term "highly productive of butyric acid or acetic acid" means that the yield of butyric acid or acetic acid in the short-chain fatty acid (SCFA) assay is ≥ 200 µg/g. Preferably, the yield is ≥ 300 µg/g, ≥ 400 µg/g, ≥ 500 µg/g, ≥ 600 µg/g, ≥ 700 µg/g, ≥ 800 µg/g, ≥ 900 µg/g, ≥ 1000 µg/g, ≥ 1100 µg/g, ≥ 1200 µg/g, ≥ 1300 µg/g, ≥ 1400 µg/g, ≥ 1500 µg/g, or ≥ 1600 µg/g.

In the present disclosure, the terms "first" (e.g., in the first product) and "second" (e.g., in the second product) are merely for the sake of distinction of designation or clarity of expression, and do not indicate a typical order, unless otherwise specified.

The present disclosure develops bacterial strains of the genus *Megasphaera* (MNH05026, MNH22004, and MNH27256) and novel compositions comprising the same. They can be used to treat and prevent cancers, autoimmune and inflammatory diseases, metabolic disorders, neurodegenerative diseases, and mental diseases, especially by directly action by short-chain fatty acids (SCFAs) and/or medium-chain fatty acids (MCFAs), or by regulating the immune system or inhibiting the activity of histone deacetylase (HDAC) through SCFA and/or MCFA mediation to achieve the purpose of treating the above diseases. It has been determined that bacterial strains from the genus *Megasphaera* can efficiently produce a number of SCFAs and MCFAs, including acetic acid, butyric acid, valeric acid, and hexanoic acid. These short-chain fatty acids have been demonstrated to improve symptoms of a variety of diseases, including enhancing the anti-tumor efficacy of immunotherapy, gastrointestinal infectious diseases, inflammatory bowel disease (IBD), metabolic diseases, autoimmune diseases, neurodegenerative diseases, and mental diseases.

Known species under the genus *Megasphaera* include *M. elsdenii, M. stantonii, M. massiliensis, M. indica, M. paucivorans, M. sueciensis, M. micronuciformis, M. hexanoica,* and *M. cerevisiae.* In the species, *M. elsdenii* type strain DSM 20460, *M. indica* type strain NMBHI-10, *M. massiliensis* type strain NP3 and the strain NCIMB 42787 (see CN112601534A), *M. cerevisiae* type strain DSM 20462, *M. paucivorans* type strain DSM 16981, *M. sueciensis* type strain DSM 17042, *M. micronuciformis* type strain DSM 17226, and *M. hexanoica* type strain MH all have the ability to produce short-chain fatty acids, especially butyric acid (see Table 1 in "Megasphaera hexanoica sp. nov., a medium-chain carboxylic acid-producing bacterium isolated from a cow rumen; DOI: 10.1099/ijsem.0.001888").

When drug screening is performed in the present disclosure, the ability of a strain under the genus *Megasphaera* to produce butyric acid is first predicted and analyzed through genomic data analysis at the gene level. A gene element is further identified by determining the key enzyme that can be expressed to produce butyric acid based on the determination of the butyrate production pathway and the integrity of the butyrate production pathway. Bacterial strains containing the gene element/key enzyme (protein) are selected, and the acid production capacity is verified at the experimental data level. Subsequently, the screened candidate strains are subjected to *in vitro* experiments and mouse experiments to verify their effects in tumor suppression. According to this screening logic, the bacterial strains MNH05026, MNH22004, and MNH27256 under the genus *Megasphaera* were screened out for anti-tumor experiments. The experiments proved that the three Megasphaera species of the present disclosure had anti-tumor effects.

At gene level: On the one hand, all of the bacterial strains MNH05026, MNH22004, and MNH27256 of the genus *Megasphaera* of the present disclosure can express butyrate-acetyl CoA-transferase subunit A (EC2.8.3.9 enzyme, see Uniprot.org for specific interpretation). It can be predicted that they have the ability to produce short-chain fatty acids, such as butyric acid. The GENE IDs of EC2.8.3.9 expressed by the bacterial strains MNH05026, MNH22004, and MNH27256 of the present disclosure involve: 650027236, 641897133, 650594268, 642201644, 650018449, 650536170, 2511555023, and 646248671 (for the sequences corresponding to the GENE IDs, see Integrated Microbial Genome (IMG); http://img.jgi.doe.gov).

On the other hand, the inventors summarized the strains under the genus *Megasphaera* with anti-tumor effects that have been recited and confirmed in literatures or patents. The whole genome data of these strains that have been disclosed were extracted from a database for whole genome data analysis. The integrity of the pyruvate pathway (Pyruvate_pathway) and/or the 4-aminobutyrate pathway (4aminobutyrate_pathway) was evaluated for these strains. The inventors found that only 2 strains had a butyrate pathway/pyruvate pathway integrity of less than 70%; but the two strains had a 4-aminobutyrate pathway integrity of higher than 70%. Both MNH22004 and MNH27256 had complete butyrate production pathways (i.e., the butyrate production pathway was 100% complete). MNH05026 had a butyrate pathway integrity of at least 70% and a 4-aminobutyrate pathway integrity of more than 80%.

At experimental data level: By using the method for measuring short-chain fatty acids (SCFAs) as described in Example 3 of the present disclosure, it has been confirmed that MNH05026, MNH22004, and MNH27256 can effectively produce butyric acid (Table 1). The experimental results are consistent with the prediction by genetic analysis. Subsequent experiments have confirmed that the bacterial strains MNH05026, MNH22004, and MNH27256 of the genus *Megashpaera* that can effectively produce butyric acid can effectively inhibit tumors, and can be used to prevent and treat cancers.

**Table 1. Results of SCFA yields for various strains of the present disclosure**

| SCFA (ug/g) Strain | MNH05026 | MNH22004 | MNH27256 |
|---|---|---|---|
| Acetic acid | 251.2136328 | 463.3187346 | 407.3867501 |
| Propionic acid | 42.84714505 | 103.9982679 | 119.2146502 |
| Isobutyric acid | 120.8069044 | 183.4828134 | 198.1745033 |
| Butyric acid | 214.7535761 | 920.3108438 | 1165.035869 |
| Isovaleric acid | 248.9603152 | 320.4344048 | 329.4617645 |

The bacterial strains MNH05026, MNH22004, and MNH27256 as disclosed herein have good ability to produce acetic acid in addition to the ability to produce butyric acid. Acetatic acid and butyric acid in short-chain fatty acids have been shown to regulate anti-tumor immunogenicity and cytokines in an inflammatory process, including interferon γ, interleukin IL-1β, IL-6, interleukin-10 (IL-10), IL-12, IL-23, MCP-1 (CCL-2), MIG (CXCL9), RANTES (CCL5), IP-10 (CXCL-10), and tumor necrosis factor (TNF-α).

In addition to exerting a therapeutic effect through the above pathways, butyric acid (butyrate) is also a metabolite that has been fully verified in the prior art to inhibit HDAC enzyme activity (Human gut bacteria as potent class I histone deacetylase inhibitors in vitro through production of butyric acid and valeric acid, https://doi.org/10.1371/journal.pone.0201073). Studies have also shown that butyric acid and valeric acid can enhance anti-tumor immunity by inhibiting the activity of histone deacetylase and increasing the immunogenic effector function of cytotoxic CD8+ T cells. Butyric acid and valeric acid have been shown to have anti-tumor effects and promote checkpoint inhibitor-mediated immunotherapy and chimeric antigen receptor (CAR) T cell therapy (https://doi.org/10.1038/s41467-021-24331-1; https://doi.org/10.1016/j.biopha.2021.111619; DOI:10.1016/j.immuni.2019.06.002). That is, butyrate-producing bacterial strains that can inhibit HDAC enzyme activity through butyric acid can be expected to be effectively used to inhibit tumors and to treat or prevent cancers.

Therefore, the present disclosure further discloses prevention and/or treatment of tumors or cancers by inhibiting histone acetylase (HDAC) activity with SCFAs and/or MCFAs produced by the strains MNH05026, MNH22004, and MNH27256; preferably, the histone acetylase is class I, class II, class III, or class IV histone acetylase. In addition, an experiment was carried out for verifying the inhibitory effects of metabolites (such as butyric acid) of the three strains MNH05026, MNH22004, and MNH27256 on HDAC.

The bacterial strains MNH05026, MNH22004, and MNH27256 of the genus *Megasphaera* of the present disclosure are butyric acid producers. Based on the known effects of butyric acid, MNH05026 can also reduce the impermeability of the blood-brain barrier that has a neuroprotective effect (Michel and Prat (2016) Ann Transl Med. 4(1): 15).

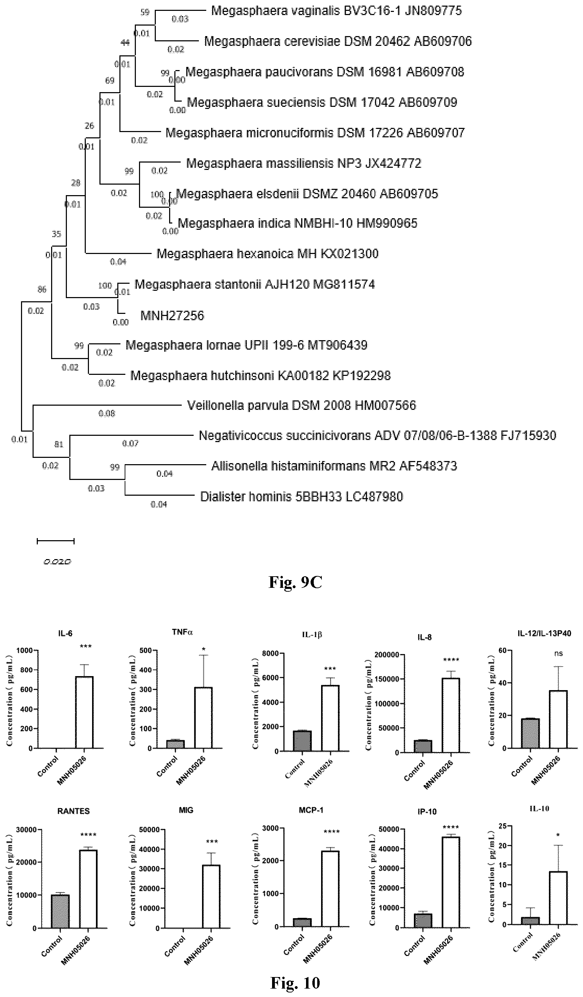

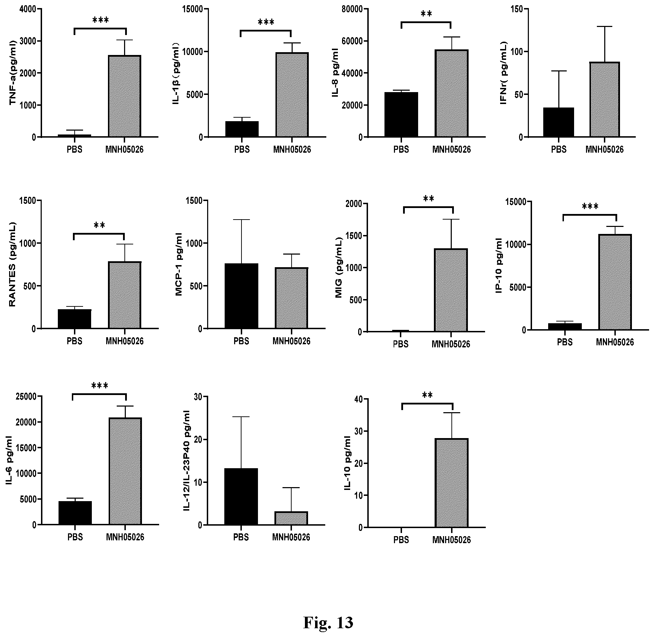

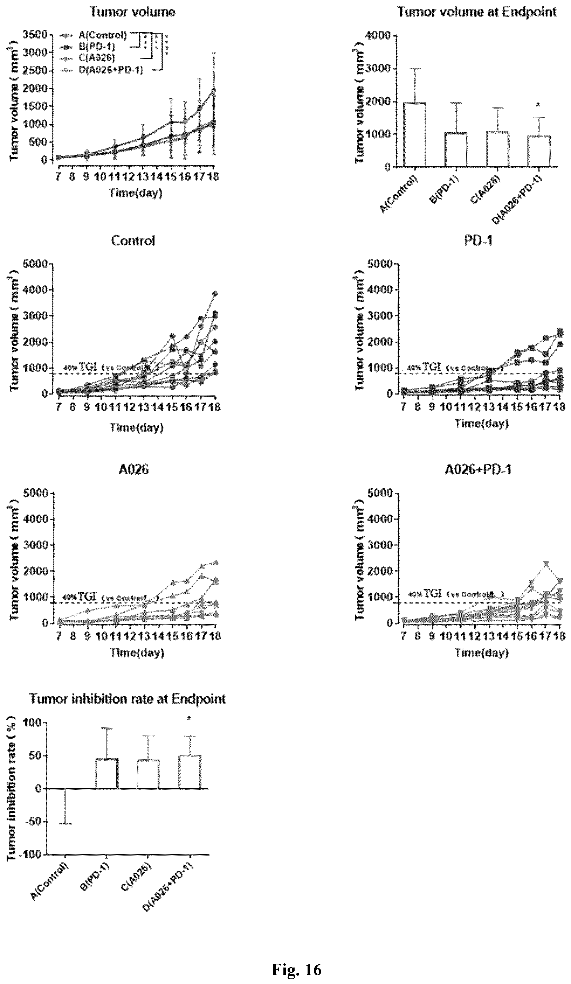

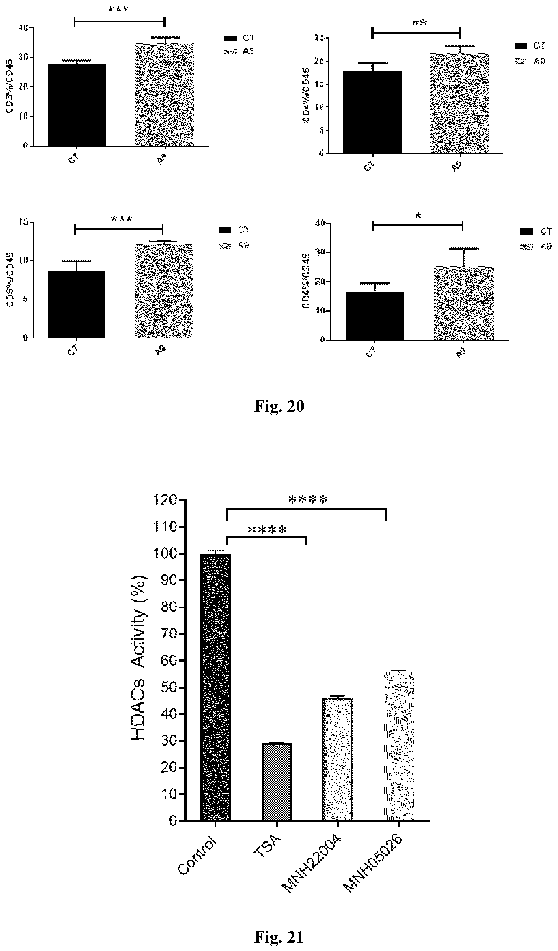

The effective inhibitory effects of MNH05026, MNH22004, and MNH27256 on tumors have been confirmed by experiments in the present disclosure. The anti-tumor effects of MNH05026, MNH22004, and MNH27256 were verified through regulation of the immune activity of macrophages as described in Example 5 (the results are shown in Table 2 and Figs. 10-12); through the immune activity of Primary PBMCs as described in Example 6 (the results are shown in Table 2 and Figs. 13-15); through an animal experiment of anti-tumor treatment as described in Example 7 (the results are shown in Table 2 and Figs. 16-19); through an immunoactivation experiment in tumor-bearing mice as described in Example 8 (the results are shown in Fig. 20); and through an HDAC activity inhibition experiment as described in Example 9 (the results are shown in Fig. 21).

**Table 2. Anti-tumor results of various strains of the present disclosure**

| | MNH050 26 | MNH220 04 | MNH272 56 | Control Group | LPS + IFNγ Positive Control Group |
|---|---|---|---|---|---|
| Average size of tumors dissected from mice (mm³) | 1566 | 1896 | 1367 | 2372 | - |
| Tumor tissue weight after dissection of mice (g) | 1.778 | 1.44 | 1.56 | 2.53 | - |
| Regulation of secretion of IL-1β by Primary PBMCs (pg/ml) | 10543.22 | 12416.2 | 11355.99 | 696.18 | - |
| Regulation of secretion of IFNγ by Primary PBMCs (pg/ml) | 26.222 | 26.22 | 25.30 | 15.27 | - |
| Regulation of the immune activity of macrophages IL-1β (pg/ml) | 4205.225 | 523.24 | 3773.69 | 253.73 | 347.23 |

The *Megasphaera* strains MNH05026, MNH22004, and MNH27256 of the present disclosure are butyric acid producers. Based on the known effects of butyric acid, MNH05026 can also reduce the impermeability of the blood-brain barrier that has a neuroprotective effect (Michel and Prat (2016) Ann Transl Med. 4(1): 15).

The *Megasphaera* strains MNH05026, MNH22004, and MNH27256 of the present disclosure can increase the activation of anti-inflammatory cytokines such as IL-1β, TNF-α, IL-23, and IL-6, upregulate protective immunogenic cytokines, and regulate chemokines. The factor is selected from interferon γ, interleukin IL-1β, IL-6, interleukin-10 (IL-10), IL-12, IL-23, MCP-1 (CCL2), MIG (CXCL9), RANTES (CCL5), IP-10 (CXCL-10), and/or tumor necrosis factor (TNF-α).

The *Megasphaera* strains MNH05026, MNH22004, MNH27256 and compositions thereof of the present disclosure can lead to increased expression of pro-inflammatory molecules such as pro-inflammatory cytokines in PBMCs (see Figs. 13-15). Administration of the pharmaceutical composition of the present disclosure can cause an increased expression of IL-1β in PBMCs. IL-1β is a pro-inflammatory cytokine. The production and secretion of IL-1β are regulated by the inflammasome, a protein complex associated with the activation of an inflammatory response. Since administration of the composition of the present disclosure has been shown to increase the expression of IL-1β, the composition of the present disclosure can be used to treat diseases characterized by a decreased expression of IL-1β.

Administration of the pharmaceutical composition of the present disclosure can result in an increased expression of tumor necrosis factor α (TNF-α). TNF-α is a pro-inflammatory cytokine that is known to participate in multiple signaling pathways to promote cell death. TNF-α initiates apoptosis by binding to its cognate receptor TNFR-1, leading to a cascade of lysis events in the apoptosis pathway.

Administration of the pharmaceutical composition of the present disclosure can result in an increased expression of IL-6 in PBMCs and THP-1. IL-6 is a pro-inflammatory cytokine produced during inflammation and promotes the differentiation of immature CD4+ T cells and the differentiation of CD8+ T cells into cytotoxic T cells. Since administration of the pharmaceutical composition of the present disclosure has been shown to increase the expression of IL-6, the pharmaceutical composition of the present disclosure can be used to treat diseases characterized by a decreased expression of IL-6.

Bettelli, *et al.* reported that IL-6 inhibited the generation of Treg. Since the pharmaceutical composition of the present disclosure increases the expression of IL-6, the pharmaceutical composition of the present disclosure can selectively reduce the number or percentage of Treg by increasing the expression of IL-6. The pharmaceutical composition of the present disclosure can be used for immunostimulation by increasing the expression of IL-6.

In addition, the inventors have also determined that the *Megasphaera* sp. MNH05026, MNH22004, and MNH27256 can alleviate LPS-induced inflammation.

It can be determined from Fig. 13 of the present disclosure that MNH05026 can regulate factors, including: interferon γ, interleukin IL-1β, IL-6, interleukin-10 (IL-10), IL-12, IL-23, MCP-1 (CCL2), MIG (CXCL9), RANTES (CCL5), IP-10 (CXCL-10), and/or tumor necrosis factor (TNF).

In some embodiments, it has been found that the *Megasphaera* strains and compositions thereof of the present disclosure are particularly beneficial in inducing the production of butyric acid, anti-tumor cytokines and chemokines, and inhibiting HDAC activity, thereby achieving the effect of preventing and treating tumors and cancers. In certain embodiments, the *Megasphaera* strains and compositions thereof of the present disclosure, alone or in combination with an immune checkpoint inhibitor, can exert the efficacy of preventing and treating tumors in a preclinical syngeneic mouse tumor model (including gastrointestinal and extra-gastrointestinal tumors). The *Megasphaera* strains and compositions thereof of the present disclosure can be used to treat or prevent cancers, including pan-tumors and solid tumors, but not limited to liver cancer, bile duct cancer, pancreatic cancer, prostate cancer, colorectal cancer, breast cancer, lung cancer, or gastric cancer.

Acetic acid, butyric acid, valeric acid, and hexanoic acid have been shown to mediate autoimmune and inflammatory-related diseases. These effects can be achieved by reducing the synthesis of inflammatory cytokines and increasing immunoregulatory T cells (Tregs) and the production of anti-inflammatory cytokines. MNH05026, MNH22004, and MNH27256 can produce acetic acid, butyric acid, valeric acid, and hexanoic acid. MNH05026, MNH22004, and MNH27256 can increase anti-inflammatory cytokines and attenuate LPS-induced inflammation. Acetic acid and butyric acid have demonstrated protective effects in the treatment and prevention of obesity, type 2 diabetes, non-alcoholic fatty liver disease (NAFLD), cardiac metabolic diseases, and related complications. Such protective effects of acetic acid and butyric acid can be achieved by increasing thermogenesis of brown adipose tissue and browning of white adipose tissue, reducing lipid accumulation in the liver and adipose tissue, enhancing intestinal integrity, and exerting immunomodulatory effects. The inventors have discovered *that Megasphaera* sp. (MNH05026, MNH22004, and MNH27256) can not only produce acetic acid and butyric acid, but also induce the production of anti-inflammatory factors and alleviate LPS-induced diseases.

In some embodiments, administration of a composition comprising *a Megasphaera* strain such as MNH05026, MNH22004, or MNH27256 can reduce HDAC activity.

In some embodiments, the present disclosure provides a method for treating or preventing an inflammatory bowel disease mediated by HDAC activity with a composition comprising a *Megasphaera* sp. such as MNH05026, MNH22004, or MNH27256. Inhibition of HDAC activity has been shown to suppress the production of pro-inflammatory cytokines in the gastrointestinal tract. Therefore, the strains of the genus *Megasphaera* and compositions thereof of the present disclosure can be used to treat inflammatory diseases. In particular, the strains of the genus *Megasphaera* and compositions thereof of the present disclosure can be used to treat or prevent disorders associated with the pathogenesis of increased colonic pro-inflammatory cytokines. In some embodiments, the strains of the genus *Megasphaera* and compositions thereof of the present disclosure are used to treat or prevent inflammatory bowel diseases. In some embodiments, the strains of the genus *Megasphaera* and compositions thereof of the present disclosure are used to treat or prevent ulcerative colitis. In some embodiments, the strains of the genus *Megasphaera* and compositions thereof of the present disclosure are used to treat or prevent Crohn's disease. In certain embodiments, the present disclosure provides the strains of the genus *Megasphaera,* MNH05026, MNH22004, and MNH27256, and compositions thereof, which are useful for treating or preventing inflammatory diseases. In a preferred embodiment, the present disclosure provides the strains of the genus *Megasphaera,* MNH05026, MNH22004, and MNH27256, and compositions thereof, which are useful for treating or preventing colitis.

In certain embodiments of the present disclosure, the bacterial strain in the composition is the strain MNH05026 of the genus *Megasphaera.*

In certain embodiments of the present disclosure, the bacterial strain in the composition belongs to a new species *of Megasphaera,* which is a strain having the following 16S rRNA gene sequence, or a closely related strain, e.g., a strain having a 16S rRNA gene sequence that is at least 95%, 96%, 97%, 98%, 98.63%, 99%, 99.7%, or 99.9% identical to SEQ ID NO: 1. It can also be a strain having a 16S rRNA gene sequence that is at least 95%, 96%, 97%, 98%, 98.63%, 99%, 99.5%, or 99.9% identical to SEQ ID NO: 2. It can also be a strain having a 16S rRNA gene sequence that is at least 95%, 96%, 97%, 98%, 98.5%, 98.63%, 99%, 99.5%, or 99.9% identical to SEQ ID NO: 3. Preferably, the strain used in the present disclosure has a 16S rRNA gene sequence as set forth in SEQ ID NO: 1.

In certain embodiments, the present disclosure provides a food product comprising the above *Megasphaera* strain or a composition thereof.

In addition, the present disclosure provides a method for treating or preventing a disease or disorder mediated by HDAC activity, comprising administering a bacterial strain of the genus *Megasphaera* or a composition thereof. The present disclosure also provides a strain comprising such cells or a biologically pure culture of such cells and compositions thereof. The present disclosure also provides a cell of the genus *Megasphaera,* such as the strains MNH05026, MNH22004, or MNH27256 or a derivative thereof, for use in treatment, in particular treatment of the diseases described herein.

The bacterial strains of the genus *Megasphaera* and compositions thereof of the present disclosure can be used to prevent and/or treat tumors or cancers, including, but not limited to, at least one of liver cancer, colon cancer, rectal cancer, colorectal cancer, lung cancer, breast cancer, cervical cancer, ovarian cancer, pancreatic cancer, bile duct cancer, kidney cancer, fibrosarcoma, metastatic melanoma, cervical cancer, neuroblastoma, lymphoma, leukemia, gastric cancer, hematological malignancy, and prostate cancer. The bacterial strains of the genus *Megasphaera* involved in the present disclosure do not belong to *Megasphaera massiliensis.*

Preferably, the bacterial strains of the genus *Megasphaera* of the present disclosure include species having at least 95% identity to SEQ ID NO: 1, SEQ ID NO: 2, or SEQ ID NO: 3.

The bacterial strains of the present disclosure include *M. elsdenii, M. stantonii, M. indica, M. paucivorans, M. sueciensis, M. micronuciformis, M. hexanoica, M. cerevisiae,* or species strains having at least 95% identity to SEQ ID NO: 1, SEQ ID NO: 2, or SEQ ID NO: 3, which strains can express EC2.8.3.9.

Preferably, the bacterial strains are regulated by GENE ID:650027236, 641897133, 650594268, 642201644, 650018449, 650536170, 2511555023, and 646248671 to express EC2.8.3.9.

The bacterial strains and compositions thereof involved in the present disclosure can regulate at least one short-chain fatty acid or short-chain fatty acid salt, wherein the short-chain fatty acid comprises acetic acid, butyric acid, valeric acid, butyric-valeric acid, or hexanoic acid. When a short-chain fatty acid exerts a therapeutic effect *in vivo,* it usually exists in the form of a short-chain fatty acid salt. Through verification, it has been found that acetate, propionate, and butyrate are the main short-chain fatty acid salts.

The bacterial strains involved in the present disclosure have a 16S rRNA sequence that is at least 95%, 95.5%, 96%, 96.5%, 97%, 97.5%, 98%, 98.5%, 98.65%, 99%, 99.5%, or 99.9% identical to SEQ ID NO: 1.

The bacterial strains and compositions thereof of the present disclosure can be used to treat or prevent metabolic diseases, e.g., at least one of type II diabetes, impaired glucose tolerance, insulin resistance, obesity, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic disease, dyslipidemia, non-alcoholic fatty liver disease (NAFLD), non-alcoholic steatohepatitis (NASH), atherosclerosis, nephropathy, diabetic neuropathy, diabetic retinopathy, dermatosis, dyspepsia, or edema.

The bacterial strains and compositions thereof of the present disclosure are used for treating or preventing autoimmune diseases or inflammatory diseases, such as asthma, arthritis, psoriasis, graft-versus-host disease, inflammatory bowel disease, Crohn's disease, ulcerative colitis, allogeneic transplant rejection, chronic inflammatory bowel disease, systemic lupus erythematosus, psoriasis, rheumatoid arthritis, multiple sclerosis, or Hashimoto's disease; allergic diseases, such as food allergy, hay fever, or asthma; or *Clostridium difficile* infection. The inflammatory diseases include meningitis, myelitis, or TNF-mediated inflammatory diseases, such as inflammatory diseases of the gastrointestinal tract, such as pouchitis, cardiovascular inflammatory diseases, such as atherosclerosis, or inflammatory lung diseases, such as chronic obstructive pulmonary disease.

The bacterial strains and compositions thereof of the present disclosure are used for treating or preventing neurological diseases, such as depression, anxiety, post-traumatic stress disorder, obsessive-compulsive disorder, Parkinson's disease, encephalatrophy, vascular or arteriosclerotic Parkinson's disease, mild cognitive impairment, HIV-related cognitive impairment, or Alzheimer's disease (AD).

The composition involved in the present disclosure comprises a pharmaceutically acceptable carri er.

Preferably, the carrier is selected from one or more of a diluent, a dispersing agent, an excipient, a stabilizer, a lubricant, or a disintegrating agent.

The dosage form of the drug involved in the present disclosure includes any one of a liquid formulation, a solid formulation, a capsule formulation, a sustained-release formulation, and a nano-formulation.

### Example 1. Isolation of the strains MNH05026, MNH22004, and MNH27256:

The intestinal strain MNH05026 involved in the present disclosure was isolated from a stool sample from a healthy female volunteer in Guangzhou City, Guangdong Province. 2-5 g of fresh stool was collected by the donor and placed into a sample collection and storage tube. After being homogenized by shaking, the processed stool sample was placed in an ice box and sent to the laboratory within 24 hours for strain isolation. Physiological saline was dispensed in a biosafety cabinet, 9 mL/tube. Anaerobic blood agar plates with strain isolation medium (Huankai Microbial) were prepared and transferred to an anaerobic workstation 24 hours in advance. The sample information, type of culture medium, isolation date, etc. were marked.

The fresh stool sample was placed in an anaerobic operation station (Don Whitley Scientific H35), and mixed well by shaking on a vortex shaker for 1 min. 1 mL of the sample was pipetted into 9 mL of physiological saline, and mixed well to afford a 10⁻¹ diluted solution, which was then serially diluted to 10⁻⁶ dilution for later use.

The 10⁻⁶ diluted solution was dropped onto anaerobic blood agar plates with isolation medium in an amount of 100 µL/plate, and spread evenly. After the plate surface was dried, the plates were inverted and incubated at 37 °C for 3-5 days. The growth status of the strain in the isolation medium was observed, and a single colony was picked up with a sterilized toothpick for strain purification. The purified strain was anaerobically cultured at 37 °C. The pure culture strain was prepared into a bacterial solution containing 20% glycerol or water, and stored at -86 °C. The strain was identified using a method based on 16S rRNA gene sequence identification.

The intestinal strain MNH22004 involved in the present disclosure was isolated from a stool sample from a healthy female volunteer in Guangzhou City, Guangdong Province. The strain MNH22004 was isolated by the above method.

The intestinal strain MNH27256 involved in the present disclosure was isolated from a stool sample from a healthy female volunteer in Guangzhou City, Guangdong Province. The strain MNH27256 was isolated by the above method.

Deposit of the strains: The strain MNH05026 (abbreviated as A026) was deposited on October 18, 2021 with the Guangdong Microbial Culture Collection Center with a deposit name of *Megasphaera* sp. MNH05026 and a deposit number of GDMCC No: 62001. The deposit address is Guangdong Institute of Microbiology, 5th Floor, No.59 Building, No. 100 Xianlie Zhong Road, Guangzhou. The proposed taxonomic name is *Megasphaera* sp.

The strain MNH22004 of the present disclosure was deposited on October 18, 2021 with the Guangdong Microbial Culture Collection Center with a deposit name *of Megasphaera* sp. MNH22004 and a deposit number of GDMCC NO: 62000. The deposit address is Guangdong Institute of Microbiology, 5th Floor, No.59 Building, No. 100 Xianlie Zhong Road, Guangzhou. The proposed taxonomic name is *Megasphaera* sp.

The strain MNH27256 of the present disclosure was deposited on October 18, 2021 with the Guangdong Microbial Culture Collection Center with a deposit name *of Megasphaera* sp. MNH27256 and a deposit number of GDMCC NO: 61999. The deposit address is Guangdong Institute of Microbiology, 5th Floor, No.59 Building, No. 100 Xianlie Zhong Road, Guangzhou. The proposed taxonomic name is *Megasphaera* sp.

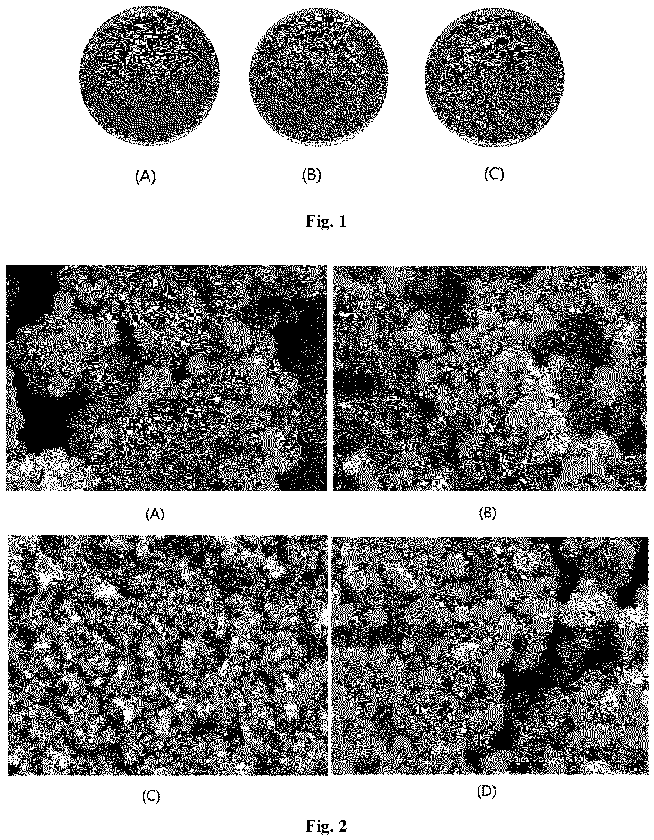

Morphological features: After the strain MNH05026 (abbreviated as A026) was inoculated onto an anaerobic blood plate with culture medium and anaerobically cultured at 37 °C for 48 h, visible colonies were formed on the anaerobic blood plate with culture medium. The colonies were round, pale yellow, opaque, and about 1 mm in diameter, with regular and smooth edges, and with no secretion formed around the colonies. The strain was Gram-negative. Morphological observation under microscope showed that the strain had no spores, no flagella, was non-motile, spherical in shape, and had a diameter of about 5-10 µm. (see Figs. 1A-4A).

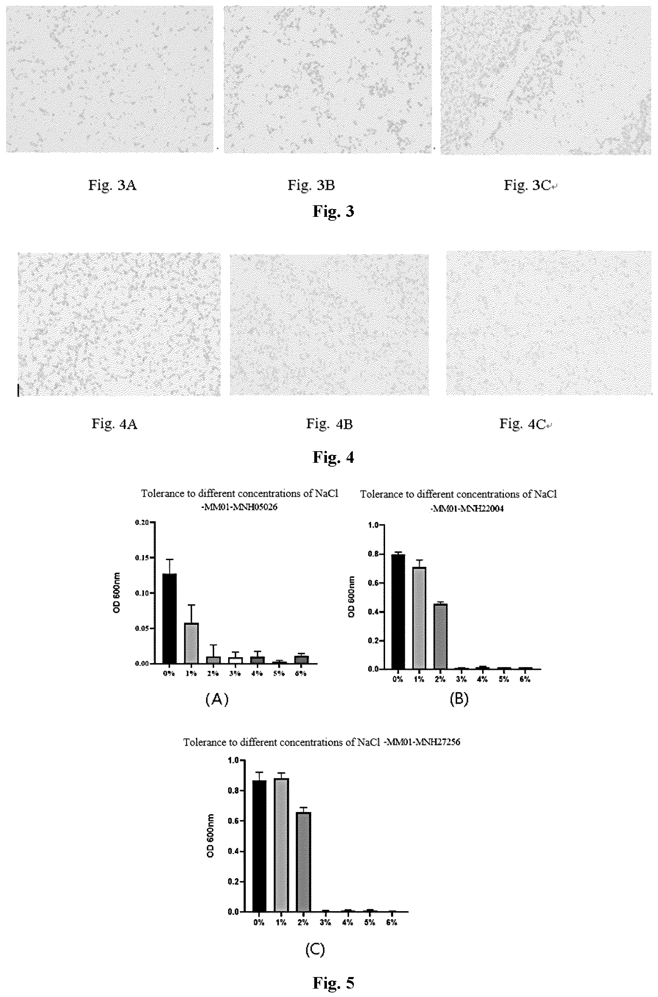

After the strain MNH22004 was inoculated onto an anaerobic blood plate with culture medium and anaerobically cultured at 37 °C for 24 h, visible colonies were formed on the anaerobic blood plate with culture medium. The colonies were round, yellow, opaque, and about 3 mm in diameter, with regular and smooth edges, and with no secretion formed around the colonies (see Fig. 1B for the colonial morphology). The strain was Gram-negative (see Fig. 3B for the microscopic morphology of the strain upon Gram-staining, and Fig. 4B for the microscopic morphology of the strain upon spore staining). Morphological observation under microscope showed that the strain had no spores, no flagella, was non-motile, short rod-shaped, and had a size of about 5 × 10-15 µm (see Fig. 2B for the microscopic colonial morphology).

After the strain MNH27256 was inoculated onto an anaerobic blood plate with culture medium and anaerobically cultured at 37 °C for 24 h, visible colonies were formed on the anaerobic blood plate with culture medium. The colonies were round, yellow, opaque, and about 2 mm in diameter, with regular and smooth edges, and with no secretion formed around the colonies (see Fig. 1C for the colonial morphology). The strain was Gram-negative (see Fig. 3C for the microscopic morphology of the strain upon Gram-staining, and Fig. 4C for the microscopic morphology of the strain upon spore staining). Morphological observation under microscope showed that the strain had no spores, no flagella, was non-motile, spherical in shape, and had a diameter of about 5 µm (see Figs. 2C and 2D for the microscopic colonial morphology).

See Figs. 5-7. Fig. 5 shows the results of tolerance of the strains MNH05026, MNH22004, and MNH27256 to different concentrations of NaCl; in which Fig. 5A shows the results of tolerance of the strain MNH05026 to different concentrations of NaCl; Fig. 5B shows the results of tolerance of the strain MNH22004 to different concentrations of NaCl; and Fig. 5C shows the results of tolerance of the strain MNH27256 to different concentrations of NaCl.

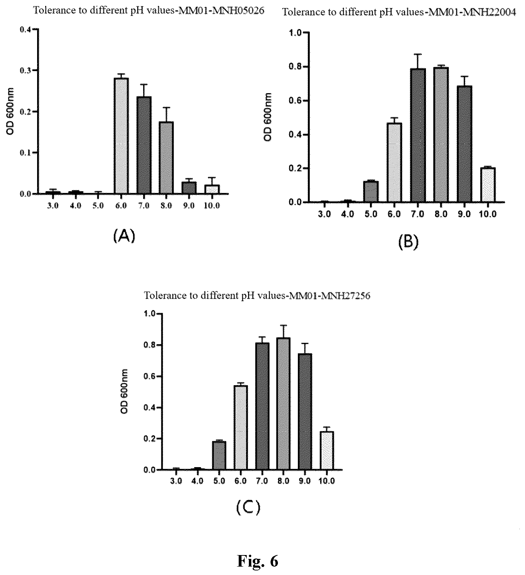

Fig. 6 shows the results of tolerance of the strains MNH05026, MNH22004, and MNH27256 to different pH values; in which Fig. 6A shows the results of tolerance of the strain MNH05026 to different pH values; Fig. 6B shows the results of tolerance of the strain MNH22004 to different pH values; and Fig. 6C shows the results of tolerance of the strain MNH27256 to different pH values.

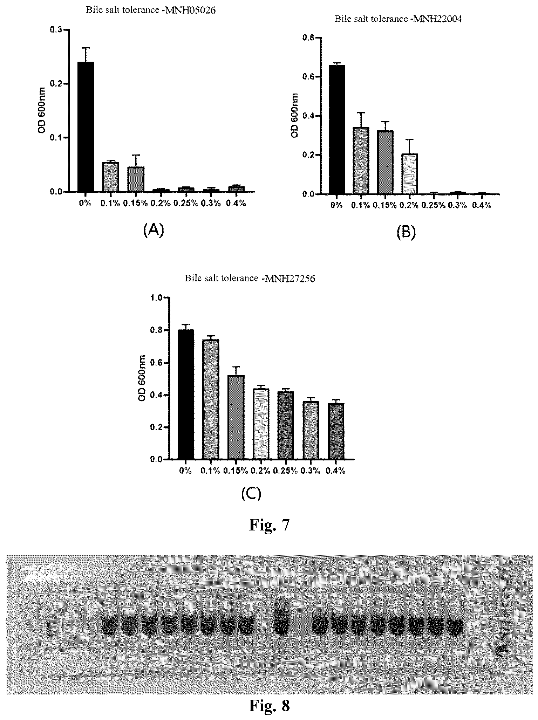

Fig. 7 shows the results of tolerance of the strains MNH05026, MNH22004, and MNH27256 to different concentrations of bile salts; in which Fig. 7A shows the results of tolerance of the strain MNH05026 to different concentrations of bile salts; Fig. 7B shows the results of tolerance of the strain MNH22004 to different concentrations of bile salts; and Fig. 7C shows the results of tolerance of the strain MNH27256 to different concentrations of bile salts.

Physiological and biochemical properties of the strains: The strain MNH05026 grew at a temperature ranging from 30 to 42 °C, with an optimal growth temperature of 37 °C. It could grow at pH 6.0-8.0, with an optimal growth pH of 6.0-7.0 (see Fig. 5A). It could tolerate up to 1% NaCl (see Fig. 6A). The strain MNH05026 could survive and grow at a bile salt concentration in the range of 0-0.15%, and could not grow at a bile salt concentration greater than or equal to 0.2% (see Fig. 7A). The strain MNH05026 could not grow under aerobic conditions, but grew well under anaerobic conditions, and thus belongs to obligate anaerobic bacteria.

The strain MNH22004 grew at a temperature ranging from 30 to 42 °C, with an optimal growth temperature of 37 °C. It could grow at pH 5.0-10.0, with an optimal growth pH of 7.0-8.0. It could tolerate up to 2% NaCl. The strain MNH22004 could survive and grow at a bile salt concentration in the range of 0-0.2%, but its growth trend was weakened as the bile salt concentration was increased. The strain MNH22004 could not grow under aerobic conditions, but grew well under anaerobic conditions, and thus belongs to obligate anaerobic bacteria. The tolerances of the strain to NaCl, pH, and bile salts are shown in Figs. 5B to 7B.

The strain MNH27256 grew at a temperature ranging from 30 to 42 °C, with an optimal growth temperature of 37 °C. It could grow at pH 5.0-10.0, with an optimal growth pH of 7.0-8.0. It could tolerate up to 2% NaCl. The strain MNH27256 could survive and grow at a bile salt concentration in the range of 0-0.4%, but its growth trend was weakened as the bile salt concentration was increased. The strain MNH27256 could not grow under aerobic conditions, but grew well under anaerobic conditions, and thus belongs to obligate anaerobic bacteria. The tolerances of the strain to NaCl, pH, and bile salts are shown in Figs. 5C to 7C.

Physiology and biochemistry of the strains by API 20A test: The test was performed using API 20A reagent strips (BioMérieux) according to the instructions. The strains were anaerobically cultured at 37 °C.

Experimental results: The strain MNH05026 could not grow on API 20A basal culture medium (see Fig. 8). The API 20A test results of MNH22004 and MNH27256 are shown in Table 3 and Table 4, respectively.

**Table 3. API 20A test results of the strain MNH 22004:**

| Test item | Test result | Test item | Test result |
|---|---|---|---|
| IND | Negative | ESC | Negative |
| URE | Negative | GLY | Non-acid producing |
| GLU | Acid-producing | CEL | Non-acid producing |
| MAN | Acid-producing | MNE | Non-acid producing |
| LAC | Non-acid producing | MLZ | Non-acid producing |
| SAC | Non-acid producing | RAF | Non-acid producing |
| MAL | Non-acid producing | SOR | Non-acid producing |
| SAL | Non-acid producing | RHA | Non-acid producing |
| XYL | Non-acid producing | TRE | Non-acid producing |
| ARA | Acid-producing | GEL | Negative |

**Table 4. API 20A test results of the strain MNH27256:**

| Test item | Test result | Test item | Test result |
|---|---|---|---|
| IND | Negative | ESC | Negative |
| URE | Negative | GLY | Non-acid producing |
| GLU | Acid-producing | CEL | Non-acid producing |
| MAN | Acid-producing | MNE | Non-acid producing |
| LAC | Non-acid producing | MLZ | Non-acid producing |
| SAC | Non-acid producing | RAF | Non-acid producing |
| MAL | Non-acid producing | SOR | Non-acid producing |
| SAL | Non-acid producing | RHA | Non-acid producing |
| XYL | Non-acid producing | TRE | Non-acid producing |
| ARA | Acid-producing | GEL | Negative |

Antibiotic sensitivity tests of the strains MNH05026, MNH22004, and MNH27256: Antibiotic sensitivity tests were carried out on the strain MNH05026 by a disk diffusion method. The test results are shown in Table 5. The strain MNH05026 was sensitive to antibiotics such as gentamicin, erythromycin, chloramphenicol, tetracycline, penicillin, ciprofloxacin, trimethoprim-sulfamethoxazole, ampicillin, lincomycin, and ceftriaxone; and the strain MNH05026 was not resistant to any of the antibiotics used. The strain MNH22004 was resistant to penicillin and ampicillin, and sensitive to antibiotics such as gentamicin, erythromycin, chloramphenicol, tetracycline, ciprofloxacin, trimethoprim-sulfamethoxazole, ceftriaxone, and lincomycin. The strain MNH27256 was resistant to penicillin, ampicillin and ceftriaxone, and sensitive to antibiotics such as gentamicin, erythromycin, chloramphenicol, tetracycline, ciprofloxacin, trimethoprim-sulfamethoxazole, and lincomycin.

**Table 5. Results from Antibiotic sensitivity tests of the strains MNH05026, MNH22004, and MNH27256:**

| Antibiotic | Diameter of inhibition zone by MNH05026 (mm) | Diameter of inhibition zone by MNH22004 (mm) | Diameter of inhibition zone by MNH27256 (mm) |
|---|---|---|---|
| Gentamicin (GEN) | 13.97 | 9.00 | 9.37 |
| Erythromycin (ERM) | 15.65 | 21.03 | 9.0 |
| Chloramphenicol (CLM) | 37.02 | 33.29 | 34.45 |
| Tetracycline (TET) | 21.44 | 10.48 | 17.75 |
| Penicillin (PEN) | 29.75 | 0 | 0 |
| Ciprofloxacin (CFX) | 37.66 | 35.42 | 34.69 |
| Trimethoprim-sulfamet hoxazole (T/S) | 10.65 | 17.08 | 12.75 |
| Ampicillin (AMP) | 19.91 | 0 | 0 |
| Lincomycin (LIN) | 38.99 | 11.34 | 8.81 |
| Ceftriaxone (CTR) | 29.42 | 12.78 | 0 |

### Example 2. Identification of the strains MNH05026, MNH22004, and MNH27256:

Genomic DNAs were extracted from fresh cultures of the strains MNH05026, MNH22004, and MNH27256. 16S rRNA gene amplification was performed using the extracted genomic DNAs of the strains as templates.

The primer pair used in the 16S rRNA gene PCR of the present disclosure was as follows:
27F: AGAGTTTGATCMTGGCTCAG, and 1492R: TACGGYTACCTTGTTACGACTT.

The PCR procedure was as follows: PCR reaction cycles: Pre-denaturation: 94 °C, 4 min; Denaturation: 94 °C, 50 sec; Annealing: 52 °C, 40 sec; Extension: 72 °C, 70 sec; Final extension: 72 °C, 10 min; 36 cycles.

After the PCR amplification was completed, the PCR products were purified and sent to Genewiz Co. for 16S rRNA gene sequencing. The 16S rRNA gene sequences of the strains returned after sequencing were submitted to the NCBI Basic Local Alignment Search Tool for 16S rRNA gene analysis to confirm the strain classification information.

The 16S rRNA gene amplification product of the strain MNH05026 was sent for sequencing to obtain the 16S rRNA gene sequence (SEQ ID NO: 1). The determined sequence was analyzed against the data in GenBank by BLAST. The alignment results showed that the strain MNH05026 belonged to the genus *Megasphaera,* and the strains with the highest similarity thereto were *Megasphaera hexanoica* (96.50%) and *Megasphaera elsdenii* (94.43%). The 16S rRNA gene sequence similarities between the strain MNH05026 and other strains of the genus *Megasphaera* were all less than 94.30%. According to Kim, *et al.* (2014), through statistical analysis of thousands of genomic and 16S rRNA gene sequences, it has been found that when the similarity between the 16S rRNA gene sequences of two strains is less than 98.65%, they can be judged to belong to different species. According to this judgment criterion, the experimental strain MNH05026 of the present disclosure represents a new species of the genus *Megasphaera.*

Based on the same analysis, the determined sequences of MNH22004 (SEQ ID NO: 2) and MNH27256 (SEQ ID NO: 3) were analyzed against the data in GenBank by BLAST. The alignment results showed that both MNH22004 (SEQ ID NO: 2) and MNH27256 (SEQ ID NO: 3) belonged to the genus *Megasphaera.* The strains with a higher similarity to MNH22004 were *Megasphaera micronuciformis* (90.92%), *Caecibacter massiliensis* (90.63%), *Megasphaera hexanoica* (90.63%), *Megasphaera elsdenii* (90.61%), *Megasphaera paucivorans* (90.59%), *Megasphaera indica* (90.29%), and *Megasphaera sueciensis* (90.18%). The 16S rRNA gene sequence similarities between the strain MNH22004 and other strains of the genus *Megasphaera* were all less than 90%. The lower (<95%) 16S rRNA gene sequence similarity indicated that the strain MNH22004 might represent a new species of the genus *Megasphaera.* The strain with the highest similarity to MNH27256 was *Megasphaera stantonii* AJH120 (MG811574), and their 16S rRNA gene similarity was 98.41%. The 16S rRNA gene sequence similarities between the strain MNH27256 and other strains of the genus *Megasphaera* were all less than 94%, indicating that MNH27256 might represent a new species of the genus *Megasphaera.*

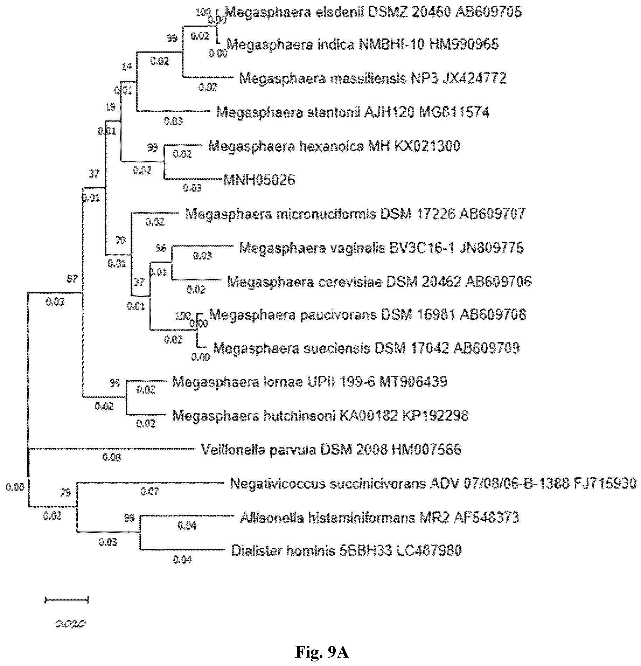

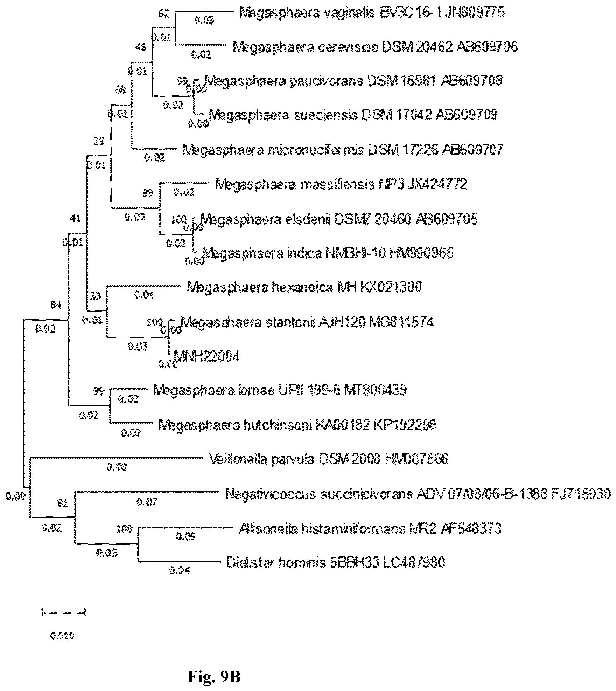

The 16S rRNA gene sequences of MNH05026, MNH22004, and MNH27256 were compared with those of related strains of the genus *Megasphaera* and closely related species retrieved from databases such as GenBank (Table 6). The whole-genome data of the strains *Megasphaera massiliensis* and *Megasphaera elsdenii* under the genus *Megasphaera* were downloaded from NCBI to compare with those of MNH05026, MNH22004, and MNH27256 of the present disclosure (Table 7). Moreover, phylogenetic trees were constructed. As can be seen from the phylogenetic trees, the strain MNH05026 and the *Megasphaera hexanoica* strain collectively form a separate branch (the Bootstrap support value is 99). The strain MNH22004 and the *Megasphaera stantonii* AJH120T strain collectively form a separate branch (the Bootstrap support value is 100). The strain MNH27256 and the *Megasphaera stantonii* AJH120 strain collectively form an evolutionary branch (the Bootstrap support value is 100). Multiple sequence alignment was performed between MNH05026, MNH 22004 and MNH 27256 and the type strains with a relatively high 16S rRNA gene sequence similarity obtained from the NCBI database, and then phylogenetic trees were constructed by the maximum likelihood method using the software MEGA 5. See Fig. 9a for MNH05026, Fig. 9b for MNH22004, and Fig. 9c for MNH27256. The phylogenetic tree nodes in the figures only display the Bootstrap values greater than 50%, and the superscript "T" indicates the type strains.

Fig. 9A shows the 16S rRNA gene phylogenetic tree of the strain MNH05026.

Fig. 9B shows the 16S rRNA gene phylogenetic tree of the strain MNH22004.

Fig. 9C shows the 16S rRNA gene phylogenetic tree of the strain MNH27256.

**Table 6. Comparison results of 16S rRNA gene sequences:**

| 16S comparison results | | |
|---|---|---|
| | Reference sequence | Identity |
| MNH05026 | Megasphaera_elsdenii | 94.06 |
| MNH05026 | Megasphaera_massiliensis | 93.028 |
| MNH05026 | Megasphaera_massiliensis_PTA_126770 | 93.495 |
| MNH05026 | MNH22004 | 93.238 |
| MNH05026 | MNH27256 | 92.729 |
| MNH22004 | MNH05026 | 93.238 |
| MNH22004 | MNH27256 | 98.362 |

**Table 7. Pairwise comparison with Megasphaera elsdenii and Megasphaera massiliensis:**

| Whole-genome comparison results | | | |
|---|---|---|---|
| | Name of reference strain | Reference sequence | ANI |
| MNH05026 | A representative strain of Megasphaera_massiliensis | GCF_000455225.1_Megasphaera_ massiliensis_genomic.fna | 76.8594 |
| MNH05026 | A representative strain of Megasphaera elsdenii | GCF_003006415.1_ASM300641v 1_genomic.fna | 77.8759 |
| MNH05026 | Megasphaera elsdenii DSM 20460 | GCF_003010495.1_ASM301049v 1 genomic.fna | 77.6074 |
| MNH05026 | A randomly selected strain of Megasphaera_massiliensis | GCF_020181515.1_ASM2018151 v1_genomic.fna | 76.8902 |
| MNH22004 | A representative strain of Megasphaera_massiliensis | GCF_Megasphaera_ massiliensis_genomic.fna | 77.7799 |
| MNH22004 | A representative strain of Megasphaera elsdenii | GCF_003006415.1_ASM300641v 1_genomic.fna | 78.9286 |
| MNH22004 | Megasphaera elsdenii DSM 20460 | GCF_003010495.1_ASM301049v 1_genomic.fna | 78.9237 |
| MNH22004 | A randomly selected strain of Megasphaera_massiliensis | GCF_020181515.1_ASM2018151 v1_genomic.fna | 77.9763 |
| MNH27256 | A representative strain of Megasphaera_massiliensis | GCF_000455225.1_Megasphaera_ massiliensis_genomic.fna | 77.7611 |
| MNH27256 | A representative strain of Megasphaera elsdenii | GCF_003006415.1_ASM300641v 1_genomic.fna | 78.885 |
| MNH27256 | Megasphaera elsdenii DSM 20460 | GCF_003010495.1_ASM301049v 1_genomic.fna | 78.8788 |
| MNH27256 | A randomly selected strain of Megasphaera_massiliensis | GCF_020181515.1_ASM2018151 v1_genomic.fna | 77.9586 |
| MNH05026 | MNH27256 | / | 76.8949 |
| MNH05026 | MNH22004 | / | 76.8087 |
| MNH22004 | MNH27256 | / | 97.5967 |

According to Table 7, MNH05026, MNH22004, and MNH27256 are not strains belonging to *Megasphaera massiliensis* and *Megasphaera elsdenii,* and have significant genomic differences from *Megasphaera massiliensis* and *Megasphaera elsdenii.*

It can be further known from Table 6 and Table 7 that MNH05026 (SEQ ID NO: 1), MNH22004 (SEQ ID NO: 2), and MNH27256 (SEQ ID NO: 3) belong to the genus *Megasphaera.* According to the criterion that ANI > 95% indicates the same species, the genomic correlation analysis based on average nucleotide identity (ANI) in the present application shows that MNH05026, (MNH22004 or MNH27256), *Megasphaera massiliensis,* and *Megasphaera elsdenii* do not belong to the same species, while MNH22004 and MNH27256 are two different strains of the same species. That is, MNH05026 represents a new species under the genus *Megasphaera,* and MNH22004 and MNH27256 belong to the same new species under the genus *Megasphaera.*

Genomic Analysis of MNH05026, MNH22004, and MNH27256:
The genome of the original strain MNH05026 was fragmented by ultrasonication with a fragmentation length range of ~350 bp, and then an Illumina sequencing library was constructed using a standard DNA library construction kit (NEB UltraTM). The constructed sequencing library was subjected to paired-end 150 bp sequencing using NovaSeq (Illumina). The sequencing yielded 1.46 Gbp data, of which Q20 accounted for 97.08%. MNH22004 and MNH27256 were sequenced using the same method to obtain 1.33 Gbp and 1.39 Gbp data, respectively, of which Q20 accounted for 97.27% and 96.95%, respectively.

The raw data of genome sequencing were filtered using fastp (version: 0.20.0), with the following filtration parameter: "--poly_g_min_len 10 --poly_x_min_len 10 -q 15 -u 40 -n 5 -1 50". The filtered raw data were subjected to genome assembly using SPAdes (version: v3.14.0), with the following assembly parameter "--isolate --cov-cutoff 10". The genome assembly resulted in total gene lengths of 2.79 Mbp, 2.58 Mbp and 2.59 Mbp, N50 lengths of 59.8 kbp, 157.9 kbp and 92.9 kbp, and GC contents of 50.46%, 52.86% and 52.85% for MNH05026, MNH22004 and MNH27256, respectively.

Genomic genes were subjected to genomic gene prediction and analysis using the prokaryotic analysis software genome annotation process prokka (version: 1.14.5), with the following parameter: "--gcode 11 --evalue 1e-09". A total of 2568 CDS sequences were obtained for MNH05026 by the prediction, with an average CDS sequence length of 957 bp. The type strain with the highest genome similarity was *Megasphaera elsdenii,* with an average nucleotide identity (ANI) of 78.58% and a gene coverage of 30.27%. Therefore, it was identified as a new species of the genus *Megasphaera.* A total of 2353 CDS sequences were obtained for MNH22004 by the prediction, with an average CDS sequence length of 967 bp. The type strain with the highest genome similarity to the experimental strain MNH22004 was *Megasphaera elsdenii,* with an average nucleotide identity (ANI) of 79.49% and a gene coverage of 42.37%. Further based on the 16S rRNA gene analysis results of the strain MNH22004, it was confirmed that the strain MNH22004 might represent a new species of the genus *Megasphaera.* A total of 2335 CDS sequences were obtained for MNH27256 by the prediction, with an average CDS sequence length of 975 bp. The type strain with the highest genome similarity to the experimental strain MNH27256 was *Megasphaera elsdenii,* with an average nucleotide identity (ANI) of 79.38% and a gene coverage of 41.04%.

Potential antibiotic resistance genes in the genome were analyzed using the RGI process (version: 4.2.2), in which the antibiotic resistance gene database was CARD (version: 3.0.0, https://card.mcmaster.ca/analyze/rgi). No antibiotic resistance gene was identified for MNH05026. The drug resistance gene information obtained from alignment of MNH22004 and MNH27256 is shown in Table 8.

**Table 8. Information of drug resistance genes**

| Strain gene | Resistance gene | Gene Name | Alignment identity (%) |
|---|---|---|---|
| MNH22004_00592 | ARO:3004442 | tet(W/N/W) | 95.3 |
| MNH27256_01102 | ARO:3002837 | lnuC | 98.17 |

Potential virulence factors and related genes in the genome were analyzed by using NCBI blastp (version: 2.7.1+) to compare against the virulence factor database VFDB (http://www.mgc.ac.cn/cgi-bin/VFs/v5/main.cgi, updated on September 19, 2019). Detailed comparison results are shown in Table 9.

**Table 9. List of potential virulence genes of MNH05026, MNH22004, and MNH27256**

| Strain gene | VFDB gene | Gene Name | Alignment identity (%) |
|---|---|---|---|
| MNH05026_00068 | VFG001967 | glf | 61.345 |
| MNH05026_00534 | VFG011430 | acpXL | 66.667 |
| MNH05026_00865 | VFG048797 | ugd | 69.33 |
| MNH05026_01427 | VFG000077 | clpP | 66.492 |
| MNH05026_01568 | VFG001855 | htpB | 61.027 |
| MNH05026_02287 | VFG002377 | ddhA | 70.968 |
| MNH22004_00028 | VFG000077 | clpP | 65.426 |
| MNH22004_00201 | VFG001855 | htpB | 61.333 |
| MNH22004_01610 | VFG011430 | acpXL | 66.667 |
| MNH22004_02389 | VFG001967 | glf | 61.236 |
| MNH27256_00034 | VFG011430 | acpXL | 66.667 |
| MNH27256_00830 | VFG048797 | ugd | 69.33 |
| MNH27256_01638 | VFG000077 | clpP | 65.426 |
| MNH27256_02035 | VFG001855 | htpB | 61.333 |

Potential secondary metabolism gene clusters in the genome were analyzed using antiSMASH5 (version: 5.1.1). No secondary metabolism gene cluster was identified for MNH05026. The alignment results of MNH22004 and MNH05026 are shown in Table 10.

**Table 10. Potential secondary metabolism gene clusters of MNH22004 and MNH05026**

| Strain | Gene cluster range | Type | From | To | Most similar known gene cluster | Similarity |
|---|---|---|---|---|---|---|
| MNH22004 | Region 13.1 | sactipeptide | 71170 | 88519 | ---- | ---- |
| MNH27256 | Region 11.1 | sactipeptide | 67838 | 85557 | ---- | ----- |

Potential primary metabolism gene clusters in the genome were analyzed using gutSMASH5 (version: 1.0.0). Detailed alignment results are shown in Tables 11-A, 11-B, and 11-C.

**Table 11-A. List of potential primary metabolism gene clusters of MNH05026:**

| Gene cluster range | Type | From | To | Most similar known gene cluster | Abbreviation | Similarity |
|---|---|---|---|---|---|---|
| Region 1.1 | Flavoenzyme_lipids_cat abolism | 18591 6 | 209530 | ---- | ---- | ---- |
| Region 2.1 | OD_fatty_acidsPFOR_I I_pathway | 91245 | 126006 | PFOR II pathway B. thetaiotaomicron | PFORII | 100% |
| Region 4.1 | OD_AA_metabolismPu trescine2spermidinePF OR_II_pathwayTPP_A A metabolism | 1 | 60249 | Arginine2putrescine R. gnavus | PUTR | 100% |
| Region 6.1 | PFOR_II_pathway | 79302 | 92953 | PFOR II pathway B. thetaiotaomicron | PFORII | 100% |
| Region 9.1 | PFOR_II_pathway | 44299 | 67814 | PFOR II pathway B. thetaiotaomicron | PFORII | 100% |
| Region 14.1 | OD_fatty_acids | 40990 | 59886 | ---- | ---- | ---- |
| Region 17.1 | PFOR_II_pathway | 32903 | 56181 | PFOR II pathway B. thetaiotaomicron | PFORII | 100% |
| Region 20.1 | porA | 1 | 15133 | porA C. sporogenes | POR | 40% |
| Region 26.1 | Others_HGD_unassigne | 12019 | 36323 | ---- | ---- | ---- |
| Region 30.1 | OD_fatty_acids | 1763 | 30762 | ---- | ---- | ---- |
| Region 31.1 | PFOR_II_pathway | 1 | 15154 | PFOR II pathway B. thetaiotaomicron | PFORII | 100% |
| Region 48.1 | Rnf_complex | 1 | 15795 | Rnf complex C. sporogenes | RNF | 83% |
| Region 54.1 | OD_fatty_acids | 1 | 13291 | Aminobutyrate to butyrate C. pasteurianum | AMINOBUT | 20% |

**Table 11-B. Potential primary metabolism gene clusters of MNH22004:**

| Gene cluster range | Type | From | To | Most similar known gene cluster | Abbreviation | Similarity |
|---|---|---|---|---|---|---|
| Region 1.1 | PFOR_II_pathway | 125150 | 148671 | PFOR II pathway B. thetaiotaomicr on | PFORII | 100% |
| Region 2.1 | porA | 31221 | 53807 | porA C. sporogenes | POR | 60% |
| Region 3.1 | Flavoenzyme_lipids_catab olism | 22755 | 52907 | ---- | ---- | ---- |
| Region 4.1 | OD_fatty_acidsPFOR_II_ pathwayFlavoenzyme_lipi ds_catabolismacrylate2pro pionateFlavoenzyme_AA_ peptides_catabolism | 39306 | 88445 | Leucine reductive branch C. difficile | LEU | 50% |
| Region 6.1 | fatty_acids-unassigned | 6397 | 30453 | ---- | ---- | ---- |
| Region 6.2 | Rnf_complex | 124441 | 149514 | Rnf complex C. sporogenes | RNF | 100% |
| Region 6.3 | Putrescine2spermidineOth ers_HGD_unassigned | 154172 | 175736 | ---- | ---- | ---- |
| Region 7.1 | TPP_AA_metabolism | 124930 | 149863 | ---- | ---- | ---- |
| Region 11.1 | proline2aminovalerate | 56222 | 80482 | Proline to aminovalerate C. sticklandii | AMI | 58% |
| Region 14.1 | OD_lactate_relatedPFOR_ II_pathway | 21761 | 61320 | PFOR II pathway B. thetaiotaomicr on | PFORII | 100% |
| Region 23.1 | Arginine2putrescine | 1 | 21392 | ---- | ---- | ---- |

**Table 11-C. Potential primary metabolism gene clusters of MNH27256:**

| Gene cluster range | Type | From | To | Most similar known gene cluster | Abbrevi ation | Similarit y |
|---|---|---|---|---|---|---|
| Region 1.1 | Others_HGD_unassi gnedPutrescine2sper midine | 46325 | 71732 | ---- | ---- | ---- |
| Region 1.2 | Rnf_complex | 76391 | 101464 | Rnf complex C. sporogenes | RNF | 100% |
| Region 2.1 | aminobutyrate2Butyr ate | 93401 | 119769 | Aminobutyrate to butyrate C. pasteurianum | AMINO BUT | 60% |
| Region 2.2 | OD_fatty_acids | 165601 | 201319 | PFOR II pathway B. thetaiotaomicron | PFORII | 100% |
| Region 4.1 | Flavoenzyme_lipids_ catabolism | 147516 | 177673 | ---- | ---- | ---- |
| Region 8.1 | TPP_AA_metabolis m | 6896 | 31829 | ---- | ---- | ---- |
| Region 10.1 | OD_fatty_acidsPFO R_II_pathwayFlavoe nzyme_lipids_catabo lismacrylate2propion ateFlavoenzyme_AA _peptides_catabolism | 16158 | 65296 | Leucine reductive branch C. difficile | LEU | 50% |
| Region 19.1 | PFOR_II_pathway | 1 | 19449 | PFOR II pathway B. thetaiotaomicron | PFORII | 100% |
| Region 25.1 | fatty_acids-unassigne d | 9272 | 31951 | ---- | ---- | ---- |
| Region 26.1 | PFOR_II_pathway | 18125 | 31920 | PFOR II pathway B. thetaiotaomicron | PFORII | 100% |
| Region 30.1 | Arginine2putrescine | 1 | 18883 | ---- | ---- | ---- |
| Region 32.1 | TPP_AA_metabolis m | 1 | 17570 | Succinate to propionate B. theta | PRO | 16% |

The ability of the strains to produce butyric acid was evaluated. Using the genes related to the butyrate production pathway as described in the prior art (Vital M, Howe C, Tiedje M. Revealing the Bacterial Butyrate Synthesis Pathways by Analyzing (Meta) genomic Data[J]. Mbio, 2014, 5(2): 1-11) as a reference database, the genome sequences of the strains were aligned against the reference database using NCBI blastp (version: 2.7.1+). Detailed alignment results are shown in Table 12. Then, the integrity of the butyrate production pathway was calculated. Through calculation, it was found that the integrity of the butyrate production pathway was 80% for the strain MNH05026, and 100% for MNH27256 and MNH22004.

**Table 12. List of potential butyrate-producing genes of MNH05026, MNH22004, and MNH27256:**

| Strain gene | Name of butyrate production pathway | Gene name | Alignment identity (%) |
|---|---|---|---|
| MNH05026_00402 | 4aminobutyrate | AbfD-Isom | 85.507 |
| MNH05026_00885 | 4aminobutyrate | AbfH | 78.919 |
| MNH05026_01758 | Pyruvate | Bcd | 85.827 |
| MNH05026_02471 | Pyruvate | But | 73.708 |
| MNH05026_01756 | Pyruvate | EtfA | 83.582 |
| MNH05026_01757 | Pyruvate | EtfB | 86.617 |
| MNH05026_00254 | Pyruvate | Hbd | 76.157 |
| MNH22004_00304 | 4aminobutyrate | AbfD-Isom | 90.476 |
| MNH22004_00694 | Pyruvate | Bcd | 87.566 |
| MNH22004_01262 | Pyruvate | But | 75.283 |
| MNH22004_01737 | Pyruvate | Cro | 80.385 |
| MNH22004_01359 | Pyruvate | EtfA | 84.615 |
| MNH22004_01358 | Pyruvate | EtfB | 88.015 |
| MNH22004_01736 | Pyruvate | Hbd | 83.566 |
| MNH22004_01042 | Pyruvate | Thl | 84.184 |
| MNH27256_00316 | 4aminobutyrate | 4Hbt | 81.628 |
| MNH27256_00317 | 4aminobutyrate | AbfD-Isom | 91.304 |
| MNH27256_01293 | Pyruvate | Bcd | 87.831 |
| MNH27256_01143 | Pyruvate | But | 75.51 |
| MNH27256_00660 | Pyruvate | Cro | 80.46 |
| MNH27256_00841 | Pyruvate | EtfA | 84.615 |
| MNH27256_00840 | Pyruvate | EtfB | 88.015 |
| MNH27256_01842 | Pyruvate | Hbd | 83.566 |
| MNH27256_02128 | Pyruvate | Thl | 83.929 |

### Fatty acid composition analysis of the strains MNH05026, MNH22004, and MNH27256:

The strain MNH05026 (abbreviated as A026) was inoculated onto a TSA plate and anaerobically cultured at 37 °C for 48 h. Then, the bacterial cells were collected and subjected to fatty acid extraction and methylation treatment. A fatty acid composition analysis was performed for the strain MNH05026 using a fully automatic bacterial identification system from MIDI Co., US (Microbial ID, Inc., Newark, Del) (Kroppenstedt, 1985; Meier, *et al.,* 1993) (see Table 13A). MNH22004 and MNH27256 were analyzed using the same method, and the results are shown in Tables 13B and 13C, respectively.

The results showed that the main fatty acids (>10%) of MNH05026 were 12-carbon saturated fatty acid (C12:0, 11.98%) and 19-carbon unsaturated fatty acid (C18:1 CIS 9 FAME 11.28%). Other fatty acids and their contents are detailed in Table 13A. The main fatty acids (>10%) of MNH22004 were 12-carbon saturated fatty acid (C12:0, 14.43%), 16-carbon saturated fatty acids (C16:0 13.87%), 3-hydroxytetradecane saturated fatty acid (C14:0 3OH 11.84%), and 18-carbon monounsaturated fatty acid (C18:1 CIS 9 10.14%). Other fatty acids and their contents are detailed in Table 13B. The main fatty acids (>10%) of MNH27256 were 12-carbon saturated fatty acid (C12:0, 15.54%), 18-carbon monounsaturated fatty acid (C18:1 CIS 9 15.03%), and 3-hydroxytetradecane saturated fatty acid (C14:0 3OH 13.22%). Other fatty acids and their contents are detailed in Table 13C.

**Table 13A. Fatty acid components of the strain MNH05026:**

| RT | Response | Ar/Ht | RFact | ECL | Peak Name | Percent | Comment1 | Comment2 |
|---|---|---|---|---|---|---|---|---|
| 1.627 | 9.323E+8 | 0.044 | ---- | 6.974 | SOLVENT PEAK | ---- | < min rt | |
| 2.093 | 1078 | 0.029 | ---- | 7.868 | ---- | ---- | < min rt | ---- |
| 2.587 | 1134 | 0.029 | ---- | 8.815 | ---- | ---- | < min rt | ---- |
| 3.162 | 500 | 0.024 | ---- | 9.917 | ---- | ---- | ---- | ---- |
| 3.206 | 412 | 0.025 | 1.155 | 10.000 | 10:0 FAME | 0.18 | ECL deviates | Reference 0.003 |
| 3.864 | 1188 | 0.027 | ---- | 10.919 | ---- | ---- | ---- | ---- |
| 3.923 | 3853 | 0.031 | 1.098 | 11.000 | 11:0 FAME | 1.57 | ECL deviates | Reference 0.002 |
| 4.497 | 363 | 0.031 | 1.069 | 11.609 | 12:0 ISO FAME | 0.14 | ECL deviates | Reference 0.002 |
| 4.797 | 527 | 0.031 | ---- | 11.927 | ---- | ---- | ---- | ---- |
| 4.866 | 30691 | 0.033 | 1.052 | 11.999 | 12:0 FAME | 11.98 | ECL deviates | Reference 0.001 |
| 5.589 | 1652 | 0.033 | 1.029 | 12.614 | 13:0 ISO FAME | 0.63 | ECL deviates | Reference 0.000 |
| 6.043 | 4478 | 0.049 | 1.016 | 13.000 | 13:0 FAME | 1.69 | ECL deviates | Reference 0.000 |
| 6.678 | 3712 | 0.038 | 1.002 | 13.455 | Sum In Feature 2 | 1.38 | ECL deviates | 12:0 3OH FAME |
| 6.731 | 1234 | 0.034 | 1.001 | 13.493 | UN 13.493 | 0.46 | ECL deviates | ---- |
| 7.111 | 505 | 0.033 | ---- | 13.766 | ---- | ---- | ---- | ---- |
| 7.377 | 2721 | 0.049 | ---- | 13.957 | ---- | ---- | ---- | ---- |
| 7.435 | 3691 | 0.038 | 0.987 | 13.999 | 14:0 FAME | 1.35 | ECL deviates | Reference -0.002 |
| 7.607 | 979 | 0.033 | 0.984 | 14.109 | 13:0 ISO 3OH FAME | 0.36 | ECL deviates | ---- |
| 7.715 | 641 | 0.037 | ---- | 14.179 | ---- | ---- | ---- | ---- |
| 8.174 | 13808 | 0.043 | 0.976 | 14.472 | 14:0 DMA | 5.00 | ECL deviates | Reference 0.000 |
| 8.410 | 774 | 0.036 | 0.973 | 14.623 | 15:0 ISO FAME | 0.28 | ECL deviates | Reference 0.000 |
| 8.552 | 682 | 0.034 | 0.971 | 14.715 | 15:0 ANTEISO FAME | 0.25 | ECL deviates | Reference 0.000 |
| 8.623 | 5508 | 0.050 | 0.970 | 14.760 | Sum In Feature 4 | 1.98 | ECL deviates | UN 14.762 15:2 ? FA |
| 8.917 | 1491 | 0.044 | 0.966 | 14.948 | 16:0 ALDE | 0.53 | ECL deviates | Reference -0.004 |
| 8.999 | 6748 | 0.042 | 0.965 | 15.001 | 15:0 FAME | 2.42 | ECL deviates | Reference 0.000 |
| 9.293 | 804 | 0.043 | ---- | 15.176 | ---- | ---- | ---- | ---- |
| 9.455 | 796 | 0.054 | ---- | 15.272 | ---- | ---- | ---- | ---- |
| 9.820 | 19989 | 0.044 | 0.957 | 15.489 | Sum In Feature 5 | 7.09 | ECL deviates | 14:0 3OH FAME |
| 10.300 | 18255 | 0.043 | 0.953 | 15.775 | 16:1 CIS 7 FAME | 6.45 | ECL deviates | ---- |
| 10.378 | 2246 | 0.054 | 0.952 | 15.821 | 16:1 CIS 9 FAME | 0.79 | ECL deviates | ---- |
| 10.678 | 12367 | 0.043 | 0.949 | 16.000 | 16:0 FAME | 4.35 | ECL deviates | Reference -0.002 |
| 10.914 | 715 | 0.037 | 0.947 | 16.135 | 15:0 ISO 3OH FAME | 0.25 | ECL deviates | ---- |
| 11.099 | 19618 | 0.044 | 0.946 | 16.241 | Sum In Feature 6 | 6.88 | ECL deviates | 16:1 CIS 7 DMA |
| 11.369 | 1825 | 0.043 | ---- | 16.396 | ---- | ---- | ---- | ---- |
| 11.504 | 3401 | 0.040 | 0.943 | 16.474 | 16:0 DMA | 1.19 | ECL deviates | Reference 0.001 |
| 11.558 | 5774 | 0.045 | 0.943 | 16.505 | 15:0 3OH FAME | 2.02 | ECL deviates | |
| 11.777 | 1038 | 0.045 | 0.941 | 16.630 | 17:0 ISO FAME | 0.36 | ECL deviates | Reference -0.002 |
| 12.011 | 6902 | 0.042 | 0.940 | 16.765 | Sum In Feature 7 | 2.41 | ECL deviates | 17:1 CIS 8 FAME |
| 12.063 | 9034 | 0.043 | 0.940 | 16.794 | Sum In Feature 8 | 3.15 | ECL deviates | 17:1 CIS 9 FAME |
| 12.180 | 4788 | 0.051 | 0.939 | 16.862 | 17:1 CIS 11 FAME | 1.67 | ECL deviates | |
| 12.422 | 5798 | 0.045 | 0.938 | 17.001 | 17:0 FAME | 2.02 | ECL deviates | Reference -0.002 |
| 12.815 | 7183 | 0.045 | 0.935 | 17.223 | UN 17.223 | 2.49 | ECL deviates | ---- |
| 12.870 | 5533 | 0.042 | 0.935 | 17.254 | 18:1 AT 17.254 DMA | 1.92 | ECL deviates | ---- |
| 12.992 | 3520 | 0.049 | ---- | 17.323 | ---- | ---- | ---- | ---- |
| 13.252 | 2209 | 0.042 | 0.933 | 17.470 | 17:0 DMA | 0.76 | ECL deviates | Reference -0.002 |
| 13.699 | 1752 | 0.039 | 0.931 | 17.722 | 18:2 CIS 9,12 FAME | 0.60 | ECL deviates | |
| 13.787 | 32682 | 0.048 | 0.931 | 17.772 | 18:1 CIS 9 FAME | 11.28 | ECL deviates | |
| 13.882 | 2502 | 0.045 | 0.930 | 17.826 | Sum In Feature 10 | 0.86 | ECL deviates | 18:1c11/t9/t6 FAME |
| 13.969 | 3364 | 0.054 | ---- | 17.875 | ---- | ---- | ---- | ---- |
| 14.188 | 7272 | 0.047 | 0.929 | 17.999 | 18:0 FAME | 2.51 | ECL deviates | Reference -0.004 |
| 14.586 | 16845 | 0.047 | 0.927 | 18.225 | 18:1 CIS 9 DMA | 5.79 | ECL deviates | ---- |
| 14.703 | 1702 | 0.062 | 0.927 | 18.292 | 18:1 CIS 11 DMA | 0.59 | ECL deviates | ---- |
| 14.871 | 897 | 0.039 | ---- | 18.388 | ---- | ---- | ---- | ---- |
| 15.520 | 833 | 0.043 | 0.924 | 18.757 | 19:1 CIS 7 FAME | 0.29 | ECL deviates | ---- |
| 15.717 | 9656 | 0.051 | 0.923 | 18.870 | 19 CYC 9,10/:1 FAME | 3.31 | ECL deviates | Reference -0.003 |
| 16.504 | 2322 | 0.045 | 0.920 | 19.322 | 19:0 CYC 9,10 DMA | 0.79 | ECL deviates | Reference -0.004 |
| ---- | 3712 | --- | ---- | ---- | Summed Feature 2 | 1.38 | 12:0 3OH FAME | 13:0 DMA |
| ---- | 5508 | ---- | ---- | ---- | Summed Feature 4 | 1.98 | UN 14.762 15:2 ? FA | 15:2 FAME |
| ---- | ---- | ---- | ---- | ---- | ---- | ---- | 15:1 CIS 7 | ---- |
| ---- | 19989 | ---- | ---- | ---- | Summed Feature 5 | 7.09 | 15:0 DMA | 14:0 3OH FAME |
| ---- | 19618 | ---- | ---- | ---- | Summed Feature 6 | 6.88 | 15:0 ANTEI 3OH FAME | 16:1 CIS 7 DMA |
| ---- | 6902 | ---- | ---- | ---- | Summed Feature 7 | 2.41 | 17:2 FAME @ 16.760 | 17:1 CIS 8 FAME |
| ---- | 9034 | ---- | ---- | ---- | Summed Feature 8 | 3.15 | 17:1 CIS 9 FAME | 17:2 FAME @ 16.801 |
| ---- | 2502 | --- | ---- | ---- | Summed Feature 10 | 0.86 | 18:1c11/t9/t6 FAME | UN 17.834 |

**Table 13B. Fatty acid components of the strain MNH22004:**

| RT | Response | Ar/Ht | RFact | ECL | Peak Name | Percent | Comment1 | Comment2 |
|---|---|---|---|---|---|---|---|---|
| 1.626 | 9.42E+08 | 0.045 | ---- | 6.975 | SOLVENT PEAK | ---- | < min rt | ---- |
| 2.587 | 462 | 0.031 | ---- | 8.815 | ---- | ---- | < min rt | ---- |
| 2.659 | 245 | 0.023 | ---- | 8.954 | ---- | ---- | < min rt | ---- |
| 3.206 | 450 | 0.028 | 1.154 | 10 | 10:0 FAME | 0.2 | ECL deviates 0.000 | Reference 0.008 |
| 3.866 | 5764 | 0.028 | ---- | 10.921 | ---- | ---- | ---- | ---- |
| 3.924 | 382 | 0.029 | 1.099 | 11 | 11:0 FAME | 0.16 | ECL deviates 0.000 | Reference 0.006 |
| 4.499 | 906 | 0.038 | 1.071 | 11.609 | 12:0 ISO FAME | 0.37 | ECL deviates 0.001 | Reference 0.006 |
| 4.868 | 35459 | 0.033 | 1.054 | 11.999 | 12:0 FAME | 14.43 | ECL deviates -0.001 | Reference 0.004 |
| 5.591 | 1077 | 0.035 | 1.031 | 12.613 | 13:0 ISO FAME | 0.43 | ECL deviates -0.001 | Reference 0.003 |
| 5.696 | 629 | 0.034 | 1.028 | 12.703 | 13:0 ANTEISO FAME | 0.25 | ECL deviates 0.000 | Reference 0.003 |
| 6.68 | 2443 | 0.035 | 1.004 | 13.454 | Sum In Feature 2 | 0.95 | ECL deviates -0.002 | 12:03OH FAME |
| 7.38 | 4724 | 0.05 | ---- | 13.957 | ---- | ---- | ---- | ---- |
| 7.438 | 2547 | 0.036 | 0.989 | 13.999 | 14:0 FAME | 0.97 | ECL deviates -0.001 | Reference 0.001 |
| 7.611 | 481 | 0.034 | 0.986 | 14.109 | 13:0 ISO 3OH FAME | 0.18 | ECL deviates -0.005 | ---- |
| 8.179 | 1425 | 0.032 | 0.978 | 14.473 | 14:0 DMA | 0.54 | ECL deviates 0.001 | Reference 0.003 |
| 8.227 | 2614 | 0.039 | ---- | 14.504 | ---- | ---- | ---- | ---- |
| 8.413 | 477 | 0.034 | 0.975 | 14.623 | 15:0 ISO FAME | 0.18 | ECL deviates 0.000 | Reference 0.001 |
| 8.626 | 6176 | 0.043 | 0.972 | 14.759 | Sum In Feature 4 | 2.32 | ECL deviates -0.003 | UN 14.762 15:2 ? FA |
| 8.922 | 3131 | 0.046 | 0.968 | 14.949 | 16:0 ALDE | 1.17 | ECL deviates -0.002 | Reference -0.001 |
| 9.002 | 1305 | 0.039 | 0.967 | 15 | 15:0 FAME | 0.49 | ECL deviates 0.000 | Reference 0.001 |
| 9.299 | 1747 | 0.047 | ---- | 15.177 | ---- | ---- | ---- | ---- |
| 9.823 | 32013 | 0.043 | 0.958 | 15.489 | Sum In Feature 5 | 11.84 | ECL deviates 0.001 | 14:03OH FAME |
| 10.304 | 20523 | 0.042 | 0.953 | 15.775 | 16:1 CIS 7 FAME | 7.55 | ECL deviates 0.001 | ---- |
| 10.681 | 37823 | 0.042 | 0.95 | 15.999 | 16:0 FAME | 13.87 | ECL deviates -0.001 | Reference -0.001 |
| 10.916 | 655 | 0.035 | 0.948 | 16.134 | 15:0 ISO 3OH FAME | 0.24 | ECL deviates -0.001 | ---- |
| 11.101 | 26265 | 0.043 | 0.947 | 16.24 | Sum In Feature 6 | 9.6 | ECL deviates 0.000 | 16:1 CIS 7 DMA |
| 11.369 | 1164 | 0.039 | ---- | 16.394 | ---- | ---- | ---- | ---- |
| 11.506 | 16019 | 0.044 | 0.944 | 16.473 | 16:0 DMA | 5.84 | ECL deviates 0.002 | Reference 0.001 |
| 11.78 | 2915 | 0.042 | 0.942 | 16.63 | 17:0 ISO FAME | 1.06 | ECL deviates 0.000 | Reference 0.000 |
| 12.011 | 4053 | 0.054 | 0.94 | 16.762 | Sum In Feature 7 | 1.47 | ECL deviates 0.002 | 17:2 FAME @ 16.760 |
| 12.178 | 2460 | 0.049 | 0.939 | 16.859 | 17:1 CIS 11 FAME | 0.89 | ECL deviates -0.005 | ---- |
| 12.426 | 2893 | 0.047 | 0.937 | 17.001 | 17:0 FAME | 1.05 | ECL deviates 0.001 | Reference 0.000 |
| 12.611 | 866 | 0.035 | 0.936 | 17.106 | UN 17.103 17:0i DMA | 0.31 | ECL deviates 0.002 | Reference 0.001 |
| 12.814 | 1112 | 0.052 | 0.935 | 17.22 | UN 17.223 | 0.4 | ECL deviates -0.003 | ---- |
| 12.995 | 2726 | 0.043 | ---- | 17.323 | ---- | ---- | ---- | ---- |
| 13.146 | 309 | 0.034 | ---- | 17.408 | ---- | ---- | ---- | ---- |
| 13.254 | 723 | 0.036 | 0.933 | 17.469 | 17:0 DMA | 0.26 | ECL deviates 0.000 | Reference -0.001 |
| 13.705 | 1834 | 0.042 | 0.93 | 17.724 | 18:2 CIS 9,12 FAME | 0.66 | ECL deviates 0.001 | ---- |
| 13.789 | 28244 | 0.047 | 0.93 | 17.772 | 18:1 CIS 9 FAME | 10.14 | ECL deviates 0.001 | ---- |
| 13.889 | 1815 | 0.064 | 0.93 | 17.829 | Sum In Feature 10 | 0.65 | ECL deviates 0.005 | 18:1c11/t9/t6 FAME |
| 14.192 | 8808 | 0.048 | 0.928 | 18 | 18:0 FAME | 3.16 | ECL deviates 0.000 | Reference -0.002 |
| 14.589 | 15537 | 0.048 | 0.926 | 18.225 | 18:1 CIS 9 DMA | 5.56 | ECL deviates 0.001 | ---- |
| 15.011 | 1177 | 0.045 | 0.925 | 18.466 | 18:0 DMA | 0.42 | ECL deviates 0.000 | ---- |
| 15.721 | 5253 | 0.045 | 0.922 | 18.869 | 19 CYC 9,10/:1 FAME | 1.87 | ECL deviates -0.001 | Reference -0.003 |
| 16.507 | 1468 | 0.039 | 0.919 | 19.321 | 19:0 CYC 9,10 DMA | 0.52 | ECL deviates -0.001 | Reference -0.003 |
| ---- | 2443 | ---- | ---- | ---- | Summed Feature 2 | 0.95 | 12:0 3OH FAME | 13:0 DMA |
| ---- | 6176 | ---- | ---- | ---- | Summed Feature 4 | 2.32 | UN 14.762 15:2 ? FA | 15:2 FAME |
| ---- | ---- | ---- | ---- | ---- | ---- | ---- | 15:1 CIS 7 | ---- |
| ---- | 32013 | ---- | ---- | ---- | Summed Feature 5 | 11.84 | 15:0 DMA | 14:0 3OH FAME |
| ---- | 26265 | ---- | ---- | ---- | Summed Feature 6 | 9.6 | 15:0 ANTEI 3OH FAME | 16:1 CIS 7 DMA |
| ---- | 4053 | ---- | ---- | ---- | Summed Feature 7 | 1.47 | 17:2 FAME @ 16.760 | 17:1 CIS 8 FAME |
| ---- | 1815 | ---- | ---- | ---- | Summed Feature 10 | 0.65 | 18:1c11/t9/t6 FAME | UN 17.834 |

**Table 13C. Fatty acid components of the strain MNH27256:**

| RT | Response | Ar/Ht | RFact | ECL | Peak Name | Percent | Comment1 | Comment2 |
|---|---|---|---|---|---|---|---|---|
| 1.626 | 9.36E+08 | 0.044 | ---- | 6.976 | SOLVENT PEAK | ---- | < min rt | ---- |
| 2.285 | 286 | 0.035 | ---- | 8.235 | ---- | ---- | < min rt | ---- |
| 2.589 | 353 | 0.03 | ---- | 8.818 | ---- | ---- | < min rt | ---- |
| 2.656 | 290 | 0.025 | ---- | 8.947 | ---- | ---- | < min rt | ---- |
| 3.208 | 484 | 0.038 | 1.154 | 10.002 | 10:0 FAME | 0.31 | ECL deviates 0.002 | Reference 0.009 |
| 3.866 | 5035 | 0.029 | ---- | 10.921 | ---- | ---- | ---- | ---- |
| 3.921 | 288 | 0.022 | 1.099 | 10.997 | 11:0 FAME | 0.18 | ECL deviates -0.003 | Reference 0.003 |
| 4.499 | 634 | 0.032 | 1.071 | 11.61 | 12:0 ISO FAME | 0.38 | ECL deviates 0.002 | Reference 0.006 |
| 4.868 | 26470 | 0.032 | 1.054 | 12 | 12:0 FAME | 15.54 | ECL deviates 0.000 | Reference 0.004 |
| 5.59 | 681 | 0.032 | 1.031 | 12.614 | 13:0 ISO FAME | 0.39 | ECL deviates 0.000 | Reference 0.003 |
| 5.694 | 510 | 0.034 | 1.028 | 12.702 | 13:0 ANTEISO FAME | 0.29 | ECL deviates -0.001 | Reference 0.001 |
| 6.68 | 2554 | 0.037 | 1.004 | 13.456 | Sum In Feature 2 | 1.43 | ECL deviates 0.000 | 12:0 3OH FAME |
| 7.38 | 3814 | 0.052 | ---- | 13.958 | ---- | ---- | ---- | ---- |
| 7.439 | 881 | 0.032 | 0.989 | 14 | 14:0 FAME | 0.49 | ECL deviates 0.000 | Reference 0.001 |
| 7.611 | 460 | 0.041 | 0.986 | 14.111 | 13:0 ISO 3OH FAME | 0.25 | ECL deviates -0.003 | ---- |
| 8.226 | 3873 | 0.051 | ---- | 14.505 | ---- | ---- | ---- | ---- |
| 8.624 | 4170 | 0.04 | 0.972 | 14.76 | Sum In Feature 4 | 2.26 | ECL deviates -0.002 | UN 14.762 15:2 ? FA |
| 8.918 | 2480 | 0.044 | 0.968 | 14.948 | 16:0 ALDE | 1.34 | ECL deviates -0.003 | Reference -0.003 |
| 9.002 | 521 | 0.035 | 0.967 | 15.002 | 15:0 FAME | 0.28 | ECL deviates 0.002 | Reference 0.001 |
| 9.299 | 1274 | 0.037 | ---- | 15.178 | | ---- | | |
| 9.824 | 24781 | 0.043 | 0.958 | 15.49 | Sum In Feature 5 | 13.22 | ECL deviates 0.002 | 14:0 3OH FAME |
| 10.305 | 10863 | 0.044 | 0.953 | 15.776 | 16:1 CIS 7 FAME | 5.77 | ECL deviates 0.002 | ---- |
| 10.68 | 17718 | 0.044 | 0.95 | 16 | 16:0 FAME | 9.37 | ECL deviates 0.000 | Reference -0.001 |
| 10.918 | 576 | 0.04 | 0.948 | 16.136 | 15:0 ISO 3OH FAME | 0.3 | ECL deviates 0.001 | ---- |
| 11.102 | 14794 | 0.043 | 0.947 | 16.241 | Sum In Feature 6 | 7.8 | ECL deviates 0.001 | 16:1 CIS 7 DMA |
| 11.37 | 745 | 0.039 | ---- | 16.395 | ---- | ---- | ---- | ---- |
| 11.505 | 8635 | 0.047 | 0.944 | 16.473 | 16:0 DMA | 4.54 | ECL deviates 0.002 | Reference 0.001 |
| 11.782 | 1583 | 0.045 | 0.942 | 16.631 | 17:0 ISO FAME | 0.83 | ECL deviates 0.001 | Reference 0.000 |
| 12.012 | 3309 | 0.046 | 0.94 | 16.764 | Sum In Feature 7 | 1.73 | ECL deviates -0.001 | 17:1 CIS 8 FAME |
| 12181 | 1606 | 0.054 | 0.939 | 16.86 | 17:1 CIS 11 FAME | 0.84 | ECL deviates -0.004 | ---- |
| 12.423 | 2003 | 0.043 | 0.937 | 17 | 17:0 FAME | 1.05 | ECL deviates 0.000 | Reference -0.002 |
| 12.608 | 702 | 0.047 | 0.936 | 17.104 | UN 17.103 17:0i DMA | 0.37 | ECL deviates 0.000 | Reference -0.001 |
| 12.817 | 514 | 0.039 | 0.935 | 17.222 | UN 17.223 | 0.27 | ECL deviates -0.001 | |
| 12.997 | 1784 | 0.045 | ---- | 17.324 | ---- | ---- | ---- | ---- |
| 13.15 | 474 | 0.043 | ---- | 17.41 | ---- | ---- | ---- | ---- |
| 13.256 | 650 | 0.04 | 0.933 | 17.47 | 17:0 DMA | 0.34 | ECL deviates 0.001 | Reference 0.000 |
| 13.79 | 29023 | 0.047 | 0.93 | 17.772 | 18:1 CIS 9 FAME | 15.03 | ECL deviates 0.001 | ---- |
| 13.885 | 1148 | 0.052 | 0.93 | 17.826 | Sum In Feature 10 | 0.59 | ECL deviates 0.002 | 18:1c11/t9/t6 FAME |
| 14.194 | 6143 | 0.046 | 0.928 | 18 | 18:0 FAME | 3.17 | ECL deviates 0.000 | Reference -0.001 |
| 14.588 | 15167 | 0.047 | 0.926 | 18.224 | 18:1 CIS 9 DMA | 7.82 | ECL deviates 0.000 | ---- |
| 14.877 | 480 | 0.037 | ---- | 18.388 | ---- | ---- | ---- | ---- |
| 15.016 | 1256 | 0.048 | 0.925 | 18.467 | 18:0 DMA | 0.65 | ECL deviates 0.001 | ---- |
| 15.722 | 4922 | 0.05 | 0.922 | 18.869 | 19 CYC 9,10/:1 FAME | 2.53 | ECL deviates -0.001 | Reference -0.002 |
| 16.51 | 1310 | 0.039 | 0.919 | 19.321 | 19:0 CYC 9,10 DMA | 0.67 | ECL deviates -0.001 | Reference -0.002 |
| ---- | 2554 | ---- | ---- | ---- | Summed Feature 2 | 1.43 | 12:0 3OH FAME | 13:0 DMA |
| ---- | 4170 | ---- | ---- | ---- | Summed Feature 4 | 2.26 | UN 14.762 15:2 ? FA | 15:2 FAME |
| ---- | ---- | --- | ---- | ---- | ---- | ---- | 15:1 CIS 7 | ---- |
| ---- | 24781 | ---- | ---- | ---- | Summed Feature 5 | 13.22 | 15:0 DMA | 14:0 3OH FAME |
| ---- | 14794 | ---- | ---- | ---- | Summed Feature 6 | 7.8 | 15:0 ANTEI 3OH FAME | 16:1 CIS 7 DMA |
| ---- | 3309 | ---- | ---- | ---- | Summed Feature 7 | 1.73 | 17:2 FAME @ 16.760 | 17:1 CIS 8 FAME |
| ---- | 1148 | ---- | ---- | ---- | Summed Feature 10 | 0.59 | 18:1c11/t9/t6 FAME | UN 17.834 |

According to the morphological, microscopic, physiological and biochemical characteristics, 16S rRNA gene sequence and genomic information, the strain MNH05026 belonged to a new species of the genus *Megasphaera,* and was temporarily named *Megasphaera* sp. MNH05026 (the patent strain deposit number was GDMCC No: 62001). The strains MNH22004 and MNH27256 were two different strains of the same new species of the genus *Megasphaera,* and were temporarily named *Megasphaera* sp. MNH22004 (the strain deposit number was GDMCC NO: 62000) and *Megasphaera* sp. MNH27256 (the patent strain deposit number was GDMCC NO: 61999).

### Example 3. Short-chain fatty acid (SCFA) assays of the strains MNH05026, MNH22004, and MNH27256

Preparation of bacterial cells: The strains MNH05026, MNH22004, and MNH27256 were respectively inoculated into TSB liquid medium, anaerobically cultured at 37 °C for 48 hours, centrifuged to collect the bacterial cells, which were stored at -86 °C for later use.

Formulation of standards: Acetic acid, propionic acid, butyric acid, isobutyric acid, valeric acid, isovaleric acid, and hexanoic acid standards were weighed, and formulated with ethyl acetate to form eight mixed standard concentration gradients: 0.1 µg/mL, 0.5 µg/mL, 1 µg/mL, 5 µg/mL, 10 µg/mL, 20 µg/mL, 50 µg/mL, and 100 µg/mL. To 600 µL of the standard was added 25 µL of 4-methylvaleric acid to a final concentration of 500 µM as an internal standard, mixed well, and charged into an injection bottle for GC-MS detection. The injection volume was 1 µL, and the split ratio was 10:1. Split injection was used.

Extraction of metabolites: The sample was thawed on ice. 80 mg of the sample was pipetted into a 2 mL glass centrifuge tube, resuspended in 900 µL of 0.5% phosphoric acid, mixed well by shaking for 2 min, and centrifuged at 14,000 g for 10 min. 800 µL of the supernatant was pipetted, to which an equal amount of ethyl acetate was added for extraction, mixed well by shaking for 2 min, and centrifuged at 14,000 g for 10 min. 600 µL of the upper organic phase was pipetted, to which 4-methylvaleric acid was added to a final concentration of 500 µM as an internal standard, mixed well, and charged into an injection bottle for GC-MS detection. The injection volume was 1 µL, and the split ratio was 10:1. Split injection was used.

Sample detection and analysis: The sample was separated on an Agilent DB-WAX capillary column (30 m × 0.25 mm ID × 0.25 µm) gas chromatography system. Temperature programming: the initial temperature was 90 °C, raised to 120 °C at 10 °C/min, then to 150 °C at 5 °C/min, finally to 250 °C at 25 °C/min, and maintained for 2min. The carrier gas was helium, with a flow rate of 1.0 mL/min.

Mass spectrometry analysis was performed on an Agilent 7890A/5975C gas chromatograph-mass spectrometer. The inlet temperature was 250 °C, the ion source temperature was 230 °C, the transmission line temperature was 250 °C, and the quadrupole temperature was 150 °C. An electron impact ionization (EI) source was used in full-scan and SIM scanning modes with an electron energy of 70 eV.

The chromatographic peak area and retention time were extracted using the MSD ChemStation software. A standard curve was plotted to calculate the content of short-chain fatty acids in the sample (see Table 14).

**Table 14. Results of short-chain fatty acid (SCFA) yields of the strains MNH05026, MNH22004, and MNH27256:**

| Type of SCFA | acetic acid | Propionic acid | Isobutyric acid | Butyric acid | Isovaleric acid | Valeric acid | Hexanoic acid |
|---|---|---|---|---|---|---|---|
| MNH05026 Yield (ug/g) | 251.21 | 42.85 | 120.81 | 214.75 | 248.96 | 59.36 | 366.76 |
| MNH22004 Yield (µg/g) | 463.32 | 104 | 183.48 | 920.31 | 320.43 | 3.52 | 0.58 |
| MNH27256 Yield (µg/g) | 407.39 | 119.21 | 198.17 | 1165.04 | 329.46 | 4.53 | 0.28 |

**Conclusion:** The strain MNH05026 can synthesize a large amount of hexanoic acid, acetic acid, isovaleric acid, butyric acid, and isobutyric acid, and a small amount of valeric acid and propionic acid during its growth. The strain MNH22004 can synthesize a large amount of acetic acid, propionic acid, isovaleric acid, butyric acid, and isobutyric acid, especially a relatively larger amount of butyric acid; and a small amount or very little amount of hexanoic acid and valeric acid during its growth. The strain MNH27256 can synthesize a large amount of acetic acid, propionic acid, isovaleric acid, butyric acid, and isobutyric acid, especially a relatively larger amount of butyric acid; and a small amount or very little amount of hexanoic acid and valeric acid during its growth. The term "highly productive" as used herein means that the yield is not less than 200 ug/g. Therefore, it can be determined that MNH05026, MNH22004, and MNH27256 of the present application are highly productive of acetic acid and butyric acid.

### Example 4: Method for preparing bacterial cells of MNH05026, MNH22004, and MNH27256 for cell screening platform

A single colony of MNH05026 was picked up with a sterilized toothpick into 10 ml of AC liquid medium (comprising (per liter): peptone, 20 g; glucose, 5 g; yeast extract, 3 g; beef extract powder, 3 g; and vitamin C, 0.2 g; pH 7.0), and anaerobically cultured at 37 °C for 1 day. 1 ml of the culture was sampled for mass spectrometry. After the identification result was correct, 2 ml of the bacterial suspension was pipetted into 80 ml of AC liquid medium and cultured at 37 °C in an anaerobic operation station. After culturing for 24 hours, 30 ml of the bacterial suspension was pipetted into 400 ml of AC liquid medium and cultured at 37 °C in an anaerobic operation station. After culturing for 24 hours, 1 ml of the bacterial solution was sampled for mass spectrometry. After the identification result was correct, the bacterial solution was wholly transferred to a 1 L centrifuge bottle and centrifuged at 6000 rpm and 4 °C for 30 min. The precipitate was resuspended in AC liquid medium: sterile glycerol (4:1).

0.1 ml of a stock solution of the MNH05026 bacterial solution with glycerol was pipetted into 0.9 ml of physiological saline for serial dilution to 10⁻8 gradient. 100 µl of the diluted solutions at concentrations of 10⁻⁶, 10⁻⁷, and 10⁻⁸ were pipetted onto anaerobic blood plates respectively. Glass beads were added and shaken well. CFU was determined using the plate counting method.

The prepared stock solution of the strain was dispensed into sterile cryovials, with 0.2 ml of the bacterial solution per vial and 10 vials for each strain. They were cryopreserved at -80 °C. After being completely frozen (24 h), a vial of frozen bacteria was removed and thawed to room temperature for later use. Viable bacterial concentration (CFU) was measured using the diluting and spreading method.

The bacterial cells of MNH22004 and MNH27256 were prepared by the same method.

### Example 5. Regulation of the immune activity of macrophages by the strains MNH05026, MNH22004, and MNH27256

The experimental method was based on Experimental Example 1 in the prior art CN112618576B. The data in the resultant graphs are shown as mean ± standard deviation (Mean ± SD). Statistical analysis was performed using a t-test (Student's t test). The significance of difference was indicated by *: * p < 0.05, ** p < 0.01, not significant, not indicated.

After the strains MNH05026, MNH22004, and MNH27256 were respectively incubated with macrophages (Wuhan Pricella Biotechnology Co., Ltd.) for 24 h, the supernatants were collected and the concentration of the cytokine IL-1β secreted was detected by ELISA. The results are shown in Table 2 above. The results in Table 2 showed that the macrophages, upon co-culture with the *Macrosphaera* bacteria, exhibited characteristics of differentiation towards M1-type macrophages. M1 macrophages, known as classic macrophages, are macrophages that can produce pro-inflammatory cytokines. M1 macrophages are characterized by secretion of pro-inflammatory factors such as TNFα, IL-1β, IL-6, IL-12, and IL-8, and play an important role in the early stage of inflammation. This experiment verified that the secretion of IL-1β was significantly increased.

In order to further explore the regulatory effects of the *Megasphaera* bacteria on more immunoreactive effector factors in macrophages, the above supernatants were further collected, and the concentrations of other immunoreactive effector factors in the supernatants was further detected by the CBA method. The results are as follows:
(1) MNH05026: The concentrations of the cytokines IL-1β, IL-8, IL-12/IL-13P40, IL-10, IP-10, RANTES, MIG, MCP-1, IL-6, and TNF secreted in the supernatant were measured (the results are shown in Fig. 10). The results showed that the macrophages, upon co-culture with MNH05026, clearly exhibited characteristics of differentiation towards M1-type macrophages. Specifically, the contents of the pro-inflammatory factors IL-1β, IL-6, IL-8, and TNF were significantly increased, and the content of IL-12/IL-13P40 showed an upregulation trend. The contents of the chemokines IP-10, RANTES, MIG, and MCP-1 were increased very significantly, and the content of the anti-inflammatory factor IL-10 was significantly increased.

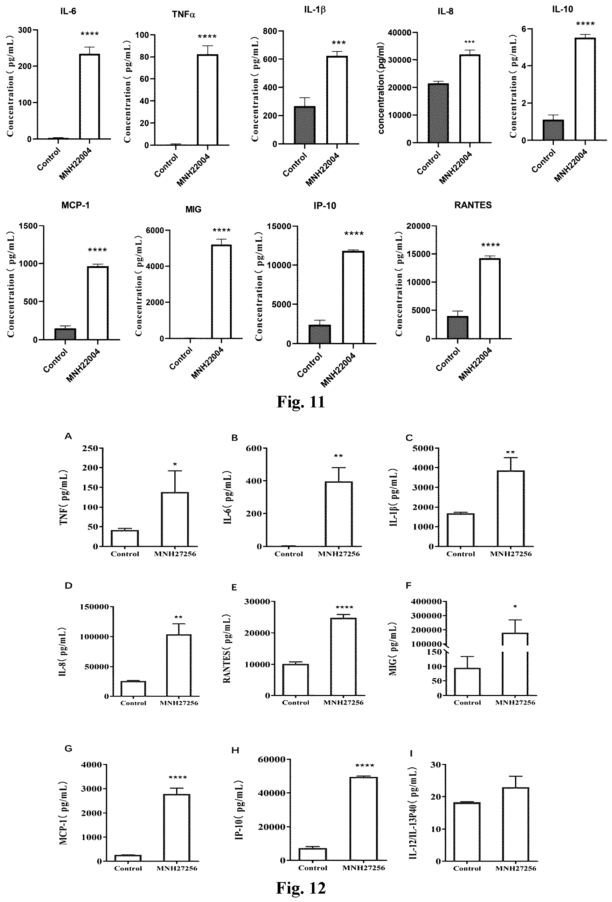

Fig. 10 shows the expression of immunoreactive effector factors in the macrophages regulated by the strain MNH05026.
(2) MNH22004: The concentrations of the cytokines IL-1β, IP-10, RANTES, MIG, MCP-1, IL-10, IL-6, and TNFα secreted in the supernatant were measured. The results are shown in Fig. 11. Upon co-culture with MNH22004, the macrophages significantly enhanced the expression of the pro-inflammatory factors IL-1β, IL-6, and TNFα, and the chemokines IL-8, IP-10, RANTES, MIG, and MCP-1, and significantly enhanced the expression of the anti-inflammatory factor IL-10.
(3) MNH27256: The concentrations of the cytokines TNFα, IL-6, IL-1β, IL-8, IP-10, MIG, MCP-1, RANTES, and IL-12/IL-13P40 secreted in the supernatant were measured. The results (see Fig. 12) showed that the macrophages, upon co-culture with MNH05026, clearly exhibited characteristics of differentiation towards M1-type macrophages. The secretion of TNFα, IL-6, IL-1β, IL-8, IP-10, MIG, MCP-1, and RANTES was significantly increased, and the secretion of IL-12/IL-13P40 showed an upregulation trend.

Fig. 12 shows the expression of immunoreactive effector factors in the macrophages regulated by the strain MNH27256.

### Example 6. Regulation of the immune activity of primary PBMCs by MNH05026, MNH22004, and MNH27256

The experimental method was based on Experimental Example 2 in the prior art CN112618576B.

After the strains MNH05026, MNH22004, and MNH27256 were respectively incubated with primary PBMCs (purchased from TPCS, batch No. A19Z289100) for 24 h, the supernatants were collected and the concentration of the cytokine IL-1β secreted was detected by ELISA. The results are shown in Table 2 above. The secretion of IL-1β in the strain treatment group was significantly increased, and the secretion in the IFNγ group was slightly higher than that in the control group but significantly lower than that in the strain treatment group. This experiment demonstrates that the *Megasphaera* bacteria can effectively regulate the expression of immunoreactive effector factors in Primary PBMCs.

In order to further explore the regulatory effects of the *Megasphaera* bacteria on more immunoreactive effector factors in Primary PBMCs, the above supernatants were further collected, and the concentrations of other immunoreactive effector factors in the supernatants was further detected by the CBA method. The results are as follows:

### MNH05026:

The concentrations of the cytokines IFNγ, TNFα, IL-1β, IP-10 (CXCL-10), RANTES (CCL5), MIG (CXCL-9), IL-6, IL-8, IL-12/IL-13P40, IL-10, and MCP-1 secreted in the supernatant were measured (the results are shown in Fig. 13). As shown in Fig. 13, MNH05026 induced an upregulation trend in the content of the inflammatory factor IFNγ, and the contents of other inflammatory factors such as TNFα, IL-6, IL-8, and IL-1β were significantly increased. The contents of the chemokines MIG (CCL-9), RANTES (CCL5), and IP-10 (CXCL10) were significantly increased, and the content of the anti-inflammatory factor IL-10 was significantly increased.

Fig. 13 shows the expression of immunoreactive effector factors in the Primary PBMCs regulated by the strain MNH05026.

### MNH22004:

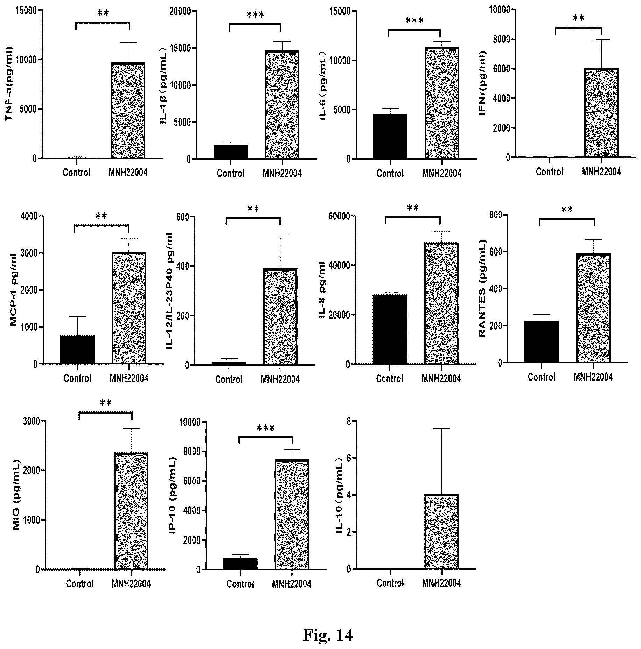

The concentrations of cytokines such as IFNγ, TNFα, IL-1β, IP-10 (CXCL-10), RANTES (CCL5), MIG (CXCL-9), IL-10, IL-6, IL-8, IL-12/IL-23P40, MCP-1 secreted in the supernatant were measured. The results are shown in Fig. 14. The results in Fig. 14 showed that MNH22004 significantly enhanced the expression of the inflammatory factors IFNγ, TNFα, IL-1β, IL-6, IL-12/IL-23P40, and the chemokines IP-10 (CXCL-10), RANTES (CCL5), MIG (CXCL-9), MCP-1, and IL-8. The content of the anti-inflammatory factor IL-10 showed an upregulation trend.

Fig. 14 shows the expression of immunoreactive effector factors in the Primary PBMCs regulated by the strain MNH22004.

### MNH27256:

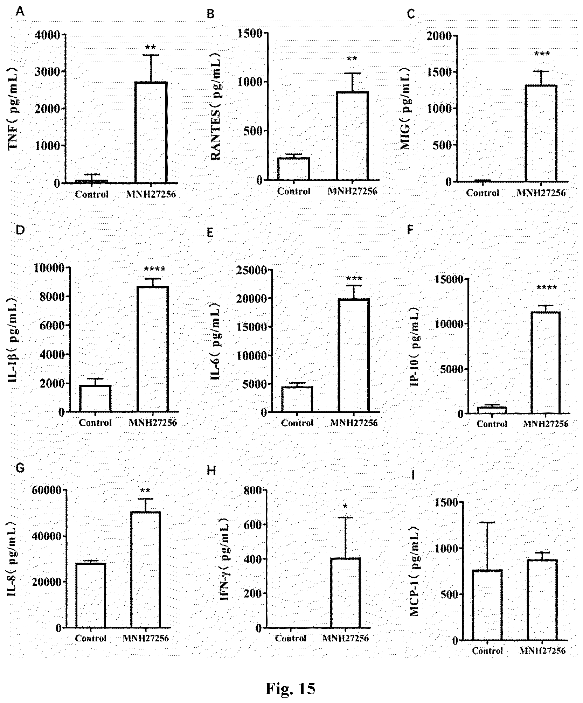

The concentrations of the cytokines TNF, IL-6, IL-1β, IL-8, IP-10, MIG, MCP-1, RANTES, and IFN-γ secreted in the supernatant were measured. The results are shown in Fig. 15. MNH27256 induced a significant increase in the inflammatory factors TNF, IL-6, IL-1β, IL-8, and IFN-γ, a significant increase in the chemokines IP-10, MIG, and RANTES, and an upregulation trend of the chemokine MCP-1.

Fig. 15 shows the expression of immunoreactive effector factors in the Primary PBMCs regulated by the strain MNH27256.

### Example 7. Animal experiments on treatment of cancers (liver cancer and lung cancer) with Megasphaera strains

To verify whether the strains MNH05026, MNH22004, and MNH27256 of the genus *Megasphaera* can be used to prevent and treat tumors, a liver cancer growth inhibition experiment was conducted using a mouse syngeneic tumor model. This experiment has been ethically reviewed by the Laboratory Animal Care and Use Committee of Moon Biotech. 2) Test strains: Glycerol cryovials of the strains MNH05026, MNH22004, and MNH27256 were thawed at 37 °C, and then inoculated onto anaerobic blood plates in an anaerobic workstation for activation. The activated strains were inoculated into MM01 liquid medium and anaerobically cultured to obtain a sufficient amount of cultures. The cultured bacterial solutions were centrifuged, concentrated, and then resuspended in solvent to afford test articles with a purity and viable bacterial count (2.04 × 10¹⁰ CFU/mL) meeting the requirements of animal experiments.

Tumor cells: H22 mouse liver cancer cells, CBP69230.

### (I) Experiments for verifying the preventive effects of the strains MNH05026 (A026), MNH22004 (A3), and MNH27256 (A9) on tumor (liver cancer)

Experimental animals: C57BL/6J mice, aged 5 weeks, 20 mice in total, purchased from Guangdong GemPharmatech Co., Ltd.

Animal experiments (taking MNH05026 as an example, the methods for MNH22004 and MNH27256 were the same as that for MNH05026): The animals were normally fed. Upon completion of the quarantine period, they were randomized into 2 groups (a control group and an experimental group) according to body weight, with 10 animals per group, and raised in separate cages. After grouping, a mouse liver cancer model was established by intradermal injection of H22 mouse liver cancer cells in a cell inoculation amount of 2×10⁶/mL, 0.1 mL/mouse. Gavage administration was initiated on the day of completion of the inoculation (DAY 0). The control group was given the culture medium, and the experimental group was given the MNH05026 bacteria. The volume of the gavage administration was 0.2 mL/mouse/dose, and the frequency of administration was once daily. General observation was conducted once a day during the quarantine period and after the daily dose during the administration. The animals were weighed upon arrival, at the end of the quarantine period, and three times a week during the administration. Starting from 7 days after tumor cell inoculation (DAY 7), the tumors were measured three times a week, and the tumor growth was recorded. The endpoint of this experiment was that 19 gavage administrations were completed. At the experimental endpoint, all mice were euthanized by cervical dislocation. The results are shown in Table 15 and Table 16.

**Table 15. Overall statistics of mice at the experimental endpoint:**

| Group | Number of mice with successful tumor inoculation | Number of mice at experimental endpoint | Number of euthanized mice | Average weight of dissected tumors (g) | Average body weight prior to dissection (g) | Average size of dissected tumors (mm³) |
|---|---|---|---|---|---|---|
| MNH05026 Control Group | 7 | 6 | 1 | 2.53 | 30.51 | 2372 |
| MNH05026 Experimental group | 10 | 10 | 0 | 1.78 | 27.66 | 1566 |
| MNH22004 Control group | 7 | 6 | 1 | 2.53 | 30.51 | 2372 |
| MNH22004 Experimental group | 6 | 5 | 1 | 1.44 | 26.68 | 1896 |
| MNH27256 Control group | 7 | **6** | **1** | **2.53** | **30.51** | **2372** |
| MNH27256 Experimental group | **9** | **9** | **0** | **1.56** | **25.71** | **1367** |

**Table 16. Tumor tissue weight after dissection of mice:**

| Group | No. | Tumor weight (g) | Mean (g) |
|---|---|---|---|
| Control | CT1-1 | 2.2896 | 2.52605 |
| | CT1-2 | 5.0875 | |
| | CT2-1 | 1.8192 | |
| | CT2-2 | 0.8667 | |
| | CT3-2 | 3.1895 | |
| | CT3-4 | 1.9038 | |
| MNH05026 | A026-25-1 | 2.1492 | 1.77817 |
| | A026-25-2 | 1.6121 | |
| | A026-25-3 | 0.9658 | |
| | A026-26-1 | 1.8486 | |
| | A026-26-2 | 1.8857 | |
| | A026-26-3 | 2.8823 | |
| | A026-27-1 | 1.9302 | |
| MNH22004 | A3-10-2 | 1.5128 | 1.44 |
| | A3-10-3 | 1.8183 | |
| | A3-11-1 | 1.5872 | |
| | A3-11-2 | 0.6208 | |
| | A3-12-4 | 1.6474 | |
| | A9-28-1 | 1.8019 | |
| | A9-28-2 | 1.0675 | |
| | A9-28-3 | 0.8394 | |
| | A9-29-1 | 1.1684 | |
| MNH27256 | A9-29-2 | 1.9207 | 1.56 |
| | A9-29-3 | 1.8605 | |
| | A9-30-2 | 3.1922 | |
| | A9-30-3 | 0.8383 | |
| | A9-30-4 | 1.3499 | |

The results showed that the tumors in the control group grew rapidly, reaching an average tumor tissue weight of approximately 2.53 g on D19. On Day 19, the average tumor tissue weights of the MNH05026, MNH22004, and MNH27256 experimental groups were approximately 1.78 g, 1.44 g, and 1.56 g, respectively, and their tumor growth rates were significantly slower than that of the control group. This experiment proves that the *Megasphaera* strains can significantly reduce the growth rate of liver cancer.

In order to more conveniently and intuitively compare the anti-tumor effects of MNH05026, MNH22004, and MNH27256, the aforementioned experimental data were summarized in Table 17.

**Table 17. Anti-tumor results of the Megasphaera strains of the present disclosure (liver cancer)**

| | | | | | |
|---|---|---|---|---|---|
| anti-tumor Effect Strain | MNH05026 | MNH22004 | MNH27256 | Control group | LPS + IFNγ Positive Control Group |
| Average size of tumors dissected from mice (mm³) | 1566 | 1896 | 1367 | 2372 | - |
| Tumor tissue weight after dissection of mice (g) | 1.778 | 1.44 | 1.56 | 2.53 | - |
| Regulation of secretion of IL-1β by Primary PBMCs (pg/ml) | 11169.08 | 12802.8 | 11623.7 | 696.18 | - |
| Regulation of secretion of IFNγ by Primary PBMCs (pg/ml) | 26.222 | 26.22 | 25.3 | 15.27 | - |
| Regulation of the immune activity of macrophages IL-1β (pg/ml) | 4205.225 | 523.24 | 3773.69 | 253.73 | 347.23 |

As can be seen from Table 17, the *Megasphaera* strains screened in the present disclosure with high integrity of the butyrate pathway and strong butyrate production capacity can regulate the immune activities of macrophages and peripheral blood mononuclear cells (PBMCs). Moreover, it is verified by the mouse experiments that the *Megasphaera* strains of the present disclosure can inhibit tumor growth, reduce tumor volume and weight, and reduce tumor growth rate.

### (II) Experiment for verifying the therapeutic effect of the strain MNH05026 on tumor:

Further experiments were carried out to verify the therapeutic effects of MNH05026 and MNH27256 alone and in combination with anti-PD-1 and Levatinib on tumor (liver cancer), taking the *Megasphaera* strain MNH05026 as a representative.

### (2.1) Experiment for verifying the therapeutic effect of MNH05026 in combination with anti-PD-1 on liver cancer

Male BALB/C mice were be subcutaneously inoculated with H22 liver cancer cells to create an ectopic syngeneic tumor model. When the average tumor volume reached 80-100 mm³, the mice was randomized into 4 groups according to tumor volume: Group A (Control), Group B (anti-PD-1), Group C (A026), and Group D (A026 + anti-PD-1), 12 mice per group. MNH05026 (A026) and anti-PD-1 antibody intervention began on the day of grouping. During the experiment, animal characteristics, tumor changes, tumor sizes, and mouse weights were monitored regularly. At the experimental endpoint, body weight changes, tumor volume changes, response rates, and tumor growth inhibition rates were analyzed. The body weights of all mice were increased steadily throughout the experiment. At the experimental endpoint, the tumor volumes in the anti-PD-1 monotherapy group, the MNH05026 (A026) monotherapy group, and the MNH05026 (A026) + anti-PD-1 combination group showed a significant decrease, as compared to the Control group.

Fig. 16 shows the therapeutic effect of MNH05026 alone or in combination with anti-PD-1 on liver cancer. In Fig. 16(A), the mouse tumor volume data are presented as mean ± standard deviation (mm³). Statistical analysis was performed using two-way ANOVA with Tukey's multiple comparisons. Significance of difference vs. the Control group: not significant (ns), p value ≥ 0.05; *, p value < 0.05; **, p value < 0.01; ***, p value < 0.001; ****, p value < 0.0001. Fig. 16(A) shows the tumor volume change curves of each group of mice at different intervention time points. In Fig. 16(B), the mouse tumor volume data at the intervention endpoint (D27) are presented as mean ± standard deviation (mm³). Statistical analysis was performed using One-way ANOVA with Dunnett's multiple comparisons. Significance of difference vs. the Control group: not significant (ns), p value ≥ 0.05; *, p value < 0.05; **, p value < 0.01; ***, p value < 0.001.

Fig. 16(B) shows a comparison of the volumes at the intervention endpoint of each group of mice. Figs. 16(A)-16(B) showed that MNH05026 (A026) exhibited a significant inhibitory effect on tumor growth when used alone or in combination with anti-PD-1.

In Figs. 16(C)-16(F), the tumor volume data of individual mice in each drug intervention group are presented as mean ± standard deviation (mm³). The upper dotted line represents the average tumor volume of the Control group (2233.92 mm³), and the lower dotted line represents the tumor volume at 40% tumor growth inhibition rate relative to the Control group (893.57 mm³). A mouse tumor volume > 893.57 mm³ indicates a tumor growth inhibition rate < 40%, which represents an anti-tumor efficacy of unresponsiveness. A mouse tumor volume ≤ 893.57 mm³ indicates a tumor growth inhibition rate ≥ 40%, which represents an anti-tumor efficacy of responsiveness. Non-response rate = Number of unresponsive mice / Total number of mice in the group × 100%. Response rate = Number of responsive mice / Total number of mice in the group × 100%. Tumor disappearance rate = Number of mice with tumor disappeared at the endpoint / Total number of mice in the group × 100%.

The response rate of each mouse to the anti-tumor efficacy of MNH05026 (A026) was further evaluated. Figs. 16(C)-16(F) showed that in the comparisons of anti-tumor response rates and tumor growth inhibition rates, compared to the non-response rate of 75% and the response rate of 25% in the Control group, the MNH05026 (A026) monotherapy group showed an average response rate of 66.67% and a tumor growth inhibition rate of 52.1 ± 35.7%; the anti-PD-1 monotherapy group showed an average response rate of 71.43% and a tumor growth inhibition rate of 65.8 ± 34.8%; and the MNH05026 (A026) + anti-PD-1 combination group showed an average response rate of 36.36% and a tumor growth inhibition rate of 57.1 ± 25.1%. In summary, the groups of anti-PD-1 alone, MNH05026 (A026) alone, and MNH05026 (A026) + anti-PD-1 combination all showed significant inhibitory effects on H22 liver cancer. MNH05026 (A026) exhibited a significant anti-liver cancer efficacy in the H22 mouse liver cancer treatment model, and the effect was comparable to that of the anti-PD-1 antibody, but the combination with anti-PD-1 antibody did not exhibit a better enhancing effect.

Fig. 16(G) shows a comparison of tumor growth inhibition rates at the intervention endpoint of each group of mice. Tumor growth inhibition rate = (Average volume in the control group - Volume in the experimental group) / Average volume in the control group × 100%. The mouse tumor growth inhibition rate data are presented as mean ± standard deviation (%). Statistical analysis was performed using One-way ANOVA with Dunnett's multiple comparisons. Significance of difference vs. the Control group: not significant (ns), p value ≥ 0.05; *, p value < 0.05; **, p value < 0.01; ***, p value < 0.001. As compared to the Control group, the A026 bacterial solution monotherapy group showed a tumor growth inhibition rate of 52.1 ± 35.7%; the anti-PD-1 monotherapy group showed a tumor growth inhibition rate of 65.8 ± 34.8%; and the A026 bacterial solution + anti-PD-1 combination group showed a tumor growth inhibition rate of 57.1 ± 25.1%. The groups of anti-PD-1 alone, A026 bacterial solution alone, and A026 bacterial solution + anti-PD-1 combination all showed significant inhibitory effects on H22 liver cancer.

Further comparison of the tumor growth inhibition rates and the above results showed that MNH05026 (A026) alone showed a good anti-tumor efficacy.

This experiment proves that MNH05026 alone or in combination with anti-PD-1 can significantly reduce the growth rate of liver cancer.

### (2.2) Experiment for verifying the therapeutic effect of MNH05026 in combination with Levatinib on liver cancer:

104 male BALB/c mice were purchased, and inoculated with tumor cells after the quarantine was completed, to establish an ectopic syngeneic liver cancer model. The day of tumor cell inoculation was D1. At D6, the average tumor volume was 87.31 mm³. Mice with abnormal tumor growth were discarded and the remaining mice were randomized into 10 groups according to the tumor volume on that day, with 7 mice per group. The average tumor volume of the enrolled mice was 87.59 mm³. Group A was the Negative Control group, Group B was the positive control Lenvatinib group, Group J was the Lenvatinib + MNH05026 combination group, and Groups C-I received other test articles in combination with Lenvatinib. On the day of grouping, interventions were carried out according to the grouping. During the experiment, animal characteristics, tumor changes, tumor sizes, and mouse weights were monitored regularly. At the experimental endpoint, body weight changes, tumor volume changes, response rates, and tumor growth inhibition rates were analyzed. Throughout the experiment, the body weights of all mice in groups A and B were increased steadily, while the body weights in group J were increased steadily after a brief decrease 3 days after administration. At the experimental endpoint, the tumor volumes in the Lenvatinib group and the MNH05026 + Lenvatinib combination group showed a significant decrease, as compared to the Control group. As compared to the Lenvatinib monotherapy group, the tumors in the MNH05026 + Lenvatinib combination group also showed a downregulation trend.

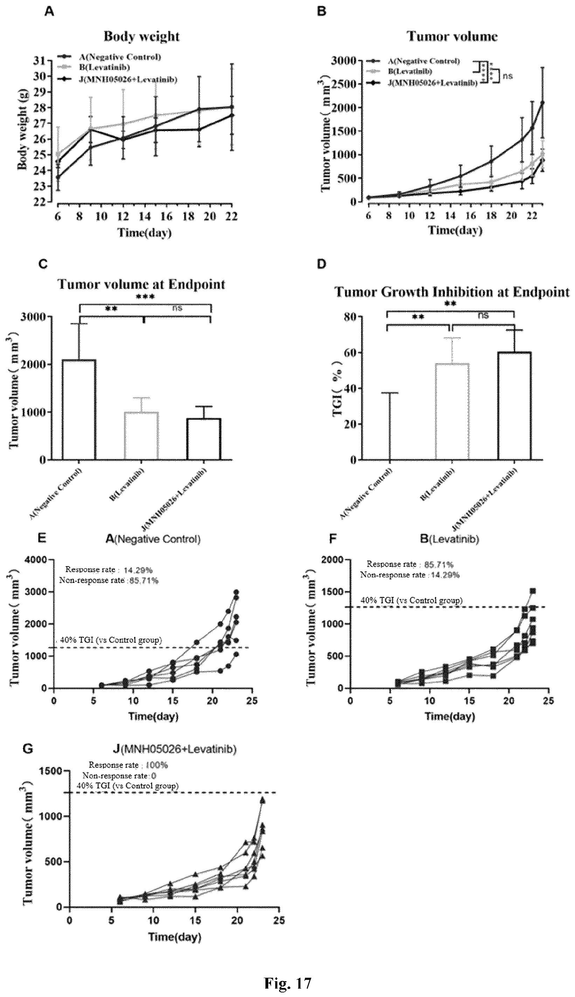

Fig. 17 shows the therapeutic effect of MNH05026 combined with Levatinib on liver cancer, in which Fig. 17(A) shows the body weight change curves of mice in different experimental groups during the intervention; Fig. 17(B) shows the tumor volume change curves of mice in different experimental groups during the intervention; Fig. 17(C) shows a comparison of tumor volumes of mice in different experimental groups at the intervention endpoint; Fig. 17(D) shows a comparison of tumor growth inhibition rates of mice in different experimental groups at the intervention endpoint; and Figs. 17(E)-(G) show the response rate of each mouse to the anti-tumor efficacy after treatment in different experimental groups, in which Fig. 17(E) shows the Control group; Fig. 17(F) shows the Levatinib monotherapy group; and Fig. 17(G) shows the MNH05026 + Levatinib combination group.

Specifically, Fig. 17(A) shows the body weight change curves of each group of mice at different intervention time points. The body weights of all mice in the negative control group A and the monotherapy group B were increased. The body weights of mice in the combination group were significantly decreased during the period of days 9-12, and remained substantially stable during the period of days 15-19. Statistical analysis was performed using Two-Way ANOVA with Dunnett's multiple comparisons at the experimental endpoint. Significance of difference vs. the Control group: not significant (ns), p value ≥ 0.05; *, p value < 0.05; **, p value < 0.01; ***, p value < 0.001; ****, p value < 0.0001.

Fig. 17(B) shows the tumor volume change curves of each group of mice at different intervention time points; Fig. 17(C) shows the tumor volumes of each group of mice at the endpoints; and Fig. 17(D) shows the TGI of the mice at the endpoints. Figs. 17(B) and 17(C) present the mouse tumor volume data as mean ± standard deviation (mm³). Statistical analysis was performed using Two-Way ANOVA with Dunnett's multiple comparisons at the experimental endpoint. Significance of difference vs. the Control group: not significant (ns), p value ≥ 0.05; *, p value < 0.05; **, p value < 0.01; ***, p value < 0.001; ****, p value < 0.0001.

Figs. 17(E)-17(G) show the response rate results. As compared to the response rate of 14.29% in the Control group, the response rate of the Lenvatinib monotherapy group was significantly increased to 85.71%, and the response rate of the MNH05026 + Lenvatinib combination group was as high as 100%.

In summary, the combination of MNH05026 and Lenvatinib exhibited a better therapeutic effect on liver cancer by reducing the growth of liver cancer and improving the response rate in mice.

### (III) Experiment for verifying the therapeutic effects of MNH05026 and MNH27256 on lung cancer:

### (3.1) Experiment for verifying the therapeutic effect of MNH05026 in combination with anti-PD-1 on lung cancer:

120 male C57BL/6J mice were purchased, and inoculated with tumor cells after the quarantine was completed, to establish an ectopic syngeneic lung cancer model. The day of tumor cell inoculation was D1. At D5, the average tumor volume was 77.81 mm³. Mice with abnormal tumor growth were discarded and the remaining mice were randomized into 10 groups according to the tumor volume on that day, with 8 mice per group. The average tumor volume of the enrolled mice was 77.79 mm³. Group A was the Negative Control group, Group B was the positive control anti-PD-1 group, Group C was the anti-PD-1 + MNH05026 combination group, and Groups D-J received other test articles in combination with anti-PD-1. On the day of grouping, interventions were carried out according to the grouping. During the experiment, animal characteristics, tumor changes, tumor sizes, and mouse weights were monitored regularly. At the experimental endpoint, body weight changes, tumor volume changes, response rates, and tumor growth inhibition rates were analyzed. The body weights of all mice in groups A to C were increased steadily throughout the experiment. At the experimental endpoint, as compared to the Control group, the tumor volumes in the anti-PD-1 group and the anti-PD-1 + MNH05026 combination group showed a significant decreasing trend, in which the tumor volume in the anti-PD-1 + MNH05026 combination group was significantly downregulated. As compared to the anti-PD-1 monotherapy group, the tumors in the MNH05026 + anti-PD-1 combination group also showed a downregulation trend.

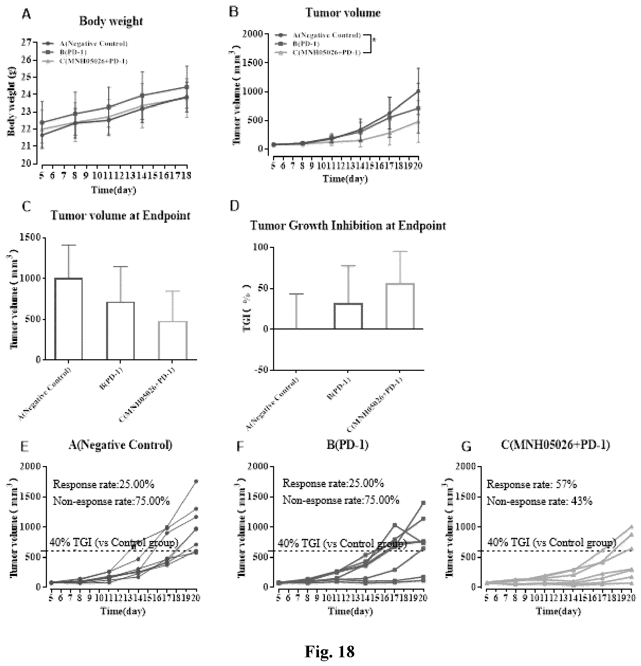

Fig. 18 shows the therapeutic effect of MNH05026 combined with anti-PD-1 on lung cancer, in which Fig. 18(A) shows the body weight change curves of mice in different experimental groups during the intervention; Fig. 18(B) shows the tumor volume change curves of mice in different experimental groups during the intervention; Fig. 18(C) shows a comparison of tumor volumes of mice in different experimental groups at the intervention endpoint; Fig. 18(D) shows a comparison of tumor growth inhibition rates of mice in different experimental groups at the intervention endpoint; and Figs. 18(E)-(G) show the response rate of each mouse to the anti-tumor efficacy after treatment in different experimental groups, in which Fig. 18(E) shows the Control group; Fig. 18(F) shows the anti-PD-1 monotherapy group; and Fig. 18(G) shows the MNH05026 + Levatinib combination group.

Specifically, Fig. 18(A) shows the body weight change curves of each group of mice at different intervention time points. The body weights of all mice in groups A to C were increased steadily, with no significant difference among the three groups. Statistical analysis was performed using Two-Way ANOVA with Dunnett's multiple comparisons at the experimental endpoint. Significance of difference vs. the Control group: not significant (ns), p value ≥ 0.05; *, p value < 0.05; **, p value < 0.01; ***, p value < 0.001; ****, p value < 0.0001.

Fig. 18(B) shows the tumor volume change curves of each group of mice at different intervention time points; Fig. 18(C) shows the tumor volumes of each group of mice at the endpoints; and Fig. 18(D) shows the TGI of the mice at the endpoints. Figs. 18(B) and 18(C) present the mouse tumor volume data as mean ± standard deviation (mm³). Statistical analysis was performed using Two-Way ANOVA with Dunnett's multiple comparisons at the experimental endpoint. Significance of difference vs. the Control group: not significant (ns), p value ≥ 0.05; *, p value < 0.05; **, p value < 0.01; ***, p value < 0.001; ****, p value < 0.0001.

Figs. 18(E)-18(G) show the response rate results. As compared to the response rate of 25% in the Control group and the anti-PD-1 monotherapy group, the response rate of the MNH05026 + anti-PD-1 combination group was increased to 57%.

In summary, the combination of MNH05026 and anti-PD-1 exhibited a better therapeutic effect on lung cancer by reducing the growth of lung cancer and improving the response rate in mice.

### (3.2) Experiment for verifying the therapeutic effect of MNH27256 on lung cancer:

Further experiment was carried out to verify the therapeutic effect of the *Megasphaera* strain on lung cancer, taking the *Megasphaera* strain MNH27256 as a representative.

To verify whether the strain MNH27256 can be used to prevent and treat lung cancer, a lung cancer growth inhibition experiment was conducted using a mouse syngeneic tumor model. This study has been reviewed by the Care and Use Committee of Moon Biotech.

Test strain: A glycerol cryovial of the strain MNH27256 was thawed at 37 °C, and then inoculated onto an anaerobic blood plate in an anaerobic workstation for activation. The activated strain was inoculated into MM01 liquid medium and anaerobically cultured to obtain a sufficient amount of cultures. The cultured bacterial solution was centrifuged, concentrated, and then resuspended in solvent to afford a test article with a purity and viable bacterial count (2.04 × 10¹⁰ CFU/mL) meeting the requirements of animal experiments.

Tumor cells: LLC1 mouse lung cancer cells, CL-0140.

Experimental animals: C57BL/6J mice, aged 5 weeks, 120 mice in total, purchased from Guangdong GemPharmatech Co., Ltd.

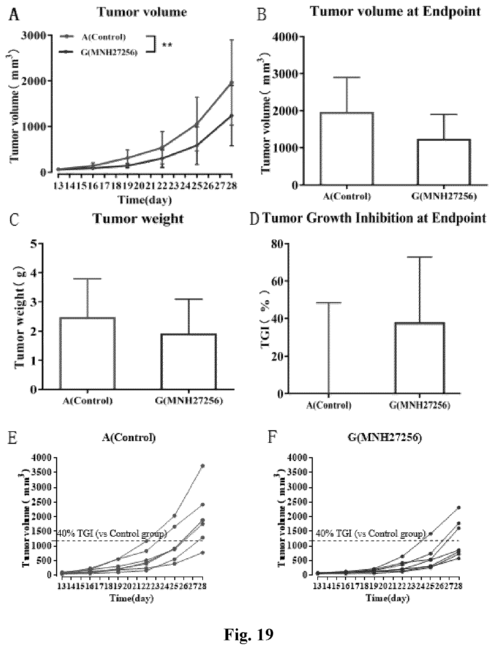

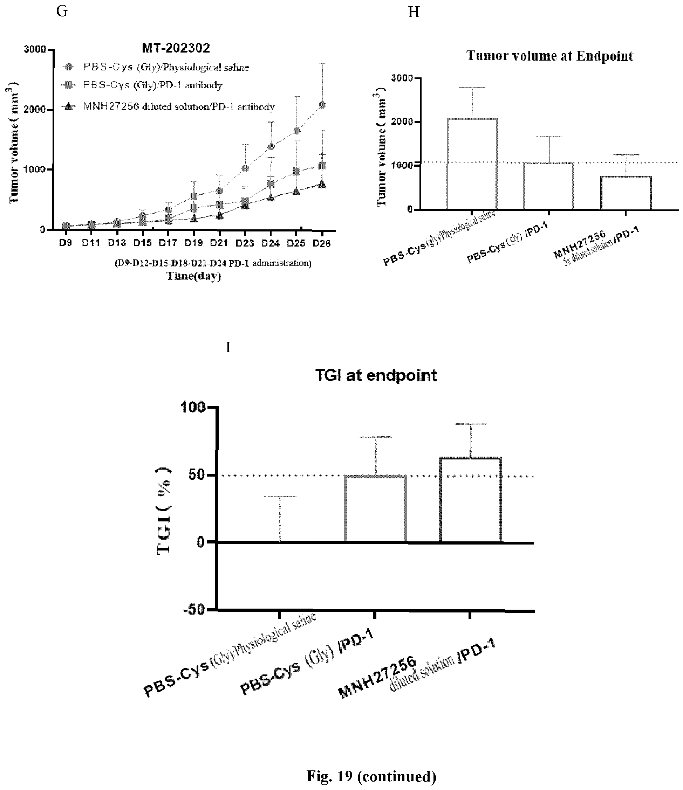

Animal experiment: The mice were normally fed. Upon completion of the quarantine period, the mice were subcutaneously inoculated with LLC1 lung cancer cells in a cell inoculation amount of 2 × 10⁶/mL, 0.1 mL/animal, to create an ectopic syngeneic tumor model. When the average tumor volume reached 80-100 mm³, the mice was randomized into 10 groups according to tumor volume: Group A (Control), Group G (MNH27256), and other groups receiving other test articles. Gavage administration was initiated on the day of grouping (D1). The control group was given a solvent, and Group G was given the MNH27256 bacteria. The volume of the gavage administration was 0.2 mL/animal/dose, and the frequency of administration was once per day. General observation was conducted once a day during the quarantine period and after the daily dose during the administration. The animals were weighed upon arrival, at the end of the quarantine period, and three times a week during the administration. The day of tumor cell inoculation was D1. The tumor diameter was measured once a day from D5 to D11, every other day from D11 to D13, every 3 days after grouping, and once a day upon the average tumor volume ≥ 1000 mm³, and the tumor growth was recorded. The endpoint of this experiment was as follows: the average tumor volume in any one of groups B to J was ≥ 1000 mm³ and significantly smaller than that in Group A, or the average tumor growth inhibition rate (TGI) was ≥ 30%; or the average tumor volume in any group of mice was greater than 2000 mm³. General clinical observation, body weight monitoring, and tumor volume (mm³) measurement were conducted during the experiment. After the experimental endpoint was reached, all the surviving mice were dissected and sampled, tumor weight was measured and tumor volume was calculated, and finally, statistical analyses and comparisons were performed for tumor volume curve change (Tumor volume), tumor volume at the endpoint, tumor weight at the endpoint, tumor growth inhibition rate (TGI) at the endpoint, etc. Tumor growth inhibition rate (TGI) = 1 - (Volume of individual tumor - Volume of individual tumor at grouping) / (Average tumor volume of Group A - Average tumor volume of Group A at grouping) × 100%. All data were expressed as mean ± SD, and plotted and statistically analyzed using the GraphPad Prism 8.0.2 software. For pairwise comparisons, a t-test (Student's t test) was used. Two-way analyses were performed by 2-way ANOVA with Sidak's multiple comparisons. The significance of difference was indicated by *: * p < 0.05, ** p < 0.01, *** p < 0.001, **** p < 0.0001. The experimental results are shown in Table 18 and Fig. 19.

**Table 18. Overall statistics of mice at the experimental endpoint:**

| | Number of mice with successful tumor inoculation | Number of mice at experimental endpoint | Number of euthanized mice | Average weight of dissected tumors (g) | Average body weight prior to dissection (g) | Average size of dissected tumors (mm³) |
|---|---|---|---|---|---|---|
| Control group | 8 | 7 | 1 | 2.475 | 26.46 | 1962.17 |
| Experimental group | 8 | 7 | 1 | 1.925 | 26.46 | 1233.67 |

Fig. 19 shows the therapeutic effect of MNH05026 alone on lung cancer, in which Fig. 19(A) shows the tumor volume change curves of mice in different experimental groups during the intervention; Fig. 19(B) shows a comparison of tumor volumes of mice in different experimental groups at the intervention endpoint; Fig. 19(C) shows a comparison of tumor weights of mice in different experimental groups at the intervention endpoint; Fig. 19(D) shows a comparison of tumor growth inhibition rates of mice in different experimental groups at the intervention endpoint; and Figs. 19(E) and (F) show the response rate of each mouse to the anti-tumor efficacy in the Control group and the MNH27256 treatment group, respectively.

As shown in Fig. 19(A), the statistical analysis results of the tumor volume curves in Group G (MNH27256) were significantly smaller than those in Group A (Control). As shown in Table 18, Fig. 19(B), and Fig. 19(C), at the experimental endpoint, the average tumor volume of the mice was 1962.17 mm³ and the average tumor tissue weight was about 2.475 g in the control group; while the average tumor volume of the mice was 1233.67 mm³ and the average tumor tissue weight was about 1.925 g in the MNH27256 treatment group. The tumor volume and tumor weight of the mice in the MNH27256 treatment group were significantly smaller than those of the control group. As shown in Fig. 19(D), at the experimental endpoint, the tumor growth inhibition rate (TGI) of Group G (MNH27256) was 38%, which was significantly higher than that of the control group, suggesting a significant effect of inhibiting tumor growth. As shown in Figs. 19(E) and 19(F), at the experimental endpoint, the response rate of the control group (A (Negative Control)) was 14.29%, and the response rate of the MNH27256 experimental group (G (MNH27256)) was 57.14%. MNH27256 significantly improved the response rate to the treatment of lung cancer.

### (3.3) Experiment for verifying the therapeutic effect of MNH27256 in combination with anti-PD-1 on lung cancer:

The experimental method in this experiment was the same as (2.1). 108 male C57BL/6J mice were purchased, and inoculated with tumor cells after the quarantine was completed, to establish an ectopic syngeneic liver cancer model. The day of tumor cell inoculation was D1. At D9, mice with abnormal tumor growth were discarded and the remaining mice were randomized into 10 groups according to the tumor volume on that day, with 8 mice per group. Group A was the Negative Control group, Group B was the positive control anti-PD-1 group, Group H was the anti-PD-1 + MNH27256 combination group, and the remaining groups received other test articles in combination with anti-PD-1. On the day of grouping, interventions were carried out according to the grouping. During the experiment, animal characteristics, tumor changes, tumor sizes, and mouse weights were monitored regularly. At the experimental endpoint, body weight changes, tumor volume changes, response rates, and tumor growth inhibition rates were analyzed.

The body weights of all mice were increased steadily throughout the experiment. At the experimental endpoint, as compared to the Control group, the tumor volumes in the anti-PD-1 group and the anti-PD-1 + MNH27256 combination group showed a significant decreasing trend, in which the tumor volume in the anti-PD-1 + MNH27256 combination group was significantly downregulated.

Fig. 19G shows the tumor volume change curves of each group of mice at different intervention time points, in which the mouse tumor volume data are presented as mean ± standard deviation (mm³). Statistical analysis was performed using Two-Way ANOVA with Dunnett's multiple comparisons at the experimental endpoint. Significance of difference vs. the Control group: not significant (ns), p value ≥ 0.05; *, p value < 0.05; **, p value < 0.01; ***, p value < 0.001; ****, p value < 0.0001.

Fig. 19H shows the tumor volume of each group of mice at the endpoint, in which the mouse tumor volume data are presented as mean ± standard deviation (mm³). Statistical analysis was performed using Two-Way ANOVA with Dunnett's multiple comparisons at the experimental endpoint. Significance of difference vs. the Control group: not significant (ns), p value ≥ 0.05; *, p value < 0.05; **, p value < 0.01; ***, p value < 0.001; ****, p value < 0.0001.

FIG. 19I shows the TGI of the mice at the endpoint. The mouse tumor volume data are presented as mean ± standard deviation (mm³). Statistical analysis was performed using Two-Way ANOVA with Dunnett's multiple comparisons at the experimental endpoint. Significance of difference vs. the Control group: not significant (ns), p value ≥ 0.05; *, p value < 0.05; **, p value < 0.01; ***, p value < 0.001; ****, p value < 0.0001. In summary, the combination of MNH27256 and anti-PD-1 exhibited a better therapeutic effect on lung cancer by reducing the growth of lung cancer in mice.

### Example 8. Immunoassay of tumor-bearing mice activated by the strains MNH22004 and MNH27256 (A9)

Fig. 20 shows an immunoassay of tumor-bearing mice activated by the strain MNH27256.

Spleen tissue was removed from the tumor-bearing mice at the experimental endpoint in the animal experiment on treatment of liver cancer with MNH27256 in Example 7.

The tissue from the tumor-bearing mice was prepared into a single-cell suspension and then labeled with specific molecule with a fluorescent molecular marker. The proportion of cells expressing the specific molecule was detected by flow cytometry. The proportional distribution of immune cells reflects the immune status of the mice to a certain extent.

### I. Tumor tissue

### 1. Preparation of tumor single-cell suspension and cell counting

0.3 g of tumor tissue was subjected to surface rinsing with PBS, cut into small pieces, and pulverized. The pulverized tumor tissue was placed into a 5 mL collagenase IV digestion solution system (1 mg/ml collagenase IV, 0.1 mg/ml DNase, and 10% FBS) and digested at 37 °C for 30 min. The digested tissue solution was filtered through a 70 um filter membrane to obtain a mouse tumor single-cell suspension. The total number of cells was counted by on-machine detection.

### 2. Staining of T cells/NK cells on the surface of tumor cells

The dyes (corresponding antibody combinations) T-Live-Tumor/NK-Live-Tumor and T-Tumor/NK-Tumor were respectively added to the mouse tumor single-cell suspension for staining of T cells/NK cells on the surface of tumor cells. The stained cells were resuspended in PBS and subjected to on-machine detection.

### 3. Staining for intracellular transcription factor Foxp3 in tumor cells

The dyes (corresponding antibody combinations) Treg-Live-Tumor and Treg-Surface-Tumor were successively added to the mouse tumor single-cell suspension for staining. The stained cells were resuspended in PBS, then treated with a fixative and a membrane-rupturing solution successively, and stained by Treg-Foxp3-Tumor intranuclear staining according to the instructions of the detection kit. The stained cells were resuspended in PBS and subjected to on-machine detection.

### II. Spleen tissue

### 1. Preparation of spleen single-cell suspension and cell counting

0.1 g of spleen tissue was subjected to surface rinsing with PBS and then subjected to tissue pulverization. The pulverized spleen tissue was resuspended in PBS and filtered through a 70 um filter membrane to obtain a mouse spleen single-cell suspension, which was subjected to on-machine detection.

Red blood cell lysis solution, Spleen-Counting, pre-cooled PBS, etc. were successively added to a small amount of the mouse spleen single-cell suspension as prepared above. On-machine detection was performed, and the mouse spleen single-cell number was accurately counted.

Red blood cell lysis solution, PBS, etc. were successively added to the remaining mouse spleen single-cell suspension as prepared above for cell processing. The processed cell suspension passed through a 300-mesh filter membrane and then was subjected to on-machine detection.

### 2. Staining of T cells/NK cells on the surface of spleen cells

The single-cell suspension was fully resuspended. 30 µl of the suspension was transferred to a 96-well round-bottom plate and centrifuged at 500 g for 5 min to discard the supernatant. 30 µl of T-Live staining solution (Mouse BD Fcblock, Live/Dead Fixable Green Dead Stain) was added to a single sample for staining for 15 min at room temperature in the dark. Upon completion of the staining, it was resuspended in 200 µl of PBS and centrifuged at 500g for 5 min to discard the supernatant. 30 µl of T staining solution (Percp/Cyanine5.5 anti-mouse CD45; APC anti-mouse CD3; PE-Cy^{™}7 Rat anti-mouse CD4; PE Rat Anti-Mouse CD8a) was added to a single sample for staining for 15 min at room temperature in the dark. Upon completion of the staining, it was resuspended in 200 µl of PBS and centrifuged at 500g for 5 min to discard the supernatant. The cells were resuspended in 150 µl of PBS and subjected to on-machine detection. The data collection was terminated when CD45 was gated.

30 µl of NK-Live staining solution (Mouse BD Fcblock, Live/Dead Fixable Green Dead Stain) was added to a single sample for staining for 15 min at room temperature in the dark. Upon completion of the staining, it was resuspended in 200 µl of PBS and centrifuged at 500g for 5 min to discard the supernatant. 30 µl of NK staining solution (Percp/Cyanine5.5 anti-mouse CD45 APC/Cyanine7 anti-mouse CD3ε APC anti-mouse NK-1.1 PE anti-mouse CD19) was added to a single sample for staining for 15 min at room temperature in the dark. Upon completion of the staining, the time of staining completion was recorded, and it was resuspended in 200 µl of PBS and centrifuged at 500g for 5 min to discard the supernatant. The cells were resuspended in 150 µl of PBS and subjected to on-machine detection. The detection speed was medium speed. The data collection was terminated when CD45 was gated.

The proportions of T cells (CD3/CD4/CD8) in spleen tissue were detected and analyzed (see Fig. 20a). As compared to the control group, the proportions of CD3/CD4/CD8 cells in mouse spleen were significantly upregulated. This shows that MNH27256 (A9) can activate the systemic immunity of mice by increasing the proportions of T cells in mouse spleen and exert an anti-tumor effect.

The proportion of CD4 T cells in tumor tissue was detected and analyzed (see Figs. 20b-20d). As compared to the control group, the proportion of CD4 cells in mouse tumor tissue was significantly upregulated. This shows that MNH27256 (A9) can activate local immunity in mouse tumor by increasing the proportion of T cells in mouse tumor and exert an anti-tumor effect.

The data were analyzed by CyExpert and statistically processed by GraphPad Prism V9. Statistical analysis was performed using one-way ANOVA with Dunnet's multiple comparisons: * p < 0.05, ** p < 0.01, *** p < 0.001.

Therefore, the strain MNH27256 can exert an anti-tumor activity by stimulating activation of the immune system of mice.

Based on the same experimental method, in the tumor-bearing mouse activation immunoassay, the strain MNH22004 also caused a significant upregulation of the proportions of CD3/CD4/CD8 cells in mouse spleen and a significant upregulation of the proportion of CD4 cells in mouse tumor tissue. This suggests that MNH22004 can increase the proportion of T cells in mouse spleen to activate the systemic immunity of mice and exert an anti-tumor effect. It also suggests that MNH22004 can increase the proportion of T cells in mouse tumor to activate local immunity in mouse tumor and exert an anti-tumor effect.

### Example 9. Inhibitory effects of the strains MNH22004 and MNH05026 on the activity of histone deacetylases (HDACs)

In order to verify whether MNH22004 and MNH05026 have an inhibitory effect on the activity of histone deacetylases, an HDAC Inhibitor Drug Screening Kit (Fluorometric) purchased from Abcam Co. was used in this study to detect the inhibitory effect on HDAC activity *in vitro.*

Preparation of culture supernatant of MNH22004: The strain MNH22004 was inoculated into a liquid culture medium, anaerobically cultured at 37 °C for 48 h, and centrifuged to remove bacterial cells. The culture supernatant was filtered with a 0.22 µm filter, aliquoted, and stored at -80 °C for later use.

Preparation of culture supernatant of MNH05026: The strain MNH05026 was inoculated into a liquid culture medium, anaerobically cultured at 37 °C for 48 h, and centrifuged to remove bacterial cells. The culture supernatant was filtered with a 0.22 µm filter, aliquoted, and stored at -80 °C for later use.

Preparation of test samples: 1) Control group: MM01 culture medium was diluted 10 times with PBS to obtain 10% MM01; 2) Positive control group: TSA, an HDAC inhibitor, was diluted with PBS to a final concentration of 40 µM; 3) MNH22004 group: the culture supernatant of MNH22004 was diluted 10 times with PBS to obtain an experimental sample containing 10% bacterial supernatant; 4) MNH05026 group: the culture supernatant of MNH05026 was diluted 10 times with PBS to obtain an experimental sample containing 10% bacterial supernatant.

Preparation of reaction reagent for HDAC detection: An appropriate amount of detection reaction system was prepared according to the instructions of the kit. 50 µL of the reaction reagent was required for each reaction.

Detection of HDAC activity: 50 µL of the test sample was added to a 96-well white plate, and then 50 µL of the reaction reagent was added respectively. They were mixed well and incubated at 37 °C for 30 min. 10 µL of Lysine Developer was added to the reaction well and mixed well to terminate the reaction. The plate was incubated at 37 °C for 30 min. Finally, the fluorescence intensity of the sample was detected using a microplate reader with the following microplate reader settings: Ex. = 350-380 nm, and Em. = 440-460 nm. To analyze the HDAC inhibitory activity of the sample, the fluorescence intensity value of the Control was set as 100%. The fluorescence intensities of the positive control group and the MNH22004 group were respectively divided by that of the Control and then multiplied by 100% to obtain the relative HDAC activities.

HDAC inhibitors exhibit good anti-tumor effects on a variety of tumor cells, including bladder, bone, breast, uterus, central nervous system, esophagus, lung, ovary, pancreas, bile duct, prostate cancer cells. They can cause obvious apoptosis, proliferation inhibition, and cell cycle arrest in these tumor cells. HDACs mainly regulate the onset of cancer and are described as oncogenes. Reducing or inhibiting HDAC expression activity has been shown to have multiple anti-cancer effects. Trichostain-A (TSA) is one of HDAC inhibitors. Literatures have shown that TSA can upregulate the expression of the cyclin kinase inhibitors p21 and p27, downregulate the levels of cyclin A, cyclin D1, and cyclin D2, and inhibit CDK activation, thereby arresting tumor cells at G1 or G2 phase, blocking the continued growth of tumor cells (such as MCF-7 breast cancer cells and HeLa cervical cancer cells), and promoting their apoptosis (See, Zhou Yi, Role and mechanism of Trichostain A in tumor immunotherapy, Chinese Medicinal Biotechnology). Studies have shown that TSA inhibits tumor angiogenesis mainly by downregulating the expression of genes related to tumor angiogenesis caused by hypoxia and directly inhibiting the migration and proliferation of endothelial cells. Yang, *et al.* (see, Yang QC, Zeng BF, Shi ZM, et al., Inhibition of hypoxia-induced angiogenesis by trichostatin A via suppression of HIF-1a activity in human osteosarcoma. J Exp Clin Cancer Res, 2006, 25(4):593-599) found that in human osteosarcoma, TSA inhibited tumor angiogenesis by inhibiting the expression of HIF-1α and VEGF, inducing the degradation of their receptors, and upregulating the transcription of p53 and VHL, through, for example., cell cycle arrest, proliferation inhibition, apoptosis, differentiation, senescence, and disruption of angiogenesis. Therefore, the strains of the present disclosure have HDAC inhibitory activity, suggesting that the drug or composition of the present disclosure can be used to prevent or treat tumors or cancers mediated by HDAC activity. Since the genes regulated by HDAC play an important role in angiogenesis, the drug or composition of the present disclosure can be used to prevent tumor metastasis.

HDAC inhibitors also have anti-fibrosis effects. Diabetes is a group of diseases in which a low level of insulin and/or peripheral insulin resistance leads to hyperglycemia. It has been proposed to treat diabetes by inhibiting HDAC activity through multiple mechanisms, including inhibition of Pdxl (Park, et al., 2008, J Clin Invest, 118, 2316-24), and enhancement of the expression of the transcription factor Ngn3 to increase the endocrine repertoire, progenitor cells (Haumaitre, et al., 2008, Mol Cell Biol, 28, 6373-83), enhancement of insulin expression (Molsey, et al., 2003, J Biol Chem, 278, 19660-6), etc. HDAC inhibition is also a promising therapy for advanced diabetic complications such as diabetic nephropathy and retinal ischemia (Christensen, et al., 2011, Mol Med, 17(5-6), 370-390). Therefore, the composition of the present disclosure can be used to treat or prevent diabetes mediated by HDAC activity.

Fig. 21 is a graph showing the inhibitory effects of MNH22004 and MNH05026 on deacetylase activity.

The results (see Fig. 21) showed that, as compared to the HDAC activity in the Control group, the supernatant of MNH22004 had a significant inhibitory effect on HDAC activity, which was similar to the effect of TSA in the HDAC inhibitor control group. These results suggest that MNH22004 can inhibit HDAC activity, and therefore can be used to prevent or treat diseases mediated by HDAC activity by inhibiting HDAC activity.

The results (see Fig. 21) also showed that, as compared to the HDAC activity in the Control group, the supernatant of MNH05026 had a significant inhibitory effect on HDAC activity, which was similar to the effect of TSA in the HDAC inhibitor control group. These results suggest that MNH05026 can inhibit HDAC activity, and therefore can be used to prevent or treat diseases mediated by HDAC activity by inhibiting HDAC activity.

The functions of HDACs in cancers are related to the aberrant expression or function of the genes that promote cell proliferation and tumorigenic phenotypes. In some cancers, HDACs primarily regulate the onset of the cancers and have been described as oncogenes. Reducing or inhibiting HDAC expression activity has been shown to have multiple anti-cancer effects, such as cell cycle arrest, proliferation inhibition, apoptosis, differentiation, senescence, and disruption of angiogenesis. Therefore, the strain MNH22004 of the present disclosure has an HDAC inhibitory activity, suggesting that the drug or composition of the present disclosure can be used to prevent or treat tumors or cancers mediated by HDAC activity. Since the genes regulated by HDAC play an important role in angiogenesis, the drug or composition of the present disclosure can be used to prevent tumor metastasis.

In certain embodiments, the composition of the prevent disclosure is used for the treatment or prevention of tumors, wherein the treatment or prevention is accomplished by reducing or preventing HDAC activation.

The strains MNH22004 and MNH05026 of the present disclosure can inhibit HDAC activity and activate T cells, thereby achieving anti-tumor effects.

The composition of the present disclosure has an HDAC inhibitory activity, and can be used for nervous system diseases, inflammatory bowel diseases such as IBD, and cardiovascular diseases according to the HDAC inhibitory activity (HDAC inhibition also results in beneficial outcomes in various types of neurodegenerative diseases, inflammation disorders, and cardiovascular diseases. HDAC and HDAC Inhibitor: From Cancer to Cardiovascular Diseases, https://pubmed.ncbi.nlm.nih.gov/26865995/). It can be determined that the strains of the present disclosure can also be used to treat nervous system diseases mediated by HDAC activity, inflammatory bowel diseases such as IBD, and cardiovascular diseases.

### Example 10. Effect of the strain MNH27256 on IFNβ expression

### 1.1 Experimental methods

Interferons (IFNs) are a group of cytokines secreted by monocytes and lymphocytes, which can regulate immune responses. Viruses, bacterial endotoxins, artificially synthesized double-stranded RNAs, or the like can stimulate the production of interferons. Macrophages, lymphocytes, somatic cells, etc. in the human body can all produce interferons. IFNβ belongs to type I interferons and can promote the activities of NK cells, macrophages, and T lymphocytes, thereby exerting anti-viral, anti-tumor, immunomodulatory, and other effects. In order to verify whether MNH27256 can promote IFNβ expression, the constructed THP-1 cells (a cell line constructed by Moon Co.) carrying the IFNβ-promoter reporter were used in this study to evaluate the effect of MNH27256 on the transcriptional activity of IFNβ.

The THP-1 cells carrying the IFNβ-promoter reporter were constructed by inserting the reporter gene into a vector, infecting cells with the vector, and screening the cell line expressing the reporter gene (specifically, see Reference 1: Huashan Du, Tianmin Xu, Manhua Cui, "cGAS-STING signaling in cancer immunity and immunotherapy", Biomedicine & Pharmacotherapy, 133 (2021) 110972; Reference 2: Jiang, et al., "cGAS-STING, an important pathway in cancer immunotherapy", Journal of Hematology & Oncology, (2020) 13:81; and Reference 3: Khiem C. Lam, et al., "Microbiota triggers STING-type I IFN-dependent monocyte reprogramming of the tumor microenvironment", Cell, 184, 5338-5356).

Preparation of culture supernatant of MNH27256: The strain MNH27256 was inoculated into MM01 liquid medium, anaerobically cultured at 37 °C for 48 h, and centrifuged to remove bacterial cells. The culture supernatant was filtered with a 0.22 µm filter, aliquoted, and stored at -80 °C for later use.

Control group: containing 10% by volume of MM01 bacterial culture medium.

MSA-2 (40 µM) group: MSA-2 (a Sting agonist) (from TargetMol, Cat No. T8798 (Target Molecule Corp.)) can promote IFNβ expression; Positive control.

MNH27256 group: containing 10% by volume of MNH27256 bacterial culture supernatant.

The THP-1-IFNβ-promoter reporter cells were inoculated in a 96-well plate, with 1×10⁵ cells per well. The cells were treated according to the group setting. After culturing for additional 24 h, the cells were centrifuged at 300 g for 5 min. The culture supernatant was discarded, and 50 µL of 1× luciferase detection reagent was added to allow for the reaction for 1 min. The chemiluminescence values were detected in a microplate reader. The relative luminescence values were normalized to the Control group and the effect of MNH27256 on IFNβ transcriptional activity was evaluated.

### 1.2 Experimental results

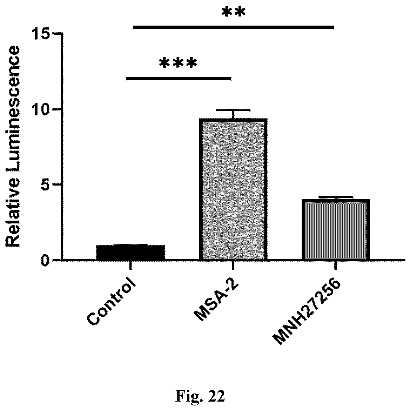

The results showed that MNH27256 significantly promoted the transcriptional activity of IFNβ (see Fig. 22). It is known to those skilled in the art that interferons (IFNs) are a group of cytokines secreted by host cells, which can regulate immune responses. Viruses, bacterial endotoxins, artificially synthesized double-stranded RNAs, or the like can stimulate the production of interferons. Macrophages, lymphocytes, somatic cells, etc. in the human body can all produce interferons. IFNβ belongs to type I interferons and can promote the activities of NK cells, macrophages, and T lymphocytes, thereby exerting anti-viral, anti-tumor, immunomodulatory, and other effects. These results indicate that MNH27256 has potential immunomodulatory, antiviral, and anti-tumor effects.

For the 16S rRNA sequence of the strain MNH05026, SEQ ID NO: 1 - see Sequence 1 in the sequence listing.

For the 16S rRNA sequence of the strain MNH22004, SEQ ID NO: 2 - see Sequence 2 in the sequence listing.

For the 16S rRNA sequence of the strain MNH27256, SEQ ID NO: 3 - see Sequence 3 in the sequence listing.

The above examples are only used to illustrate but not to limit the technical solutions of the present disclosure. Those of ordinary skill in the art should understand that the technical solutions described in the foregoing embodiments can be modified, or some or all of the technical features can be equivalently substituted without departing from the spirit and scope of the present disclosure. Such modifications or substitutions will not cause the essence of the corresponding technical solutions to deviate from the scope of technical solutions in the embodiments of the present disclosure.

## Claims

1. A bacterial strain of the genus *Megasphaera,* wherein the bacterial strain of the genus *Megasphaera* has a 16S rRNA sequence with at least 95%, 95.5%, 96%, 96.5%, 97%, 97.5%, 98%, 98.5%, 98.65%, 99%, 99.5%, 99.9%, or 100% identity to the 16S rRNA sequence of SEQ ID NO: 1, SEQ ID NO: 2, and/or SEQ ID NO: 3.

2. The bacterial strain of the genus *Megasphaera* according to claim 1, wherein the bacterial strain comprises a 16S rRNA sequence with at least 97%, 97.5%, 98%, 98.5%, 98.65%, 99%, 99.5%, 99.9%, or 100% identity to SEQ ID NO: 2 and/or SEQ ID NO: 3.

3. The bacterial strain of the genus *Megasphaera* according to claim 1, wherein the bacterial strain has a butyrate production pathway with at least 70% integrity;
preferably, the butyrate production pathway is pyruvate pathway and/or 4-aminobutyrate pathway;
preferably, the bacterial strain of the genus *Megasphaera* is highly productive of butyric acid and/or acetic acid.

4. The bacterial strain of the genus *Megasphaera* according to any one of claims 1 to 3, wherein the bacterial strain is one of the strains deposited with the Guangdong Microbial Culture Collection Center with deposit numbers of GDMCC No: 62001, GDMCC No: 62000, and/or GDMCC No: 61999.

5. The bacterial strain of the genus *Megasphaera* according to any one of claims 1 to 3, wherein the genome of the bacterial strain has an average nucleotide identity of greater than or equal to 79%, greater than or equal to 90%, greater than or equal to 95%, greater than or equal to 96%, greater than or equal to 97%, greater than or equal to 98%, greater than or equal to 99%, or greater than or equal to 99.5% to the genome of the strain according to claim 4; and/or
the genome of the bacterial strain has an alignment score of greater than or equal to 60%, greater than or equal to 70%, greater than or equal to 80%, greater than or equal to 90%, greater than or equal to 95%, greater than or equal to 96%, greater than or equal to 97%, greater than or equal to 98%, or greater than or equal to 99% to the genome of the strain according to claim 4;
preferably, the genome of the bacterial strain has an average nucleotide identity (ANI) value of at least 79%, 80%, 85%, 88%, 90%, 95%, 96%, 97%, 98%, 99%, 99.5%, 99.8%, 99.9%, or 100% to the genome of the bacterial strain of the genus *Megasphaera* with a deposit number of GDMCC No: 62001, GDMCC No: 62000, and/or GDMCC No: 61999;
preferably, the average nucleotide identity (ANI) value is 79%, 80%, 81%, 82%, 83%, 84%, 85%, 86%, 87%, 88%, 89%, 90%, 91%, 92%, 93%, 94%, 95%, 95.5%, 96%, 96.5%, 97%, 97.5%, 98%, 98.5%, 99%, 99.5%, 99.6%, 99.7%, 99.8%, 99.9%, or 100%.

6. A microbial agent, wherein the microbial agent comprises at least one of the bacterial strain of the genus *Megasphaera* of any one of claims 1 to 5, or a supernatant, an extract, or a metabolite of the bacterial strain of the genus *Megasphaera*;
preferably, the metabolite of the bacterial strain of the genus *Megasphaera* comprises at least one of (a) to (d): (a) acetic acid or acetate, (b) propionic acid or propionate, (c) butyric acid or butyrate, or (d) valeric acid or valerate;
preferably, the bacterial strain of the genus *Megasphaera* is highly productive of acetic acid and/or butyric acid.

7. A composition, comprising the microbial agent of claim 6, and at least one of an excipient, a diluent, or a carrier;
preferably, the microbial agent is in a powder or liquid dosage form;
preferably, the microbial agent in a powder dosage form is obtained by freeze drying, sublimation drying, or spray drying.

8. The composition according to claim 7, wherein the composition further comprises at least one of an immunosuppressor, an HDAC activity inhibitor, a radiotherapeutic agent, a targeting drug, a chemotherapeutic agent, a photosensitizing agent, a photothermal agent, an immunotherapeutic agent, or an agent for enhancing cell therapy;
wherein the immunosuppressor comprises at least one of a PD-1 inhibitor, a CTLA-4 inhibitor, a PD-L1 inhibitor, or a TIM-3 or LAG-3 inhibitor;
more preferably, the immunosuppressor comprises at least one of a PD-1 antibody, a CTLA-4 antibody, a PD-L1 antibody, a TIM-3 antibody, or an LAG-3 antibody;
more preferably, the PD-L1 antibody is selected from durvalumab, atezolizumab, or avelumab; more preferably, the PD-1 antibody or PD-L1 antibody is selected from pembrolizumab or nivolumab;
more preferably, the CTLA-4 antibody is selected from ipilimumab.

9. The composition according to claim 7, wherein the composition further comprises a target drug;
preferably, the target drug is a VEGF inhibitor or an RTK inhibitor;
preferably, the VEGF inhibitor or RTK inhibitor is selected from at least one of bevacizumab, pazopanib, sunitinib, sorafenib, axitinib, regorafenib, cabozantinib, nintedanib, ramucirumab, and lenvatinib.

10. Use of the bacterial strain of the genus *Megasphaera* of any one of claims 1 to 5, the microbial agent of claim 5, or the composition of any one of claims 7 to 9 in the manufacture of a pharmaceutical composition for preventing and/or treating a tumor.

11. A pharmaceutical composition for preventing and/or treating a tumor, comprising a first drug; wherein
the first drug is the bacterial strain of the genus *Megasphaera* of any one of claims 1 to 5, the microbial agent of claim 6, or the composition of any one of claims 7 to 9;
preferably, the tumor comprises a solid tumor, a soft tissue tumor, a hematopoietic tumor, an adenoma, or a metastatic tumor;
preferably, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma, and fibrosarcoma; or the tumor is a tumor caused by a virus or a tumor caused by a bacterium, such as a tumor caused by human papilloma virus infection, hepatitis B virus infection or hepatitis C virus infection, human immunodeficiency virus infection, *Helicobacter pylori* infection, Epstein-Barr virus infection, or *Fusobacterium nucleatum* infection;
preferably, the pharmaceutical composition treats or prevents the tumor by at least one manner selected from: (a) inhibiting tumor growth or proliferation, (b) preventing or inhibiting tumor cell spread or metastasis, (c) promoting tumor cell apoptosis, (d) anti-aging, (e) destroying angiogenesis in tumor tissue, (f) improving the response rate to an anti-tumor therapy, (g) inhibiting HDAC activity, (h) enhancing the anti-tumor activity of cytotoxic T cells, (i) enhancing subject's immune response, (j) inhibiting immune escape of the tumor, (k) enhancing the efficacy of a therapeutic agent, such as improving the efficacy of CAR-T therapy, (l) regulating the level of one or more symbiotic microorganisms in the subject's intestine, (m) improving the therapeutic efficacy of an immunosuppressor or immunosuppressive drug, such as improving the therapeutic efficacy of a PD-1 drug; (n) improving the therapeutic efficacy of a target drug such as lenvatinib; (o) enhancing the ability of CD8+ T cells to kill tumor cells, or (p) promoting recruitment of CD8+ T cells to the tumor;
more preferably, the inhibiting tumor growth or proliferation comprises reducing tumor volume, reducing tumor weight, and/or inhibiting tumor cell growth.

12. The pharmaceutical composition for preventing or treating a tumor according to claim 11, further comprising a second drug, wherein the first drug and/or the second drug has an effect of enhancing the activity of antigen-specific CD4+ T cells, or enhancing the activity of antigen-specific CD8+ T cells;
preferably, the second drug comprises at least one of an immunosuppressor, an HDAC activity inhibitor, a radiotherapeutic agent, a targeting drug, a chemotherapeutic agent, a photosensitizing agent, a photothermal agent, an immunotherapeutic agent, or an agent for enhancing cell therapy;
wherein the immunosuppressor comprises at least one of a PD-1 inhibitor, a CTLA-4 inhibitor, a PD-L1 inhibitor, or a TIM-3 or LAG-3 inhibitor;
more preferably, the immunosuppressor comprises at least one of a PD-1 antibody, a CTLA-4 antibody, a PD-L1 antibody, a TIM-3 antibody, or an LAG-3 antibody;
more preferably, the PD-L1 antibody is selected from durvalumab, atezolizumab, or avelumab; more preferably, the PD-1 antibody or PD-L1 antibody is selected from pembrolizumab or nivolumab;
more preferably, the CTLA-4 antibody is selected from ipilimumab.

13. The pharmaceutical composition for preventing or treating a tumor according to claim 11, comprising a second drug selected from any one of a regulatory T cell (Treg) inhibitor, a Treg ablator, a Treg migration inhibitor, a Treg function inhibitor, and a combination thereof;
preferably, the first drug and/or the second drug inhibits or reduces the activity, function or migration of Treg cells;
preferably, the Treg function inhibitor comprises a VEGF inhibitor or an RTK inhibitor;
more preferably, the VEGF inhibitor or RTK inhibitor is selected from at least one of bevacizumab, pazopanib, sunitinib, sorafenib, axitinib, regorafenib, cabozantinib, nintedanib, ramucirumab, and lenvatinib.

14. The composition according to claim 12 or 13, wherein the composition is administered by a manner comprising: sequentially administering the first drug and the second drug, simultaneously administering the first drug and the second drug, administering the first drug before administering the second drug, or administering the first drug and the second drug according to their respective frequencies of use;
preferably, the anti-tumor drug comprises a PD-1 inhibitor or a target drug, and the effective dose of the PD-1 inhibitor or target drug is 1-100 mg/kg;
more preferably, the effective dose of the PD-1 inhibitor or target drug comprises: 1 mg/kg, 2 mg/kg, 3 mg/kg, 4 mg/kg, 5 mg/kg, 6 mg/kg, 7 mg/kg, 8 mg/kg, 10 mg/kg, 20 mg/kg, 30 mg/kg, 40 mg/kg, 50 mg/kg, 60 mg/kg, 70 mg/kg, 80 mg/kg, 90 mg/kg, or 100 mg/kg; and optionally the target drug is lenvatinib.

15. The pharmaceutical composition according to claim 7, **characterized by** any one or more of (1) to (9) below:
(1) the pharmaceutical composition is in a dosage form suitable for infants, children, or adults;
(2) the pharmaceutical composition is in a dosage form for gastrointestinal administration or parenteral administration;
(3) the pharmaceutical composition is an oral preparation or an injection;
(4) the strain is capable of at least partially proliferating in the intestinal tract of a subject;
(5) the strain, or a metabolite or culture of the strain, is present in the pharmaceutical composition in a mass percent content of 1-80%, 2-70%, 5-60%, 10-50%, 20-40%, 45%, 50%, 55%, or 60%;
(6) the strain is in a form selected from viable bacteria, attenuated bacteria, killed bacteria, lyophilized bacteria, or irradiated bacteria;
(7) the pharmaceutical composition comprises 1×10³ to 1×10¹³ colony forming units (CFU) of the strain per gram of the pharmaceutical composition by weight;
(8) the pharmaceutical composition is in a powder or liquid dosage form; and
(9) the pharmaceutical composition is prepared into a lyophilized powder, a tablet, a capsule, a granule, or an injection.

16. A pharmaceutical composition comprising a bacterial strain of the genus *Megasphaera,* for use in the treatment or prevention of a tumor;
the bacterial strain of the genus *Megasphaera* is selected from the bacterial strains of the genus *Megasphaera* according to any one of claims 1 to 5;
preferably, the tumor comprises a solid tumor, a soft tissue tumor, a hematopoietic tumor, an adenoma, or a metastatic tumor;
preferably, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma, and fibrosarcoma; or the tumor is a tumor caused by a virus or a tumor caused by a bacterium, such as a tumor caused by human papilloma virus infection, hepatitis B virus infection or hepatitis C virus infection, human immunodeficiency virus infection, *Helicobacter pylori* infection, Epstein-Barr virus infection, or *Fusobacterium nucleatum* infection;
preferably, the strain or a metabolite of the strain or the culture of claim 4 treats or prevents the tumor by at least one manner selected from: (a) inhibiting tumor volume growth; (b) inhibiting tumor weight increase; (c) inhibiting tumor cell growth; (d) improving the tumor response rate to a therapy; (e) improving the therapeutic efficacy of an immunosuppressive drug, e.g., improving the therapeutic efficacy of a PD-1 drug; (f) inhibiting tumor cell metastasis; (g) reducing PD-1 drug resistance; (h) preventing or inhibiting tumor cell spread or metastasis; (i) inhibiting HDAC activity through at least one of acetic acid or acetate, propionic acid or propionate, butyric acid or butyrate, valeric acid or valerate in SCFAs; (j) inhibiting the tumor through at least one short-chain fatty acid or short-chain fatty acid salt of acetic acid or acetate, propionic acid or propionate, butyric acid or butyrate, valeric acid or valerate in SCFAs; (k) inhibiting the tumor by regulating the subject's immune system to exert an immunomodulatory effect; (l) promoting aging or apoptosis of cancer cells or tumor cells; (m) stimulating the subject's immune system; or (n) inhibiting angiogenesis in tumor tissue.

17. The pharmaceutical composition comprising a bacterial strain of the genus *Megasphaera* according to claim 16, wherein the bacterial strain in the composition expresses butyrate-acetyl CoA-transferase subunit A (EC2.8.3.9 enzyme), and/or has a butyrate production pathway with at least 70% integrity, and/or achieves the prevention and/or treatment of the tumor or cancer by inhibiting histone acetylase (HDAC) activity;
preferably, the bacterial strain is regulated by GENE ID:650027236, 641897133, 650594268, 642201644, 650018449, 650536170, 2511555023, and/or 646248671 to express EC2.8.3.9;
preferably, the butyrate production pathway is pyruvate pathway and/or 4-aminobutyrate pathway; preferably, the histone acetylase is class I, class II, class III, and/or class IV histone acetylase.

18. The pharmaceutical composition comprising a bacterial strain of the genus *Megasphaera* according to claim 16, wherein the bacterial strain is selected from a strain with at least 95%, 96%, 96.5%, 97%, 97.5%, 98%, 98.5%, 98.65%, 99%, 99.5%, 99.9%, or 100% identity to the 16S rRNA sequence of SEQ ID NO: 1, SEQ ID NO: 2, and/or SEQ ID NO: 3, or a strain of the genus *Megasphaera* that expresses EC2.8.3.9 and/or has a butyrate production pathway with more than 70% integrity;
preferably, the bacterial strain is selected from at least one of *Megasphaera stantonii*, *Megasphaera indica, Megasphaera paucivorans, Megasphaera sueciensis, Megasphaera micronuciformis*, *Megasphaera hexanoica*, *Megasphaera cerevisiae*, *Megasphaera hominis*, *Megasphaera butyrica*, *Megasphaera hutchinsoni*, *Megasphaera lornae*, and *Megasphaera vaginalis*;
preferably, the bacterial strain is regulated by GENE ID:650027236, 641897133, 650594268, 642201644, 650018449, 650536170, 2511555023, and/or 646248671 to express EC2.8.3.9.

19. The pharmaceutical composition comprising a bacterial strain of the genus *Megasphaera* according to any one of claims 14 to 18, wherein the composition, by producing a short-chain fatty acid, inhibits histone acetylase activity, and/or enhances the anti-tumor activity of cytotoxic T cells, and/or enhances subject's immune response, and/or inhibits tumor cell growth or spread, and/or inhibits immune escape of the tumor, and/or enhances the efficacy of a therapeutic agent, and/or regulates the level of one or more symbiotic microorganisms in the subject's intestine, and/or upregulates a pro-inflammatory cytokine and/or chemokine, and/or reduces intestinal barrier permeability, and/or upregulates a protective immunogenic cytokine, and/or modulates a chemokine; preferably, the pro-inflammatory cytokine is selected from one or more of interferon γ, interleukin IL-1β, IL-6, IL-12, IL-23, and tumor necrosis factor; the chemokine is selected from one or more of MCP-1 (CCL2), MIG (CXCL9), RANTES (CCL5), and IP-10 (CXCL-10);
preferably, the short-chain fatty acid comprises at least one of acetic acid, butyric acid, or valeric acid;
preferably, the bacterial strain is capable of increasing production of one or more pro-inflammatory cytokines and/or chemokines by human macrophages, monocytes, peripheral blood mononuclear cells, or monocyte-derived dendritic cells, and/or is capable of increasing infiltration of T-cells in the tumor.

20. The pharmaceutical composition comprising a bacterial strain of the genus *Megasphaera* according to any one of claims 10 to 16, **characterized by** any one or more of (1) to (7) below:
(1) the pharmaceutical composition is in a dosage form suitable for infants, children, or adults;
(2) the pharmaceutical composition is in a dosage form for gastrointestinal administration or parenteral administration;
(3) the pharmaceutical composition is an oral preparation or an injection;
(4) the strain is capable of at least partially proliferating in the intestinal tract of a subject;
(5) the strain, or a metabolite or culture of the strain, is present in the pharmaceutical composition in a mass percent content of 1-80%, 2-70%, 5-60%, 10-50%, 20-40%, 45%, 50%, 55%, or 60%;
(6) the strain is in a form selected from viable bacteria, attenuated bacteria, killed bacteria, lyophilized bacteria, or irradiated bacteria; and
(7) the pharmaceutical composition comprises 1×10³ to 1×10¹³ colony forming units (CFU) of the strain per gram of the pharmaceutical composition by weight.

21. Use of the bacterial strain of any one of claims 1 to 5 or a metabolite thereof or the bacterial agent of claim 6 in the manufacture of a medicament for treating or preventing a tumor; preferably, the tumor comprises a solid tumor, a soft tissue tumor, a hematopoietic tumor, an adenoma, or a metastatic tumor;
preferably, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma, and fibrosarcoma; or the tumor is a tumor caused by a virus or a tumor caused by a bacterium, such as a tumor caused by human papilloma virus infection, hepatitis B virus infection or hepatitis C virus infection, human immunodeficiency virus infection, *Helicobacter pylori* infection, Epstein-Barr virus infection, or *Fusobacterium nucleatum* infection;
preferably, the strain or a metabolite of the strain or the culture of claim 4 treats or prevents the tumor by at least one manner selected from: (a) inhibiting tumor volume growth; (b) inhibiting tumor weight increase; (c) inhibiting tumor cell growth; (d) improving the tumor response rate to a therapy; (e) improving the therapeutic efficacy of an immunosuppressive drug, e.g., improving the therapeutic efficacy of a PD-1 drug; (f) inhibiting tumor cell metastasis; (g) reducing PD-1 drug resistance; (h) preventing or inhibiting tumor cell spread or metastasis; (i) inhibiting HDAC activity through at least one of acetic acid or acetate, propionic acid or propionate, butyric acid or butyrate, valeric acid or valerate in SCFAs; (j) inhibiting the tumor through at least one short-chain fatty acid or short-chain fatty acid salt of acetic acid or acetate, propionic acid or propionate, butyric acid or butyrate, valeric acid or valerate in SCFAs; (k) inhibiting the tumor by regulating the subject's immune system to exert an immunomodulatory effect; (l) promoting aging or apoptosis of cancer cells or tumor cells; (m) stimulating the subject's immune system; or (n) inhibiting angiogenesis in tumor tissue.

22. Use of the bacterial strain of any one of claims 1 to 5 or a metabolite thereof or the bacterial agent of claim 6 in the manufacture of a medicament for preventing or treating a disease mediated by HDAC activity, wherein the disease comprises a disease mediated by HDAC activity; the disease mediated by HDAC activity comprises at least one of a tumor, a metabolic disease, diabetes, an autoimmune disease, an infectious disease, a central nervous disease, and inflammatory bowel disease;
preferably, the bacterial strain is used for treating the disease mediated by HDAC activity;
more preferably, the bacterial strain treats the disease by inhibiting HDAC activity through a short-chain fatty acid or short-chain fatty acid salt; the diseases comprises at least one of liver cancer, colon cancer, rectal cancer, colorectal cancer, lung cancer, breast cancer, ovarian cancer, pancreatic cancer, bile duct cancer, kidney cancer, fibrosarcoma, metastatic melanoma, cervical cancer, neuroblastoma, lymphoma, leukemia, gastric cancer, hematological malignancy, prostate cancer, dermatosis, dyspepsia, edema, asthma, arthritis, graft-versus-host disease, Crohn's disease, ulcerative colitis, allogeneic transplant rejection, chronic inflammatory bowel disease, systemic lupus erythematosus, psoriasis, rheumatoid arthritis, multiple sclerosis, Hashimoto's disease, food allergy, meningitis, myelitis, gastrointestinal inflammatory disease, cardiovascular inflammatory disease, chronic obstructive pulmonary disease, depression, anxiety, post-traumatic stress disorder, obsessive-compulsive disorder, Parkinson's disease, cerebral atrophy, angiosclerotic Parkinson's disease, atherosclerotic Parkinson's disease, mild cognitive impairment, cognitive impairment, or Alzheimer's disease.

23. Use of the bacterial strain of any one of claims 1 to 5 or a metabolite thereof, the bacterial agent of claim 6, or the pharmaceutical composition of any one of claims 11 to 20 in the manufacture of a medicament for treating and/or preventing a disease mediated by HDAC activity;
wherein the bacterial strain of the genus *Megasphaera* and/or a metabolite of the bacterial strain of the genus *Megasphaera* and/or a supernatant of the bacterial strain of the genus *Megasphaera* treats or prevents a tumor by inhibiting HDAC activity;
preferably, the tumor comprises at least one of liver cancer, colon cancer, rectal cancer, colorectal cancer, lung cancer, breast cancer, cervical cancer, ovarian cancer, pancreatic cancer, bile duct cancer, kidney cancer, fibrosarcoma, metastatic melanoma, cervical cancer, neuroblastoma, lymphoma, leukemia, gastric cancer, hematological malignancy, and prostate cancer;
preferably, the HDAC activity is inhibited by regulating a short-chain fatty acid/short-chain fatty acid salt; the short-chain fatty acid comprises one or more of acetic acid, butyric acid, and valeric acid; more preferably, the short-chain fatty acid is butyric acid; preferably, the short-chain fatty acid salt comprises one or more of acetate, propionate, and valerate; more preferably, the short-chain fatty acid salt is butyrate; and preferably, the histone acetylase is class I, class II, class III, and/or class IV histone acetylase.

24. Use of a bacterial strain of the genus *Megasphaera* and/or a composition comprising the bacterial strain of the genus *Megasphaera* in the manufacture of a medicament for preventing or treating a tumor;
wherein the bacterial strain has a 16S rRNA sequence with at least 95%, 95.5%, 96%, 96.5%, 97%, 97.5%, 98%, 98.5%, 98.65%, 99%, 99.5%, 99.9%, or 100% identity to the 16S rRNA sequence of SEQ ID NO: 1, SEQ ID NO: 2, and/or SEQ ID NO: 3;
the composition comprising the bacterial strain of the genus *Megasphaera* comprises (1) at least one of the bacterial strain, or a supernatant, an extract, or a metabolite of the bacterial strain, and (2) at least one of an excipient, a diluent, or a carrier.

25. The use of the bacterial strain of the genus *Megasphaera* and/or a composition comprising the bacterial strain of the genus *Megasphaera* in the manufacture of a medicament for preventing or treating a tumor according to claim 24 , **characterized by** any one or more of (1) to (9) below:
(1) the pharmaceutical composition is in a dosage form suitable for infants, children, or adults;
(2) the pharmaceutical composition is in a dosage form for gastrointestinal administration or parenteral administration;
(3) the pharmaceutical composition is an oral preparation or an injection;
(4) the strain is capable of at least partially proliferating in the intestinal tract of a subject;
(5) the strain, or a metabolite or culture of the strain, is present in the pharmaceutical composition in a mass percent content of 1-80%, 2-70%, 5-60%, 10-50%, 20-40%, 45%, 50%, 55%, or 60%;
(6) the strain is in a form selected from viable bacteria, attenuated bacteria, killed bacteria, lyophilized bacteria, or irradiated bacteria;
(7) the pharmaceutical composition comprises 1×10³ to 1×10¹³ colony forming units (CFU) of the strain per gram of the pharmaceutical composition by weight;
(8) the pharmaceutical composition is in a powder or liquid dosage form; and
(9) the pharmaceutical composition is prepared into a lyophilized powder, a tablet, a capsule, a granule, or an injection.

26. The use of the bacterial strain of the genus *Megasphaera* and/or a composition comprising the bacterial strain of the genus *Megasphaera* in the manufacture of a medicament for preventing or treating a tumor according to claim 25, wherein the bacterial strain is a strain deposited with the Guangdong Microbial Culture Collection Center with a deposit number of GDMCC No: 62001, GDMCC No: 62000, and/or GDMCC No: 61999;
preferably, the bacterial strain has an average nucleotide identity (ANI) value of at least 79% to the bacterial strain of the genus *Megasphaera* with a deposit number of GDMCC No: 62001, GDMCC No: 62000, and/or GDMCC No: 61999;
preferably, the average nucleotide identity (ANI) value is 79%, 80%, 81%, 82%, 83%, 84%, 85%, 86%, 87%, 88%, 89%, 90%, 91%, 92%, 93%, 94%, 95%, 95.5%, 96%, 96.5%, 97%, 97.5%, 98%, 98.5%, 99%, 99.5%, 99.6%, 99.7%, 99.8%, 99.9%, or 100%.

27. The use of the bacterial strain of the genus *Megasphaera* and/or a composition comprising the bacterial strain of the genus *Megasphaera* in the manufacture of a medicament for preventing or treating a tumor according to claim 25 or 26, wherein the tumor is a disease mediated by HDAC activity;
preferably, the tumor mediated by HDAC activity comprises at least one of liver cancer, colon cancer, rectal cancer, colorectal cancer, lung cancer, breast cancer, cervical cancer, ovarian cancer, pancreatic cancer, bile duct cancer, kidney cancer, fibrosarcoma, metastatic melanoma, cervical cancer, neuroblastoma, lymphoma, leukemia, gastric cancer, hematological malignancy, prostate cancer, or neuroendocrine cancer.

28. The use of the bacterial strain of the genus *Megasphaera* and/or a composition comprising the bacterial strain of the genus *Megasphaera* in the manufacture of a medicament for preventing or treating a tumor according to claim 27, wherein the tumor comprises a solid tumor.

29. The use of the bacterial strain of the genus *Megasphaera* and/or a composition comprising the bacterial strain of the genus *Megasphaera* in the manufacture of a medicament for preventing or treating a tumor according to any one of claims 25 to 28, wherein the bacterial strain is capable of partially or completely colonizing the intestine.

30. The use of the bacterial strain of the genus *Megasphaera* and/or a composition comprising the bacterial strain of the genus *Megasphaera* in the manufacture of a medicament for preventing or treating a tumor according to any one of claims 25 to 28, wherein the bacterial strain and/or the composition comprising the bacterial strain of the genus *Megasphaera* is used for reducing tumor volume, inhibiting tumor growth, preventing metastasis, or preventing angiogenesis.

31. Use of a bacterial strain of the genus *Megasphaera* and/or a composition comprising the bacterial strain of the genus *Megasphaera* in the manufacture of a medicament for treating, preventing, or delaying immunosenescence;
preferably, the bacterial strain and/or the composition comprising the bacterial strain of the genus *Megasphaera* is used for treating a cardiovascular disease; a neurodegenerative disease, such as Alzheimer's disease or Parkinson's disease; a cancer; type II diabetes; or an autoimmune disease by treating, preventing, or delaying immunosenescence.

32. The use of the bacterial strain of the genus *Megasphaera* and/or a composition comprising the bacterial strain of the genus *Megasphaera* in the manufacture of a medicament for preventing or treating a tumor according to any one of claims 25 to 30, or the use in the manufacture of a medicament for treating, preventing, or delaying immunosenescence according to claim 31;
wherein the bacterial strain is present in the medicament in a volume proportion of 20-60%, preferably 40%, 45%, 50%, 55%, or 60%.

33. The use according to claim 32, wherein the bacterial strain comprises viable bacteria, attenuated bacteria, killed bacteria, lyophilized bacteria, or irradiated bacteria.

34. The use according to claim 32, wherein the medicament is in a dosage form suitable for infants, children, or adults.

35. The use according to claim 34, wherein the medicament is in a dosage form for gastrointestinal administration or parenteral administration.

36. The use according to claim 34, wherein the medicament is an oral drug or an injection.

37. The use according to claim 32, wherein the medicament further comprises one or more second active ingredients.

38. The use according to claim 37, wherein the medicament is an oral drug or an injection, and the medicament comprises the second active ingredient;
preferably, the second active ingredient comprises at least one of a target drug, an immunosuppressor, an HDAC activity inhibitor, a radiotherapeutic agent, a targeting drug, a chemotherapeutic agent, a photosensitizing agent, a photothermal agent, an immunotherapeutic agent, or an agent for enhancing cell therapy; wherein the immunosuppressor comprises at least one of a PD-1 inhibitor, a CTLA-4 inhibitor, a PD-L1 inhibitor, or a TIM-3 or LAG-3 inhibitor;
more preferably, the immunosuppressor comprises at least one of a PD-1 antibody, a CTLA-4 antibody, a PD-L1 antibody, a TIM-3 antibody, an LAG-3 antibody, or lenvatinib;
more preferably, the target drug is a VEGF inhibitor or an RTK inhibitor; the VEGF inhibitor or RTK inhibitor is selected from at least one of bevacizumab, pazopanib, sunitinib, sorafenib, axitinib, regorafenib, cabozantinib, nintedanib, ramucirumab, and lenvatinib;
more preferably, the PD-L1 antibody is selected from durvalumab, atezolizumab, or avelumab; more preferably, the PD-1 antibody or PD-L1 antibody is selected from pembrolizumab or nivolumab;
more preferably, the CTLA-4 antibody is selected from ipilimumab.

39. The use according to any one of claims 32 to 38, wherein the medicament is an oral drug or an injection; and the bacterial strain promotes, increases, or enhances the anti-tumor effect of the second active ingredient.

40. The use according to any one of claims 32 to 38, wherein the medicament is an oral drug or an injection; the bacterial strain is present in the medicament in a unit dose of 9×10⁸ - 2×10⁹ CFU/mL or 1×10^{6 -} 2×10⁹ CFU/mg of the bacterial strain of the genus *Megasphaera*; the unit dose is preferably 9×10⁸ - 2×10⁹ CFU /mL or 1×10⁶ - 2×10⁹ CFU /mg of the bacterial strain of the genus *Megasphaera*; and the unit dose is preferably at least one of 9×10⁸ CFU/mL or 9×10⁸ CFU/mg, 6.0×10⁹ CFU/mL or 6.0×10⁹ CFU/mg, 6.3×10⁹ CFU/mL or 6.3×10⁹ CFU/mg, 6.5×10⁹ CFU/mL or 6.5×10⁹ CFU/mg, 9.2×10⁹ CFU/mL or 9.2×10⁹ CFU/mg, 9.3×10⁹ CFU/mL or 9.3×10⁹ CFU/mg, 9.4×10⁹ CFU/mL or 1.1×10⁹ CFU/mg, 1.2×10⁹ CFU/mL or 1.8×10⁹ CFU/mg, 1.4×10⁹ CFU/mL or 1.6×10⁹ CFU/mg, 1.7×10⁹ CFU/mL or 1.2×10¹⁰ CFU/mg, 1.8×10⁹ CFU/mL or 1.4×10⁹ CFU/mg, 1.6×10⁹ CFU/mL or 1.6×10¹⁰ CFU/mg, 1.8×10¹⁰ CFU/mL or 1.8×10¹⁰ CFU/mg, and 2×10¹⁰ CFU/mL or 2×10¹⁰ CFU/mg;
optionally, the anti-tumor medicament comprises a PD-1 inhibitor, a VEGF inhibitor, or an RTK inhibitor, and the effective dose of the PD-1 inhibitor, the VEGF inhibitor, or the RTK inhibitor is 1-120 mg/kg; preferably, the effective dose of the PD-1 inhibitor, such as a PD-1 antibody, or the VEGF inhibitor or the RTK inhibitor, such as lenvatinib, comprises: 1 mg/kg, 2 mg/kg, 3 mg/kg, 4 mg/kg, 5 mg/kg, 6 mg/kg, 7 mg/kg, 8 mg/kg, 10 mg/kg, 20 mg/kg, 30 mg/kg, 40 mg/kg, 50 mg/kg, 60 mg/kg, 70 mg/kg, 80 mg/kg, 90 mg/kg, 100 mg/kg, 110 mg/kg, or 120 mg/kg; and optionally the target drug is lenvatinib.

41. A method for treating or preventing a tumor or diabetes, comprising the step of administering to a subject in need thereof an effective amount of the strain of any one of claims 1 to 5, a metabolite of the strain, or the microbial agent of claim 6;
preferably, the tumor comprises a solid tumor, a soft tissue tumor, a hematopoietic tumor, an adenoma, or a metastatic tumor;
preferably, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, melanoma, lymphoma, small cell lung cancer, squamous cell carcinoma, chondrosarcoma, and fibrosarcoma; or the tumor is a tumor caused by a virus or a tumor caused by a bacterium, such as a tumor caused by human papilloma virus infection, hepatitis B virus infection or hepatitis C virus infection, human immunodeficiency virus infection, *Helicobacter pylori* infection, Epstein-Barr virus infection, or *Fusobacterium nucleatum* infection;
preferably, the strain or a metabolite of the strain or the culture of claim 4 treats or prevents the tumor by at least one manner selected from: (a) inhibiting tumor volume growth; (b) inhibiting tumor weight increase; (c) inhibiting tumor cell growth; (d) improving the tumor response rate to a therapy; (e) improving the therapeutic efficacy of an immunosuppressive drug, e.g., improving the therapeutic efficacy of a PD-1 drug; (f) inhibiting tumor cell metastasis; (g) reducing PD-1 drug resistance; (h) preventing or inhibiting tumor cell spread or metastasis; (i) inhibiting HDAC activity through at least one of acetic acid or acetate, propionic acid or propionate, butyric acid or butyrate, valeric acid or valerate in SCFAs; (j) inhibiting the tumor through at least one short-chain fatty acid or short-chain fatty acid salt of acetic acid or acetate, propionic acid or propionate, butyric acid or butyrate, valeric acid or valerate in SCFAs; (k) inhibiting the tumor by regulating the subject's immune system to exert an immunomodulatory effect; (l) promoting aging or apoptosis of cancer cells or tumor cells; (m) stimulating the subject's immune system; or (n) inhibiting angiogenesis in tumor tissue.

42. The method according to claim 41, wherein the strain or a metabolite or culture of the strain achieves the treatment or prevention of the tumor by inhibiting HDAC activity and/or promoting IFNβ transcriptional activity;
preferably, the culture is a fermentation broth or a supernatant of the fermentation broth of the strain.

43. The method according to any one of claims 41 to 42, wherein the strain, or the metabolite, fermentation broth, or supernatant of the fermentation broth of the strain achieves the treatment or prevention of the tumor by upregulating a pro-inflammatory factor or a chemokine for immunostimulation and/or by downregulating an anti-inflammatory factor for immunomodulation; preferably, the pro-inflammatory factor is selected from one or more of IFNγ, IFNβ, IL-1β, IL-6, IL-1β, MCP-1 (CCL2), MIG (CXCL-9), RANTES (CCL5), and TNFα.
